(19) Europäisches Patentamt
European Patent Office
Office européen des brevets

(11) EP 0 412 594 B1

(12) EUROPEAN PATENT SPECIFICATION

(45) Date of publication and mention of the grant of the patent: **03.01.1996 Bulletin 1996/01**

(51) Int. Cl.$^6$: **C07D 249/12**, C07D 249/14, C07D 403/10, C07D 401/14, C07D 413/14, A61K 31/41

(21) Application number: **90202014.8**

(22) Date of filing: **23.07.1990**

# (54) Substituted triazolinones, triazolinethiones, and triazolinimines as angiotensin II antagonists

Substituierte Triazolinone, Triazolinthione und Triazolinimine als Angiotensin II-Antagonisten

Triazolinones, triazolinethiones et triazolinimines substituées comme antagonistes d'angiotensine II

(84) Designated Contracting States:
**CH DE FR GB IT LI NL**

(30) Priority: **28.07.1989 US 386328**
**04.04.1990 US 504507**

(43) Date of publication of application:
**13.02.1991 Bulletin 1991/07**

(73) Proprietor: **MERCK & CO. INC.**
**Rahway New Jersey 07065-0900 (US)**

(72) Inventors:
- **Chang, Linda L.**
  **Wayne, NJ 07470 (US)**
- **Ashton, Wallace T.**
  **Clark, NJ 07066 (US)**
- **Maccoss, Malcolm**
  **Freehold, NJ 07728 (US)**
- **Chakravarty, Prasun K.**
  **Edison, NJ 08820 (US)**
- **Greenlee, William J.**
  **Teaneck, NJ 07666 (US)**
- **Patchett, Arthur A.**
  **Westfield, NJ 07090 (US)**
- **Walsh, Thomas F.**
  **Westfield, NJ 07090 (US)**

(74) Representative: **Cole, William Gwyn et al**
**Harlow Essex CM20 2QR (GB)**

(56) References cited:
**EP-A- 0 323 737** **EP-A- 0 323 841**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.8/3.4

## Description

INTRODUCTION OF THE INVENTION:

This invention relates to novel substituted triazolinone, triazolinethione and triazolinimine compounds and derivatives thereof which are useful as angiotensin II antagonists in the treatment of elevated blood pressure and congestive heart failure. Thus, the substituted triazolinone, triazolinethione and triazolinimine compounds of the invention are useful as antihypertensives.

BACKGROUND OF THE INVENTION

Renin-angiotensin system (RAS) plays a central role in the regulation of normal blood pressure and seems to be critically involved in hypertension development and maintenance as well as congestive heart failure. Angiotensin II (A II) is an octapeptide hormone produced mainly in the blood during the cleavage of angiotensin I by angiotensin converting enzyme (ACE) localized on the endothelium of blood vessels of lung, kidney, and many other organs. It is the end product of the renin-angiotensin system (RAS) and is a powerful arterial vasoconstrictor that exerts its action by interacting with specific receptors present on cell membranes. One of the possible modes of controlling the RAS is angiotensin II receptor antagonism. Several peptide analogs of A II are known to inhibit the effect of this hormone by competitively blocking the receptors, but their experimental and clinical applications have been limited by the partial agonist activity and lack of oral absorption [M. Antonaccio. Clin. Exp. Hypertens. A4, 27-46 (1982); D. H. P. Streeten and G. H. Anderson, Jr. - Handbook of Hypertension, Clinical Pharmacology of Antihypertensive Drugs, ed. A. E. Doyle, Vol. 5, pp. 246-271, Elsevier Science Publisher, Amsterdam, The Netherlands, 1984].

Recently, several non-peptide compounds have been described as A II antagonists. Illustrative of such compounds are those disclosed in U.S. Patents 4,207,324; 4,340,598; 4,576,958; 4,582,847; and 4,880,804; in European Patent Applications 028,834; 245,637; 253,310; and 291,969; and in articles by A.T. Chiu, et al. [Eur. J. Pharm. Exp. Therap, 157, 13-21 (1988)] and by P.C. Wong, et al. [J. Pharm. Exp. Therap, 247, 1-7(1988)]. All of the U.S. Patents, European Patent Applications 028,834 and 253,310 and the two articles disclose substituted imidazole compounds which are generally bonded through a lower alkyl bridge to a substituted phenyl. European Patent Application 245,637 discloses derivatives of 4,5,6,7-tetrahydro-2H-imidazo[4,5-c]pyridine-6-carboxylic acid and analogs thereof as antihypertensive agents, specifically $Ca^{2+}$ channel blockers.

European patent specification no. 0 323 841-A discloses substituted pyrrole, pyrazole and triazole compounds which are useful as angiotensin II antagonists. Further triazole derivatives are described in US Patent No. 4,577,020 and European patent specification no. 0 165 777-A which are cross-referenced in EP-0 323 841-A.

DETAILED DESCRIPTION OF THE INVENTION

This invention relates to novel substituted triazole compounds and derivatives thereof which are useful as angiotensin II antagonists, as antihypertensives, in the treatment of congestive heart failure and in the treatment of elevated intraocular pressure. The compounds of this invention have the general formula (I):

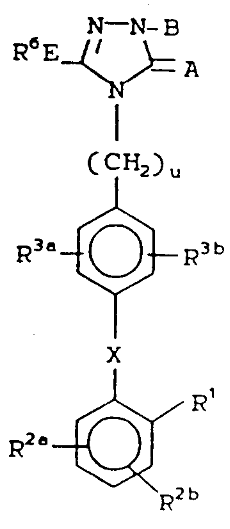

(I)

wherein:

$R^1$ is

(a) $-CO_2R^4$,
(b) $-SO_3R^5$,
(c) $-NHSO_2CF_3$,
(d) $-PO(OR^5)_2$,
(e) $-SO_2-NH-R^9$,
(f) $-CONHOR^5$,
(g) $-SO_2NH$-heteroaryl,
(h) $-CH_2SO_2NH$-heteroaryl,
(i) $-SO_2NHCOR^{23}$,
(j) $-CH_2SO_2NHCOR^{23}$,
(k) $-CONHSO_2R^{23}$,
(l) $-CH_2CONHSO_2R^{23}$,
(m) $-NHSO_2NHCOR^{23}$,
(n) $-NHCONHSO_2R^{23}$,
(o)

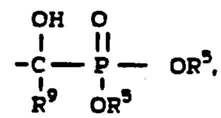

(p)

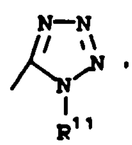

(q)

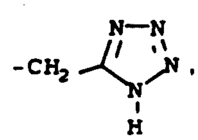

(r)

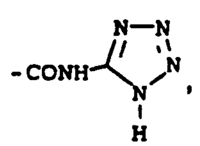

(s) $-CONHNHSO_2CF_3$ ,

(t)

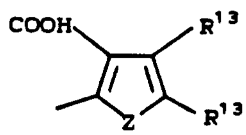

(u)

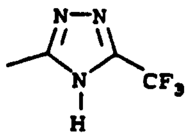

(v)

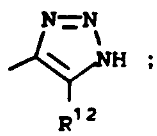

(w)

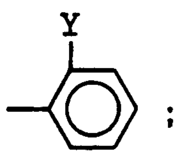

wherein:

heteroaryl is an unsubstituted, monosubstituted or disubstituted 5- or 6-membered aromatic ring which can optionally contain from 1 to 3 heteroatoms selected from the group consisting of O; N and S and wherein the substitiuents are members selected from the group consisting of -OH, -SH, $C_1$-$C_4$-alkyl, $C_1$-$C_4$-alkoxy, $-CF_3$, halo (Cl, Br, F, I), $-NO_2$, $-CO_2H$, $-CO_2$-$C_1$-$C_4$-alkyl, $-NH_2$, -NH($C_1$-$C_4$-alkyl) or -N($C_1$-$C_4$-alkyl$)_2$ and

Y is

(1) $-CO_2R^4$,
(2) $-SO_3R^5$,
(3) $-NHSO_2CF_3$,
(4) $-PO(OR^5)_2$, or
(5) $-SO_2$-NH-$R^9$;
(6) 1H-tetrazol-5-yl;

$R^{2a}$ and $R^{2b}$ are each independently:

(a) hydrogen,
(b) halogen (Cl, Br, I, F)

(c) $-NO_2$,
(d) $NH_2$,
(e) $C_1$-$C_4$-alkylamino,
(f) $-SO_2NHR^9$,
(g) $CF_3$,
(h) $C_1$-$C_4$-alkyl
(i) $C_1$-$C_4$-alkoxy; or

when $R^{2a}$ and $R^{2b}$ are on adjacent carbons, they can be bonded together to form a phenyl ring;

$R^{3a}$ is

(a) H,
(b) halo (Cl, Br, I, F)
(c) $C_1$-$C_6$-alkyl,
(d) $C_1$-$C_6$-alkoxy,
(e) $C_1$-$C_6$-alkoxy-$C_1$-$C_4$-alkyl;

$R^{3b}$ is

(a) H,
(b) halo (Cl, Br, I, F)
(c) $NO_2$,
(d) $C_1$-$C_6$-alkyl,
(e) $C_1$-$C_5$-alkylcarbonyloxy,
(f) $C_3$-$C_6$-cycloalkyl
(g) $C_1$-$C_6$-alkoxy,
(h) $-NHSO_2R^4$,
(i) hydroxy-$C_1$-$C_4$-alkyl,
(j) aryl-$C_1$-$C_4$-alkyl
(k) $C_1$-$C_4$-alkylthio
(l) $C_1$-$C_4$-alkylsulfinyl
(m) $C_1$-$C_4$-alkylsulfonyl
(n) $NH_2$
(o) $C_1$-$C_4$-alkylamino
(p) di($C_1$-$C_4$-alkyl)amino
(q) $CF_3$
(r) $-SO_2-NHR^9$
(s) aryl;
(t) furyl; or

when $R^{3a}$ and $R^{3b}$ are on adjacent carbons, they can be bonded together to form a phenyl ring;
wherein aryl is phenyl or naphthyl optionally substituted with one or two substituents selected from the group consisting of halo (Cl, Br, I, F) $C_1$-$C_4$-alkyl, $C_1$-$C_4$-alkoxy, $NO_2$, $CF_3$, $C_1$-$C_4$-alkylthio, OH or $NH_2$;

$R^4$ is H, straight chain or branched $C_1$-$C_6$ alkyl, benzyl or phenyl;

$R^5$ is H or $-CH(R^4)-O-CO-R^4$;

E is a single bond, $-NR^{13}(CH_2)_s-$,$-S(O)_x(CH_2)_s-$ where x is 0 to 2 and s is 0 to 5, -CH(OH)-, -0$(CH_2)_s$-, -C0-;

$R^6$ is

(a) aryl as defined above optionally substituted with 1 or 2 substituents selected from the group consisting of halo (Cl, Br, I, F), -0-$C_1$-$C_4$-alkyl, $C_1$-$C_4$-alkyl, $-NO_2$, $-CF_3$, -$SO_2NR^9R^{10}$, -S-$C_1$-$C_4$-alkyl, -OH, $-NH_2$, $C_3$-$C_7$-cycloalkyl, $C_3$-$C_{10}$-alkenyl;
(b) straight chain or branched $C_1$-$C_6$-alkyl, $C_2$-$C_6$-alkenyl or $C_2$-$C_6$-alkynyl each of which can be optionally substituted with one or more substituents selected from the group consisting of aryl as defined above, $C_3$-$C_7$-cycloalkyl, halo (Cl, Br, I, F), -OH, -O-$C_1$-$C_4$-alkyl, -$NH_2$, -NH($C_1$-$C_4$-alkyl), -N($C_1$-$C_4$-alkyl)$_2$, -NH-$SO_2R^4$, -$COOR^4$, -$SO_2NHR^9$, -S-$C_1$-$C_4$-alkyl;

(c) an unsubstituted, monosubstituted or disubstituted aromatic 5- or 6-membered ring which can contain one or two heteroatoms selected from the group consisting of N, O, S, and wherein the substituents are members selected from the group consisting of -OH, -SH, $C_1$-$C_4$-alkyl, $C_1$-$C_4$-alkyloxy, -$CF_3$, halo (Cl, Br, I, F), or $NO_2$;
(d) mono-, di-, tri- or polyfluoro-$C_1$-$C_5$-alkyl;
(e) $C_3$-$C_7$-cycloalkyl optionally substituted with one or more substituents selected from the group consisting of $C_1$-$C_4$-alkyl, O-$C_1$-$C_4$-alkyl, S-$C_1$-$C_4$-alkyl, OH, perfluoro-$C_1$-$C_4$-alkyl, or halo (Cl, Br, F, I);
(f) $C_3$-$C_7$-cycloalkyl-$C_1$-$C_3$-alkyl wherein the cycloalkyl is substituted as in (e) above;

A is =O, =S or =$NR^{21}$;

B is

(a) H provided A is not $NR^{21}$:
(b) $C_1$-$C_{10}$-alkyl;
(c) substituted $C_1$-$C_{10}$-alkyl in which one or more substituent(s) is selected from

(1) halogen (I, Br, Cl, F),
(2) hydroxy,
(3) $C_1$-$C_{10}$-alkoxy,
(4) $C_1$-$C_5$-alkoxycarbonyl, (5) $C_1$-$C_4$-alkylcarbonyloxy,
(6) $C_3$-$C_8$-cycloalkyl,
(7) aryl,
(8) substituted aryl in which the substituents are $V_1$, $V_2$, $V_3$, $V_4$ and $V_5$,
(9) $C_1$-$C_{10}$-alkyl-S(0)$_p$ in which p is 0 to 2,
(10) $C_3$-$C_8$-cycloalkyl-S(O)$_p$,
(11) phenyl-S(O)$_p$,
(12) substituted phenyl-S(0)$_p$ in which the substituents are $V_1$-$V_5$,
(13) oxo,
(14) carboxy,
(15) $NR^9R^9$,
(16) $C_1$-$C_5$-alkylaminocarbonyl,
(17) di($C_1$-$C_5$-alkyl)aminocarbonyl,
(18) cyano;

(d) $C_2$-$C_{10}$-alkenyl,
(e) $C_2$-$C_{10}$-alkynyl,
(f) $C_3$-$C_8$-cycloalkyl,
(g) substituted $C_3$-$C_8$-cycloalkyl or substituted $C_3$-$C_8$-cycloalkyl-$C_1$-$C_4$-alkyl having one or more substituents selected from the group:

(1) halo (Cl, Br, F, I),
(2) hydroxy,
(3) $C_1$-$C_6$-alkyl,
(4) $C_1$-$C_6$-alkoxy,
(5) $C_1$-$C_4$-alkylcarbonyloxy,
(6) $C_1$-$C_5$-alkoxycarbonyl,
(7) carboxy,
(8) oxo,
(9) $C_1$-$C_5$-alkylaminocarbonyl,
(10) di($C_1$-$C_5$-alkyl)aminocarbonyl
(11) $C_1$-$C_4$-alkylcarbonyl;
(12) aryl,
(13) substituted aryl in which the substituents are $V_1$, $V_2$, $V_3$, $V_4$ and $V_5$;

(h) aryl,

(i) substituted aryl in which the substituents are $V_1$, $V_2$, $V_3$, $V_4$ and $V_5$,

(j) aryl-$(CH_2)_r$-$(Q)_c$-$(CH_2)_t$-

(k) substituted aryl-$(CH_2)_r$-$(Q)_c$-$(CH_2)_t$- in which the aryl group is substituted with $V_1$, $V_2$, $V_3$, $V_4$ and $V_5$

(l) a heterocyclic ring of 5 to 6 atoms containing one or two heteroatoms selected from:

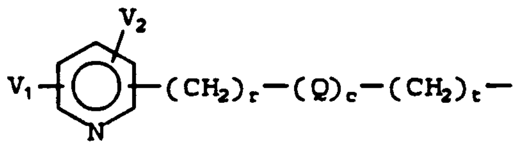

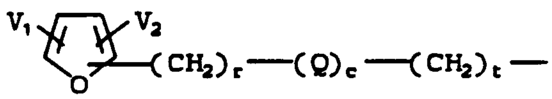

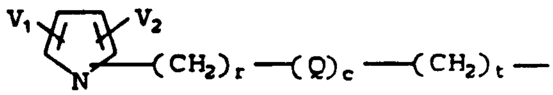

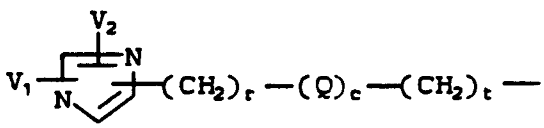

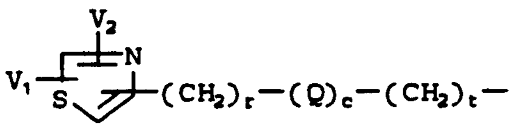
;

| | |
|---|---|
| $R^9$ is | H, $C_1$-$C_5$-alkyl, phenyl or benzyl; |
| $R^{10}$ is | H, $C_1$-$C_4$-alkyl, or |
| $R^9$ and $R^{10}$ | together can be -$(CH_2)_m$- where m is 3-6; |
| $R^{11}$ is | H, $C_1$-$C_6$-alkyl, $C_2$-$C_4$-alkenyl, $C_1$-$C_4$-alkoxy-$C_1$-$C_4$-alkyl, or -$CH_2$-$C_6H_4R^{20}$; |
| $R^{12}$ is | -CN, -$NO_2$ or -$CO_2R^4$; |
| $R^{13}$ is | H, $C_1$-$C_4$-acyl, $C_1$-$C_6$-alkyl, allyl, $C_3$-$C_6$-cycloalkyl, phenyl or benzyl; |
| $R^{14}$ is | H, $C_1$-$C_8$-alkyl, $C_1$-$C_8$-perfluoroalkyl, $C_3$-$C_6$-cycloalkyl, phenyl or benzyl; |
| $R^{15}$ is | H, $C_1$-$C_6$-alkyl, hydroxy; |
| $R^{16}$ is | H, $C_1$-$C_6$-alkyl, $C_3$-$C_6$-cycloalkyl, phenyl or benzyl; |

$R^{17}$ is $-NR^9R^{10}$, $-OR^{10}$, $-NHCONH_2$, $-NHCSNH_2$, $-NHSO_2CF_3$,

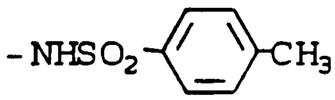 or 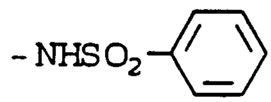 ;

$R^{18}$ and $R^{19}$ are independently $C_1$-$C_4$-alkyl or taken together are $-(CH_2)_q$-where q is 2 or 3;

$R^{20}$ is H, $-NO_2$, $-NH_2$, -OH or $-OCH_3$;

$R^{21}$ is

(a) H,
(b) aryl as defined above optionally substituted with 1 or 2 substituents selected from the group consisting of halo (Cl, Br, I, F) -0-$C_1$-$C_4$-alkyl, $C_1$-$C_4$-alkyl, $-NO_2$, $-CF_3$, $-SO_2NR^9R^{10}$, -S-$C_1$-$C_4$-alkyl, -OH, $-NH_2$, $-COOR^4$, $C_3$-$C_7$-cycloalkyl, $C_3$-$C_{10}$-alkenyl;
(c) straight chain or branched $C_1$-$C_6$-alkyl, $C_2$-$C_6$-alkenyl or $C_2$-$C_6$-alkynyl each of which can be optionally substituted with one or more substituents selected from the group consisting of aryl as defined above, $C_3$-$C_7$-cycloalkyl, halo (Cl, Br, I, F), -OH, -O-$C_1$-$C_4$-alkyl, $-NH_2$, -NH($C_1$-$C_4$-alkyl), -N($C_1$-$C_4$-alkyl)$_2$, $-NH-SO_2R^4$, $-COOR^4$, $-SO_2NHR^9$, -S-$C_1$-$C_4$-alkyl;
(d) an unsubstituted, monosubstituted or disubstituted aromatic 5 or 6 membered ring which can contain one or two heteroatoms selected from the group consisting of N, O, S, and wherein the substituents are members selected from the group consisting of -OH, -SH, $C_1$-$C_4$-alkyl, $C_1$-$C_4$-alkyloxy, $-CF_3$, $-COOR^4$, halo (Cl, Br, I, F), or $NO_2$; or
(e) $C_3$-$C_7$-cycloalkyl optionally substituted with one or more substituents selected from the group consisting of $C_1$-$C_4$-alkyl, -O-$C_1$-$C_4$-alkyl, -S-$C_1$-$C_4$-alkyl, -OH, $-COOR^4$, perfluoro-$C_1$-$C_4$-alkyl or halo (Cl, Br, F, I);

$R^{23}$ is

(a) aryl as defined above,
(b) heteroaryl as defined above,
(c) $C_3$-$C_7$-cycloalkyl,
(d) $C_1$-$C_4$-alkyl optionally substituted with a substituent selected from the group consisting of aryl as defined above, heteroaryl as defined above, -OH, -SH, $C_1$-$C_4$-alkyl, -O($C_1$-$C_4$-alkyl), S($C_1$-$C_4$-alkyl), $-CF_3$, halo (Cl, Br, F, I), $-NO_2$, $-CO_2H$, $-CO_2$-$C_1$-$C_4$-alkyl, $-NH_2$, -NH($C_1$-$C_4$-alkyl), -N($C_1$-$C_4$-alkyl)$_2$, $-N(CH_2CH_2)_2L$ where L is a single bond, $CH_2$, O, $S(O)_p$, or $NR^9$, $-PO_3H$, -PO(OH)(O-$C_1$-$C_4$-alkyl);

X is

(a) a single bond,
(b) -CO-,
(c) -O-,
(d) -S-,
(e)

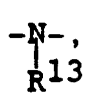

(f)

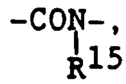

(g)

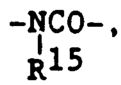

(h) $-OCH_2-$,
(i) $-CH_2O-$
(j) $-SCH_2-$,
(k) $-CH_2S-$,
(l) $-NHC(R^9)(R^{10})-$,
(m) $-NR^9SO_2-$,
(n) $-SO_2NR^9-$,
(o) $-C(R^9)(R^{10})NH-$,
(p) $-CH=CH-$,
(q) $-CF=CF-$,
(r) $-CH=CF-$,
(s) $-CF=CH-$,
(t) $-CH_2CH_2-$,
(u) $-CF_2CF_2-$,
(v)

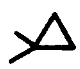
or
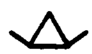

(w)

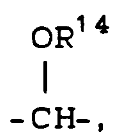

(x)

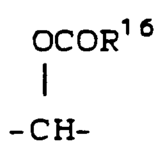

(y)

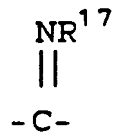 ,

or

(z)

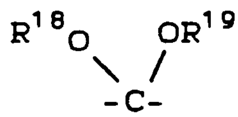 ;

| | |
|---|---|
| Q is | -C(O)-, -S-, -O- or $-NR^4$; |
| c is | 0 or 1; |
| r and t | are 0 to 2; |
| $V_1$, $V_2$, $V_3$, $V_4$ and $V_5$ | are each independently selected from: |

(a) H,
(b) $C_1$-$C_5$-alkoxy,
(c) $C_1$-$C_5$-alkyl,
(d) hydroxy,
(e) $C_1$-$C_5$-alkyl-$S(O)_p$,
(f) -CN,
(g) $-NO_2$,
(h) $-NR^9R^{10}$;
(i) $C_1$-$C_5$-alkyl-$CONR^9R^{10}$,
(j) $-CONR^9R^{10}$
(k) $-CO_2R^9$,
(l) $C_1$-$C_5$-alkyl-carbonyl,
(m) $CF_3$,
(n) halogen (I, Br, Cl, F),
(o) hydroxy-$C_1$-$C_4$-alkyl-,
(p) carboxy-$C_1$-$C_4$-alkyl-,
(q) -1H-tetrazol-5-yl,
(r) $-NH-SO_2CF_3$,
(s) aryl,
(t) $C_1$-$C_5$-alkyl-$CO_2R^9$,
(u) aryloxy,
(v) aryl-$C_1$-$C_3$-alkoxy,
(w) aryl-$C_1$-$C_3$-alkyl,
(x) carboxyphenyl,
(y) heteroaryl,
(z) 2-oxazolin-2-yl optionally bearing one or more $C_1$-$C_4$-alkyl substituents,
(aa) $-(CH_2)_tOCOR^9$,
(bb) $-(CH_2)_tOCONR^9R^{10}$,
(cc) $-(CH_2)_tNR^4COR^9$,
(dd) $-(CH_2)_tNR^4CO_2R^9$,

(ee) $-(CH_2)_tNR^4CONR^9R^{10}$,
(ff) $-(CH_2)_tNR^4CON(CH_2CH_2)_2G$,
(gg) $-(CH_2)_tOCON(CH_2CH_2)_2G$,
(hh) $-N(CH_2CH_2)_2G$,
(ii) $-C_1-C_5$-alkyl-$CON(CH_2CH_2)_2G$,
(jj) $-CON(CH_2CH_2)G$,

wherein G is O, $S(O)_p$ or $NR^9$, and

u is 1 or 2;

Z is O, $NR^{13}$ or S; and,

the pharmaceutically acceptable salts thereof.

One embodiment of the compounds of Formula (I) are those compounds wherein:

$R^1$ is

(a) -COOH,
(b)

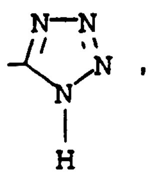

(c) $-NH-SO_2CF_3$,
(d) $-CONH-SO_2R^{23}$,
(e) $-SO_2NH-COR^{23}$,
(f) $-SO_2NH$-heteroaryl;

$R^{2a}$ is H;

$R^{2b}$ is H, F, Cl, $CF_3$ or $C_1$-$C_4$-alkyl;

$R^{3a}$ is H;

$R^{3b}$ is H, F, Cl, $CF_3$, $C_1$-$C_4$-alkyl, $C_5$-$C_6$-cycloalkyl, $-COOCH_3$, $-COOC_2H_5$, $-SO_2-CH_3$, $NH_2$, $-N(C_1-C_4\text{-alkyl})_2$ or $-NH-SO_2CH_3$;

E is a single bond, -O- or -S-;

$R^6$ is

(a) $C_1$-$C_6$-alkyl optionally substituted with a substituent selected from the group consisting of Cl, $CF_3$, -OH, $-O-CH_3$, $-OC_2H_5$, $-S-CH_3$, $-S-C_2H_5$, phenyl, or cyclopropyl;
(b) $C_2$-$C_6$-alkenyl or $C_2$-$C_6$-alkynyl;
(c) aryl as defined above optionally substituted with a substituent selected from the group consisting of halo (Cl, F, Br, I), $-CF_3$, $-NO_2$, -OH, $-NH_2$, $-S-CH_3$, $-S-C_2H_5$, $-SO_2NH_2$ $-O-CH_3$;
(d) a heteroaryl which is a member selected from the group consisting of 2-pyridyl, 4-pyridyl, 2-pyrimidyl, 4-pyrimidyl, imidazoyl, thiazolyl, thienyl, or furyl; or,
(e) perfluoro-$C_1$-$C_4$-alkyl which is a member selected from the group consisting of $CF_3$-, $CF_3CF_2$-, $CF_3CF_2CF_2$-, or $CF_3CF_2CF_2CF_2$-;
(f) $C_3$-$C_7$-cycloalkyl optionally substituted with a substituent selected from the group consisting of methyl, ethyl, $CF_3$ or $CF_3CF_2$;

A is =0, =S or $=NR^{21}$;

B is

(a) H provided A is not $NR^{21}$,
(b) $C_1$-$C_{10}$-alkyl,
(c) substituted $C_1$-$C_{10}$-alkyl in which one or two substituents are selected from:

(1) hydroxy,
(2) $C_1$-$C_5$-alkoxy,
(3) $C_1$-$C_5$-alkoxycarbonyl,
(4) $C_1$-$C_4$-alkylcarbonyloxy,
(5) $C_3$-$C_8$-cycloalkyl,
(6) phenyl,
(7) substituted phenyl in which the substituents are $V_1$, $V_2$, $V_3$, $V_4$ and $V_5$,
(8) $C_1$-$C_5$-alkyl-$S(O)_p$
(9) phenyl-$S(O)_p$
(10) substituted phenyl-$S(O)_p$ in which the substituent is V,
(11) oxo,
(12) carboxy,
(13) $C_1$-$C_5$-alkylaminocarbonyl;

(d) mono-, di-, tri-, or polyfluoro-$C_1$-$C_{10}$-alkyl,
(e) $C_2$-$C_{10}$-alkenyl,
(f) $C_2$-$C_{10}$-alkynyl.
(g) $C_3$-$C_8$-cycloalkyl,
(h) substituted $C_3$-$C_8$-cycloalkyl or substituted $C_3$-$C_8$-cycloalkyl-$C_1$-$C_4$-alkyl in which one or more substituent(s) is selected from:

(1) hydroxy,
(2) $C_1$-$C_5$-alkoxy,
(3) $C_1$-$C_5$-alkoxycarbonyl,
(4) $C_1$-$C_4$-alkylcarbonyloxy,
(5) $C_1$-$C_6$-alkyl,
(6) phenyl,
(7) substituted phenyl in which the substituents are $V_1$, $V_2$, $V_3$, $V_4$, and $V_5$,
(8) oxo,
(9) carboxy,
(10) $C_1$-$C_5$-alkylaminocarbonyl;

(i) aryl,
(j) substituted aryl in which the substituents are $V_1$, $V_2$, $V_3$, $V_4$ and $V_5$,
(k) aryl-$(CH_2)_r$-$(Q)_c$-$(CH_2)_t$-,
(l) substituted aryl-$(CH_2)_r$-$(Q)_c$-$(CH_2)_t$-

(m) a heterocyclic ring of 5 to 6 atoms containing one or two heteroatoms selected from:

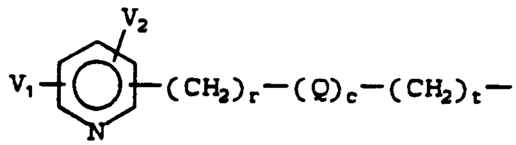

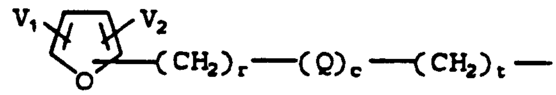

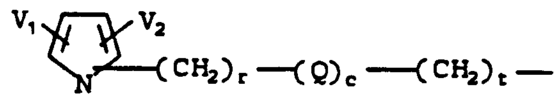

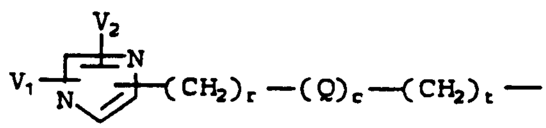

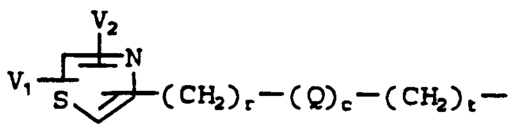

;

$V_1$, $V_2$, $V_3$, $V_4$ and $V_5$ are independently selected from:

(a) hydrogen,
(b) $C_1$-$C_5$-alkoxy,
(c) $C_1$-$C_5$-alkyl,
(d) hydroxy,
(e) $NR^9R^{10}$,
(f) $CO_2R^9$,
(g) trifluoromethyl;
(h) halogen;
(i) hydroxy-$C_1$-$C_4$-alkyl;
(j) -1H-tetrazol-5-yl,
(k) $-NH-SO_2CF_3$;
(l) CN;
(m) $NO_2$;
(n) $C_1$-$C_5$-alkyl-$CO_2R^9$,
(o) aryl,
(p) aryl-$C_1$-$C_3$-alkyl,
(q) heteroaryl,
(r) $C_1$-$C_5$-alkyl-$CONR^9R^{10}$,
(s) $-CONR^9R^{10}$,
(t) 2-oxazolin-2-yl optionally bearing one or more $C_1$-$C_4$-alkyl substituents,
(u) $C_1$-$C_5$-alkyl-$S(O)_p$,
(v) $(CH_2)_tOCOR^9$,
(w) $(CH_2)_tNR^4COR^9$;

u is 1;

X is:

(a) a single bond;
(b) -C(O)-;
(c) $-NR^{15}C(O)-$.

In one class of this embodiment are those compounds of formula (I) wherein:

E is a single bond or -S-;

$R^{2a}$, $R^{2b}$, $R^{3a}$ and $R^{3b}$ are each H;

$R^6$ is $C_1$-$C_6$ alkyl.

Illustrating this class are those compounds of formula (I) wherein:

A is =0, =S or $=NR^{21}$;

B is

(a) H provided A is not $NR^{21}$,
(b) $C_1$-$C_{10}$-alkyl,
(c) $C_3$-$C_8$-cycloalkyl,
(d) $C_3$-$C_8$-cycloalkyl-$C_1$-$C_4$-alkyl,
(e) substituted $C_1$-$C_{10}$-alkyl, $C_3$-$C_8$-cycloalkyl, or $C_3$-$C_8$-cycloalkyl-$C_1$-$C_4$-alkyl each of which can have one or two substituents selected from the group:

(1) hydroxy,
(2) $C_1$-$C_5$-alkoxy,
(3) $C_1$-$C_5$-alkoxycarbonyl,
(4) phenyl,
(5) carboxy,
(6) $C_1$-$C_5$-alkylaminocarbonyl,
(7) oxo;

(f) mono-, di-, tri-, or polyfluoro-$C_1$-$C_{10}$-alkyl,
(g) phenyl,
(h) phenyl substituted with $V_1$, $V_2$, $V_3$, $V_4$ and $V_5$,
(i) phenyl-$(CH_2)_r$-$(Q)_c$-$(CH_2)_t$-,
(j) phenyl-$(CH_2)_r$-$(Q)_c$-$(CH_2)_t$- in which the phenyl is substituted with $V_1$, $V_2$, $V_3$, $V_4$ and $V_5$,
(k) a heterocyclic moiety selected from:

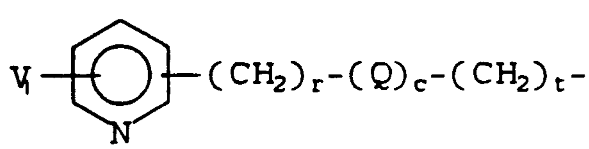

or

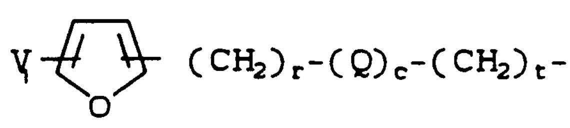

;

$V_1$, $V_2$, $V_3$, $V_4$ and $V_5$ are selected from:

(a) hydrogen,

(b) $C_1$-$C_5$-alkyl,
(c) $C_1$-$C_5$-alkoxy,
(d) $CO_2R^9$,
(e) halogen,
(f) hydroxy-$C_1$-$C_4$-alkyl-,
(g) $C_1$-$C_5$-alkyl-$CO_2R^9$,
(h) $C_1$-$C_5$-alkyl-$CONR^9R^{10}$,
(i) $CONR^9R^{10}$,
(j) CN,
(k) $NO_2$,
(l) $CF_3$;
(m) aryl,
(n) heteroaryl,
(o) 2-oxazolin-2-yl optionally bearing one or more $C_1$-$C_4$-alkyl substituents,
(p) $C_1$-$C_5$-alkyl-$S(O)_p$,
(q) $(CH_2)_tOCOR^9$,
(r) $(CH_2)_tNR^4COR^9$,
(s) hydroxy,
(t) $NR^9R^{10}$;

X is -$NR^{15}C(O)$- or a carbon-carbon single bond.

Another class of compounds of formula (I) are those wherein:

| | |
|---|---|
| $R^1$ is | 5-tetrazolyl or carboxy; |
| $R^{2a}$, $R^{2b}$, $R^{3a}$, and $R^{3b}$ | are each H; |
| $R^6$ is | $C_1$-$C_6$-alkyl; |
| A is | O or S; |
| B is H; | substituted or unsubstituted $C_1$-$C_{10}$-alkyl, $C_3$-$C_8$-cycloalkyl or $C_3$-$C_8$-cycloalkyl-$C_1$-$C_4$-alkyl; substituted or unsubstituted aryl, heteroaryl, or aralkyl; |
| E and X | are each single bonds; and, |
| u is | 1. |

Exemplifying this class are the following compounds:

(1) 5-n-butyl-4-[(2′-carboxybiphenyl-4-yl)methyl]-2,4-dihydro-3H-1,2,4-triazole-3-thione;
(2) 5-n-butyl-2,4-dihydro-4-[[2′-(5-tetrazolyl)biphenyl-4-yl]methyl]-3H-1,2,4-triazol-3-one;
(3) 2-benzyl-5-n-butyl-2,4-dihydro-4-[[2′-(5-tetrazolyl)biphenyl-4-yl]methyl]-3H-1,2,4-triazol-3-one;
(4) 5-n-butyl-2,4-dihydro-2-phenyl-4-[[2′-(5-tetrazolyl)biphenyl-4-yl]methyl]-3H-1,2,4-triazol-3-one;
(5) 5-n-butyl-2-(2-chlorophenyl)-2,4-dihydro-4-[[2′-(5-tetrazolyl)biphenyl-4-yl]methyl]-3H-1,2,4-triazol-3-one;
(6) 5-n-butyl-2-[2-(carbomethoxy)benzyl]-2,4-dihydro-4-[[2′-(5-tetrazolyl)biphenyl-4-yl]methyl]-3H-1,2,4-triazol-3-one;
(7) 5-n-butyl-2-(2-carboxybenzyl)-2,4-dihydro-4-[[2′-(5-tetrazolyl)biphenyl-4-yl]methyl]-3H-1,2,4-triazol-3-one;
(8) 2-s-butyl-5-n-butyl-2,4-dihydro-4-[[2′-(5-tetrazolyl)biphenyl-4-yl]methyl]-3H-1,2,4-triazol-3-one;
(9) 5-n-butyl-2-(3-chlorophenyl)-2,4-dihydro-4-[[2′-(5-tetrazolyl)biphenyl-4-yl]methyl]-3H-1,2,4-triazol-3-one;
(10) 5-n-butyl-2-(4-chlorophenyl)-2,4-dihydro-4-[[2′-(5-tetrazolyl)biphenyl-4-yl]methyl]-3H-1,2,4-triazol-3-one;
(11) 5-n-butyl-2-[α-(carbomethoxy)benzyl]-2,4-dihydro-4-[[2′-(5-tetrazolyl)biphenyl-4-yl]methyl]-3H-1,2,4-triazol-3-one;
(12) 5-n-butyl-2,4-dihydro-2-(3-methylbenzyl)-4-[[2′-(5-tetrazolyl)biphenyl-4-yl]methyl]-3H-1,2,4-triazol-3-one;
(13) 5-n-butyl-2,4-dihydro-2-(2-methylbenzyl)-4-[[2′-(5-tetrazolyl)biphenyl-4-yl]methyl]-3H-1,2,4-triazol-3-one;
(14) 5-n-butyl-2,4-dihydro-2-(α-methylbenzyl)-4-[[2′-(5-tetrazolyl)biphenyl-4-yl]methyl]-3H-1,2,4-triazol-3-one;
(15) 5-n-butyl-2,4-dihydro-2-(4-methylphenyl)-4-[[2′-(5-tetrazolyl)biphenyl-4-yl]methyl]-3H-1,2,4-triazol-3-one;
(16) 5-n-butyl-2,4-dihydro-2-(2-methylphenyl)-4-[[2′-(5-tetrazolyl)biphenyl-4-yl]methyl]-3H-1,2,4-triazol-3-one;
(17) 5-n-butyl-2,4-dihydro-2-(2-nitrophenyl)-4-[[2′-(5-tetrazolyl)biphenyl-4-yl]methyl]-3H-1,2,4-triazol-3-one;
(18) 5-n-butyl-2,4-dihydro-2-methyl-4-[[2′-(5-tetrazolyl)biphenyl-4-yl]methyl]-3H-1,2,4-triazol-3-one;
(19) 5-n-butyl-2-cyclopentyl-2,4-dihydro-4-[[2′-(5-tetrazolyl)biphenyl-4-yl]methyl]-3H-1,2,4-triazol-3-one;
(20) 5-n-butyl-2-carbomethoxymethyl-2,4-dihydro-4-[[2′-(5-tetrazolyl)biphenyl-4-yl]methyl]-3H-1,2,4-triazol-3-one;
(21) 5-n-butyl-2-(2-carboxyphenyl)-2,4-dihydro-4-[[2′-(5-tetrazolyl)biphenyl-4-yl]methyl]-3H-1,2,4-triazol-3-one;

(22) 5-n-butyl-2,4-dihydro-2-[2-(hydroxymethyl)phenyl]-4-[[2′-(5-tetrazolyl)biphenyl-4-yl]methyl]-3H-1,2,4-triazol-3-one;

(23) 2-(biphenyl-2-yl)-5-n-butyl-2,4-dihydro-4-[[2′-(5-tetrazolyl)biphenyl-4-yl]methyl]-3H-1,2,4-triazol-3-one;

(24) 5-n-butyl-2-(2,6-dichlorophenyl)-2,4-dihydro-4-[[2′-(5-tetrazolyl)biphenyl-4-yl]methyl]-3H-1,2,4-triazol-3-one;

(25) 5-n-butyl-2,4-dihydro-2-(2-methoxyphenyl)-4-[[2′-(5-tetrazolyl)biphenyl-4-yl]methyl]-3H-1,2,4-triazol-3-one;

(26) 2-(2-chlorophenyl)-2,4-dihydro-5-n-propyl-4-[[2′-(5-tetrazolyl)blphenyl-4-yl]methyl]-3H-1,2,4-triazol-3-one;

(27) 2,4-dihydro-2-(2-nitrophenyl)-5-n-propyl-4-[[2′-(5-tetrazolyl)biphenyl-4-yl]methyl]-3H-1,2,4-triazol-3-one;

(28) 5-n-butyl-2,4-dihydro-2-(2-pyridyl)-4-[[2′-(5-tetrazolyl)biphenyl-4-yl]methyl]-3H-1,2,4-triazol-3-one;

(29) 5-n-butyl-2-(2-fluorophenyl)-2,4-dihydro-4-[[2′-(5-tetrazolyl)biphenyl-4-yl]-3H-1,2,4-triazol-3-one;

(30) 5-n-butyl-2,4-dihydro-2-(4-fluorophenyl)-4-[[2′-(5-tetrazolyl)biphenyl-4-yl]methyl]-3H-1,2,4-triazol-3-one;

(31) 5-n-butyl-2-(pentafluorophenyl)-2,4-dihydro-4-[[2′-(5-tetrazolyl)biphenyl-4-yl]methyl]-3H-1,2,4-triazol-3-one;

(32) 5-n-butyl-2-(2-bromophenyl)-2,4-dihydro-4-[[2′-(5-tetrazolyl)biphenyl-4-yl]methyl]-3H-1,2,4-triazol-3-one;

(33) 5-n-butyl-2,4-dihydro-2-(3-methylphenyl)-4-[[2′-(5-tetrazolyl)biphenyl-4-yl]methyl]-3H-1,2,4-triazol-3-one;

(34) 5-n-butyl-2,4-dihydro-2-(4-ethylphenyl)-4-[[2′-(5-tetrazolyl)biphenyl-4-yl]methyl]-3H-1,2,4-triazol-3-one;

(35) 5-n-butyl-2-(3-nitrophenyl)-2,4-dihydro-4-[[2′-(5-tetrazolyl)biphenyl-4-yl]methyl]-3H-1,2,4-triazol-3-one;

(36) 5-n-butyl-2,4-dihydro-2-(4-nitrophenyl)-4-[[2′-(5-tetrazolyl)biphenyl-4-yl]methyl]-3H-1,2,4-triazol-3-one;

(37) 5-n-butyl-2,4-dihydro-2-(3-methoxyphenyl)-4-[[2′-(5-tetrazolyl)biphenyl-4-yl]methyl]-3H-1,2,4-triazol-3-one;

(38) 5-n-butyl-2,4-dihydro-2-(4-methoxyphenyl)-4-[[2′-(5-tetrazolyl)biphenyl-4-yl]methyl]-3H-1,2,4-triazol-3-one;

(39) 5-n-butyl-2,4-dihydro-2-[2-(acetoxymethyl)phenyl]-4-[[2′-(5-tetrazolyl)biphenyl-4-yl]methyl]-3H-1,2,4-triazol-3-one;

(40) 5-n-butyl-2-(2-benzylphenyl)-2,4-dihydro-4-[[2′-(5-tetrazolyl)biphenyl-4-yl]methyl]-3H-1,2,4-triazol-3-one;

(41) 5-n-butyl-2-[4-(carbomethoxy)phenyl]-2,4-dihydro-4-[[2′-(5-tetrazolyl)biphenyl-4-yl]methyl]-3H-1,2,4-triazol-3-one;

(42) 5-n-butyl-2,4-dihydro-2-(2-isopropylphenyl)-4-[[2′-(5-tetrazolyl)biphenyl-4-yl]methyl]-3H-1,2,4-triazol-3-one;

(43) 5-n-butyl-2,4-dihydro-2-[2-(N,N-dimethylamino)phenyl]-4-[[2′-(5-tetrazolyl)biphenyl-4-yl]methyl]-3H-1,2,4-triazol-3-one;

(44) 2-[2-(acetoxymethyl)benzyl]-5-n-butyl-2,4-dihydro-4-[[2′-(5-tetrazolyl)biphenyl-4-yl]methyl]-3H-1,2,4-triazol-3-one;

(45) 5-n-butyl-2-(4-methoxy-2-nitrophenyl)-2,4-dihydro-4-[[2′-(5-tetrazolyl)biphenyl-4-yl]methyl]-3H-1,2,4-triazol-3-one;

(46) 5-n-butyl-2,4-dihydro-2-(2,3,4,5,6-pentafluorobenzyl)-4-[[2′-(5-tetrazolyl)biphenyl-4-yl]methyl]-3H-1,2,4-triazol-3-one;

(47) 2-(2-chlorophenyl)-2,4-dihydro-5-n-pentyl-4-[[2′-(5-tetrazolyl)biphenyl-4-yl]methyl]-3H-1,2,4-triazol-3-one;

(48) 2,4-dihydro-2-(2-nitrophenyl)-5-n-pentyl-4-[[2′-(5-tetrazolyl)biphenyl-4-yl]methyl]-3H-1,2,4-triazol-3-one;

(49) 2-(cyclohexylmethyl)-5-n-butyl-2,4-dihydro-4-[[2′-(5-tetrazolyl)biphenyl-4-yl]methyl]-3H-1,2,4-triazol-3-one;

(50) 5-n-butyl-2,4-dihydro-2-ethyl-4-[[2′-(5-tetrazolyl)biphenyl-4-yl]methyl]-3H-1,2,4-triazol-3-one;

(51) 5-n-butyl-2-(4-methylbenzyl)-2,4-dihydro-4-[[2′-(5-tetrazolyl)biphenyl-4-yl]methyl]-3H-1,2,4-triazol-3-one;

(52) 2,5-di(n-butyl)-2,4-dihydro-4-[[2′-(5-tetrazolyl)biphenyl-4-yl]methyl]-3H-1,2,4-triazol-3-one;

(53) 5-n-butyl-2,4-dihydro-2-isopropyl-4-[[2′-(5-tetrazolyl)biphenyl-4-yl]methyl]-3H-1,2,4-triazol-3-one;

(54) 5-n-butyl-2-cyanomethyl-2,4-dihydro-4-[[2′-(5-tetrazolyl)biphenyl-4-yl]methyl]-3H-1,2,4-triazol-3-one;

(55) 5-n-butyl-2,4-dihydro-2-n-propyl-4-[[2′-(5-tetrazolyl)biphenyl-4-yl]methyl]-3H-1,2,4-triazol-3-one;

(56) 5-n-butyl-2,4-dihydro-4-[[2′-(5-tetrazolyl)biphenyl-4-yl]methyl]-2-(2,2,2-trifluoroethyl)-3H-1,2,4-triazol-3-one;

(57) 5-n-butyl-2-[2-(carbomethoxy)phenyl]-2,4-dihydro-4-[[2′-(5-tetrazolyl)biphenyl-4-yl]methyl]-3H-1,2,4-triazol-3-one;

(58) 5-n-butyl-2,4-dihydro-4-[[2′-(5-tetrazolyl)biphenyl-4-yl]methyl]-2-[2-(trifluoromethyl)phenyl]-3H-1,2,4-triazol-3-one;

(59) 5-n-butyl-2,4-dihydro-2-phenethyl-4-[[2′-(5-tetrazolyl)biphenyl-4-yl]methyl]-3H-1,2,4-triazol-3-one;

(60) 5-n-butyl-2-[1-(carbomethoxy)ethyl]-2,4-dihydro-4-[[2′-(5-tetrazolyl)biphenyl-4-yl]methyl]-3H-1,2,4-triazol-3-one; and,

(61) 5-n-butyl-2-(1-carboxyethyl)-2,4-dihydro-4-[[2′-(5-tetrazolyl)biphenyl-4-yl]methyl]-3H-1,2,4-triazol-3-one.

DISCUSSION OF CHEMISTRY AND REACTION SCHEMES

The compounds of Formula I can be prepared by a variety of methods typified by those described below. General synthetic methods for 2,4,5-trisubstituted-2,4-dihydro-3H-1,2,4-triazol-3-ones and -triazole-3-thiones are discussed in books or review articles such as:

(1) C. Temple and J.A. Montgomery, "Triazoles: 1,2,4" (Vol. 37 of The Chemistry of Heterocyclic Compounds, A. Weissberger and E.C. Taylor, eds.), Wiley-Interscience, New York, 1981, pp. 365-442.

(2) J.B. Polya, Comprehensive Heterocyclic Chemistry. The Structure, Reactions, Synthesis and Uses of Heterocyclic Compounds, A.R. Katritzky and C.W. Rees, eds., Vol. 5, Pergamon Press, Oxford, 1984, pp. 733-790.

(3) J.H. Boyer, Heterocyclic Compounds, R.C. Elderfield, ed., Vol. 7, John Wiley & Sons, New York, 1961, pp. 384-461.

In general, the compounds of Formula I are constructed in such a way that $N^1$ and $N^2$ of the triazole ring are derived from hydrazine or a hydrazine derivative, while $N^4$ of the triazole and the 4-(arylmethyl) substituent are derived directly or indirectly from a suitably substituted benzylamine (or isocyanate or isothiocyanate) or from a benzyl halide (or methanesulfonate, p-toluenesulfonate, etc.).

Although the Reaction Schemes described below are reasonably general, it will be understood by those skilled in the art of organic synthesis that one or more functional groups present in a given compound of Formula I may render the molecule incompatible with a particular synthetic sequence. In such a case an alternative route, an altered order of steps, or a strategy of protection and deprotection may be employed. In all cases the particular reaction conditions (including reagents, solvent, temperature, and time) should be chosen so that they are consistent with the nature of the functionality present in the molecule.

The Reaction Schemes below have been generalized for simplicity. It is to be understood that the "$ArCH_2$" substituent present at $N^4$ of the triazole derivatives or in their precursors is any substituted arylmethyl moiety consistent with the definition of the $N^4$ substituent in Formula I or which may be transformed to such a grouping either before or after the assembly of the triazole ring system. Such transformations may involve protection and/or deprotection, formation of the "X" linkage between the two aromatic rings as shown in Formula I, or other modifications. It is also to be understood that in most of the Reaction Schemes, the "$ArCH_2$" (Ar = aryl) substituent may be replaced by the homologous "$Ar(CH_2)_2$" group as consistent with the definition of Formula I.

It is further to be understood that in the generalized schemes below, unless specified otherwise, the R, R′ and R″ groups represent functionalized or unfunctionalized alkyl, aryl, heteroaryl, aralkyl, and the like, while Ar′ represents a functionalized or unfunctionalized aryl or heteroaryl group. The moiety, R′X, represents an alkylating agent in which R′ is typically a functionalized or unfunctionalized alkyl or aralkyl group, while X is a leaving group such as chloro, bromo, iodo, methanesulfonate, or p-toluenesulfonate. In structures showing an "X" group double-bonded to a carbon atom (as

in 22 and products derived therefrom), X is 0 or S.

## REACTION SCHEME 1

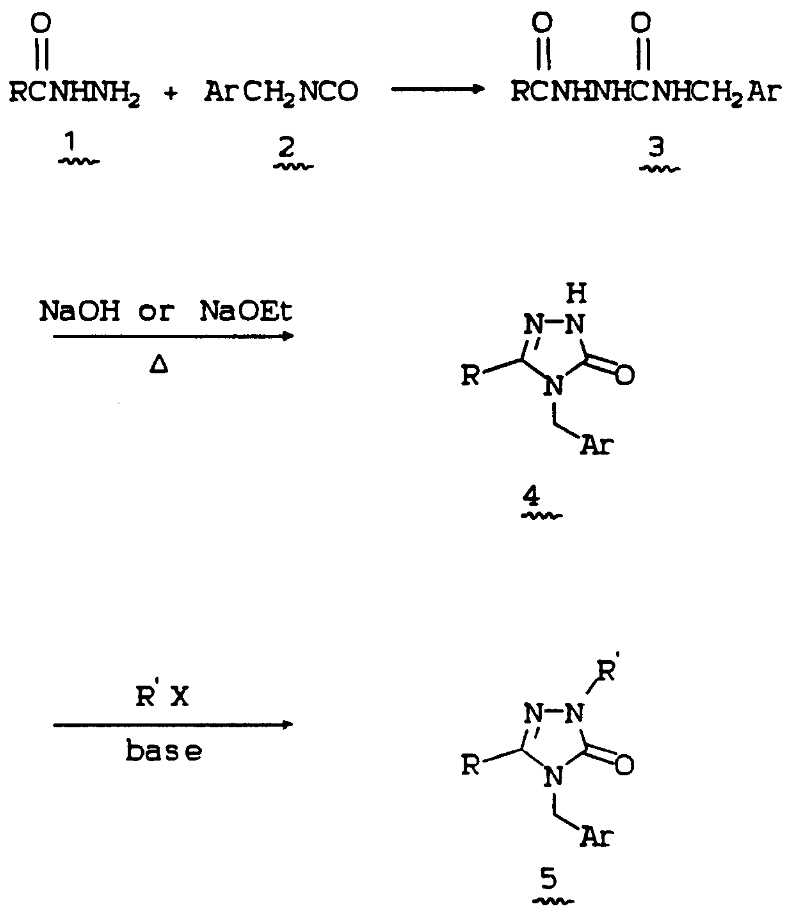


One of the most widely used routes to 2,4,5-trisubstituted-2,4-dihydro-3H-1,2,4-triazol-3-ones ("triazolinones") is shown in Reaction Scheme 1 in its adaptation for the synthesis of compounds of Formula I. Reaction of a carboxylic acid hydrazide 1 (readily obtained from the corresponding ester) with the appropriate arylmethyl isocyanate 2 gives the 1-acyl-4-(arylmethyl)semicarbazide 3. The isocyanate 2 itself is obtainable by well-known methods from various sources, including the (arylmethyl)amine (by phosgene treatment), the arylmethyl halide (by treatment with cyanate anion), and the arylacetic acid or derivative (via Curtius rearrangement of the acyl azide). Upon heating in the presence of hydroxide or alkoxide, cyclization of 3 to the triazolinone 4 occurs. Finally, in the presence of a base (e.g., sodium hydride, sodium ethoxide, sodium hydroxide, or potassium carbonate), 4 is converted to the trisubstituted triazolinone 5 on treatment with a suitable alkylating agent R′X, where R′ is alkyl, aralkyl, etc., and X is bromo, iodo, chloro, methanesulfonate, p-toluenesulfonate, and the like. Such reaction pathways have been described by D.L. Temple, Jr., and W.G. Lobeck, Jr., U.S. Patent 4,487,773 (1984), R.E. Gammans, D.W. Smith, and J.P. Yevich, U.S. Patent 4,613,600 (1986), and (in part)

H. Gehlen and W. Schade, Liebigs Ann. Chem., 675, 180 (1964), G. Palazzo, U.S. Patent 3,857,845 (1974), and K.H. Hauptmann and K. Zeile, British Patent 971,606 (1964). A modified approach to an intermediate of type 3 and its subsequent cyclization to a triazolinone analogous to 4 have been reported by H. Hrebabecky and J. Beranek, Collect. Czech. Chem. Commun., 50, 779 (1985).

## REACTION SCHEME 2

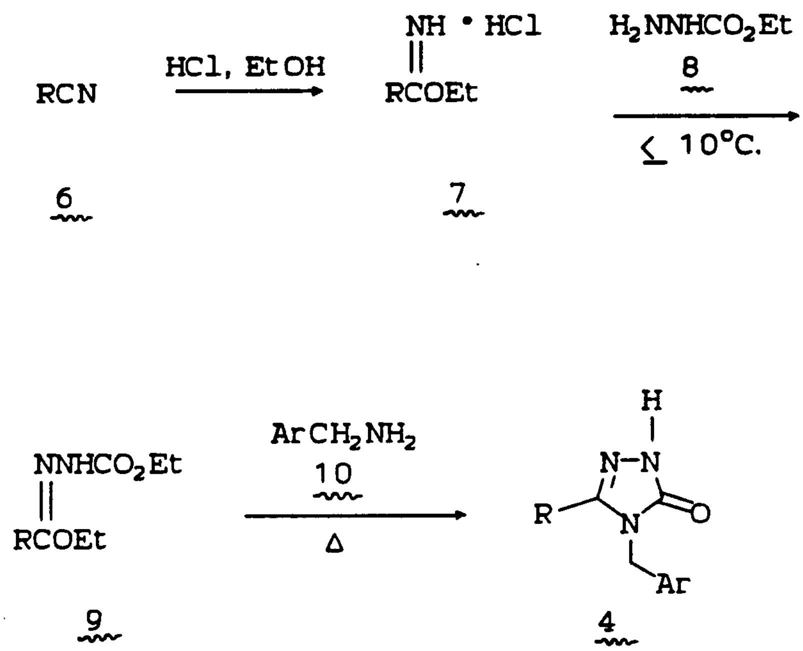


A highly useful alternative route to 4 is shown in Reaction Scheme 2. This approach has been described by M. Pesson, S. Dupin, and M. Antoine, Compt. Rend., 253, 285 (1961) and R. Un and A. Ikizler, Chim. Acta Turc., 3, 113 (1975). Addition of ethyl carbazate (8) to the imidate 7 (which is readily prepared from the corresponding nitrile 6) yields an adduct 9, which can be converted to the triazolinone 4 on heating with the (arylmethyl)amine 10 (typically at temperatures from 70-150°C.). As in Reaction Scheme 1, 4 can be alkylated to give the trisubstituted triazolinone 5.

## REACTION SCHEME 3

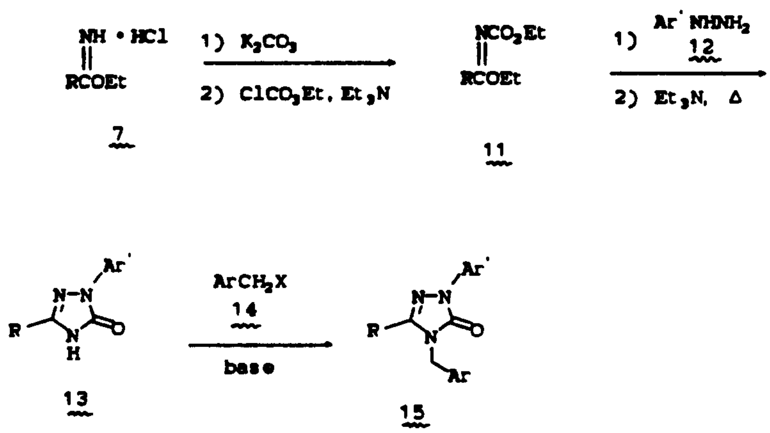


7 11 13 15

The procedures of Reaction Schemes 1 and 2 are not suitable for the introduction of most aryl or heteroaryl substituents at $N^2$. In contrast, the procedures of Reaction Schemes 3 to 6 are especially well suited for the synthesis of compounds of Formula I having aryl or heteroaryl substituents at $N^2$, since the triazolinone ring is constructed with the $N^2$-substituent in place, whereas the $N^4$-substituent is introduced subsequently by alkylation. Reaction Scheme 3 presents a route patterned after that reported by K. Yabutani, K. Taninaka, M. Kajioka, K. Takagi, H. Matsui, K. Sutoh, and M. Yamamoto, European Patent Application 220,952 (1987). The N-carbethoxy imidate 11 (obtained by reaction of 7 with ethyl chloroformate) is treated with an arylhydrazine 12 (or analog), typically at about 40-50°C. Without isolation of the intermediate, further heating at elevated temperature (usually in the range of 90-150°C.) in the presence of a tertiary amine such as triethylamine effects cyclization to the triazolinone 13. In the presence of a suitable base (e.g., sodium hydride, sodium alkoxide, sodium hydroxide) treatment of 13 with the appropriate $ArCH_2X$, where X = bromo, iodo, chloro, methanesulfonate, p-toluenesulfonate, and the like, yields the $N^4$-alkylated product 15. A variant of the method using a thioimidate has been described by M. Kajioka, H. Kurono, K. Okawa, and M. Harada, U.S. Patent No.

4,318,731 (1982).

## REACTION SCHEME 4

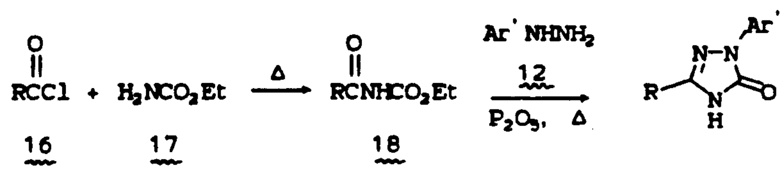


16 17 18 13

An alternative route to the $N^2$-substituted triazolinone intermediate 13 is shown in faction Scheme 4. This chemistry has been described by T.N. Ghosh and M.V. Betrabet, J. Indian Chem. Soc., 7, 899 (1930), S. Bellioni, Ann. Chim. (Rome), 52, 187 (1962), G. Palazzo and G. Picconi, Boll. Chim. Farm., 105, 217 (1966), and British Patent 1,021,070 (1966). An acid chloride 16 is heated with urethane (17) (typically at 80-100°C.), to give the acylurethane 18. Reaction of 18 with an arylhydrazine 12 and phosphorus pentoxide (usually in toluene or xylene at reflux) gives 13, which can then be further alkylated on $N^4$ as in Reaction Scheme 3. A (thioacyl)urethane modification of this pathway has been reported by D.L. Temple, Jr., and W.G. Lobeck, Jr., U.S. Patent 4,487,773 (1984).

## REACTION SCHEME 5

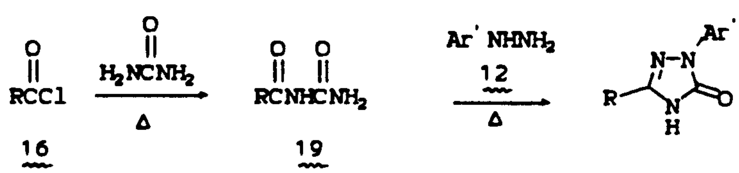


16 19 13

A variation of Reaction Scheme 4, shown in Reaction Scheme 5, has been described by P. Gold-Aubert, D. Melkonian, and L. Toribio, Helv. Chim. Acta, 47, 1188 (1964) and A.L. Langis, U.S. Patent 3,499,000 (1970). The readily prepared acylurea 19 upon heating with an arylhydrazine 12 (at about 150-200°C.) is converted to the triazolinone

intermediate 13.

## REACTION SCHEME 6

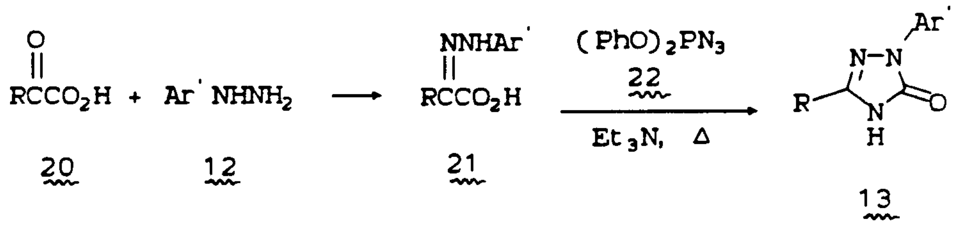


20 12 21 13

In a quite different approach (Reaction Scheme 6), L. Maravetz, U.S. Patent 4,705,557 (1987) and G. Theodoridis, International Patent Application WO87/03782 (1987) disclose condensing an $\alpha$-keto acid 20 with the arylhydrazine 12 to give derivatives such as 21, which can be converted to the triazolinone intermediate 13 by heating with diphenylphosphoryl azide and triethylamine (typically at 75-115°C.). In the last step, an intermediate acyl azide loses nitrogen and undergoes the Curtius rearrangement to an isocyanate, which undergoes ring closure. As shown in Reaction Scheme 3, 13 can then be alkylated on $N^4$ to give the trisubstituted triazolinone 15.

## REACTION SCHEME 7

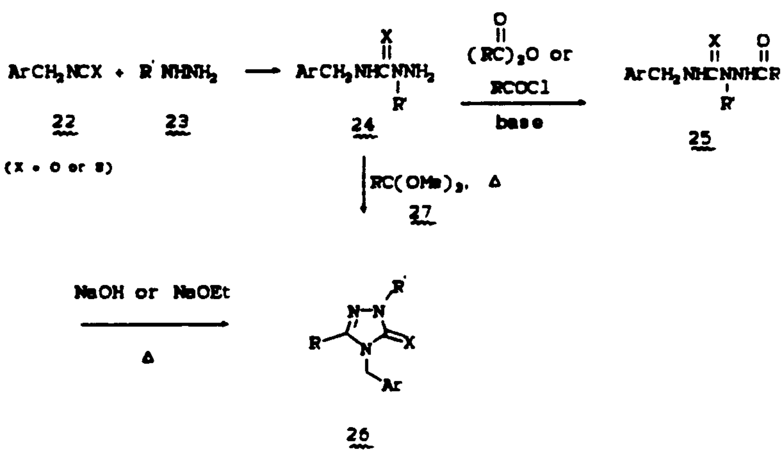


22 23 24 25
(X = O or S)
26

2,4,5-Trisubstituted-2,4-dihydro-3H-1,2,4-triazole-3-thiones ("triazolinethiones") cannot generally be prepared by routes analogous to those in Reaction Schemes 1 to 6 because of the propensity for alkylation to occur on sulfur rather than on the open ring nitrogen. It is thus preferable to have all of the substituents in place at the time of the ring closure to form the heterocycle. As shown in Reaction Scheme 7, for certain R′ groups (e.g., R′ = $CH_3$), reaction of the hydrazine derivative 23 with the appropriate isocyanate or isothiocyanate 22 yields the 2,4-disubstituted semicarbazide or thiosemicarbazide 24. Acylation of 24 gives 25, which can be cyclized upon heating with hydroxide or alkoxide to give the trisubstituted triazolinone or triazolinethione 26. This approach has been detailed by J.M. Kane and F.P. Miller, U.S. Patent 4,775,688 (1988) and G.F. Duffin, J.D Kendall, and H.R.J. Waddington, J. Chem. Soc., 3799 (1959). Alternative methods

of ring closure, such as heating 24 with the orthoester 27, can also be utilized.

## REACTION SCHEME 8

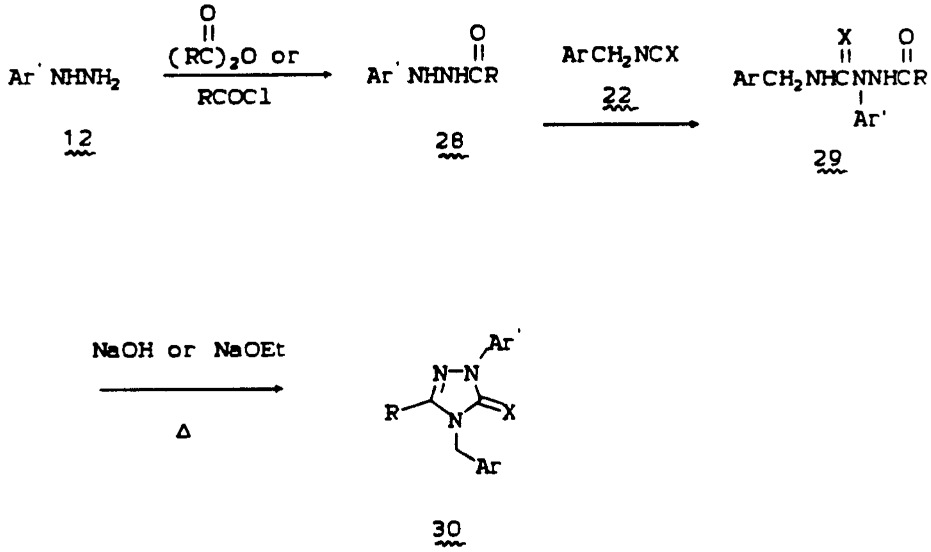


12 28 29 30

In Reaction Scheme 8, acylation of an aryl- or heteroaryl hydrazine gives 28, which can be reacted with the isocyanate or isothiocyanate 22 to yield the 1-acyl-2,4-disubstituted-semicarbazide or -thiosemicarbazide 29. Cyclization of 29 upon heating with hydroxide or alkoxide affords the triazolinone or triazolinethione 30. This chemistry has been described by H. Gehlen and W. Schade, Liebigs Ann. Chem., 675, 180 (1964).

## REACTION SCHEME 9

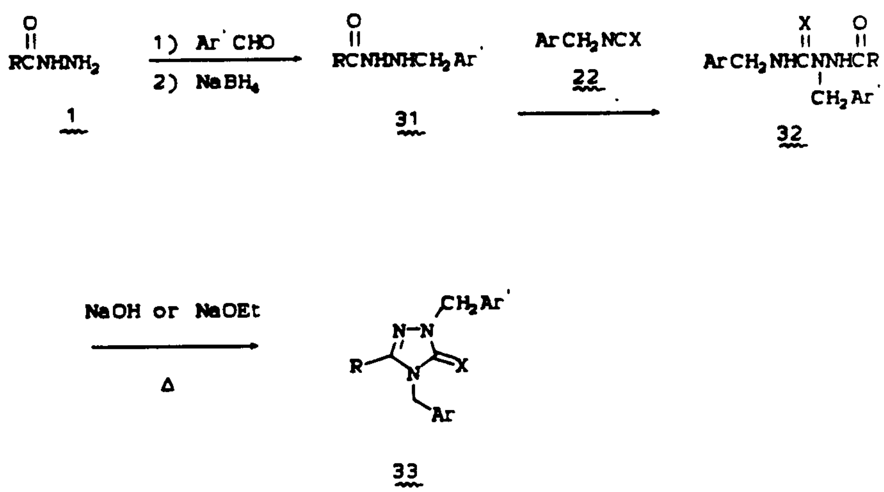


1 31 32 33

The method of F. Russo, M. Santagati, and G. Pappalardo [Ann. Chim. (Rome), 62, 351 (1972)] (Reaction Scheme 9) is useful for the synthesis of trisubstituted triazolinones and triazolinethiones having benzylic substituents at $N^2$. Treatment of a hydrazide 1 with an aromatic or heteroaromatic aldehyde followed by reduction with sodium borohydride gives the substituted hydrazide 31. Reaction of 31 with the isocyanate or isothiocyanate 22 affords the semicarbazide or thiosemicarbazide derivative 32, which is cyclized to the triazolinone or triazolinethione 33 upon heating with hydroxide

or alkoxide.

## REACTION SCHEME 10

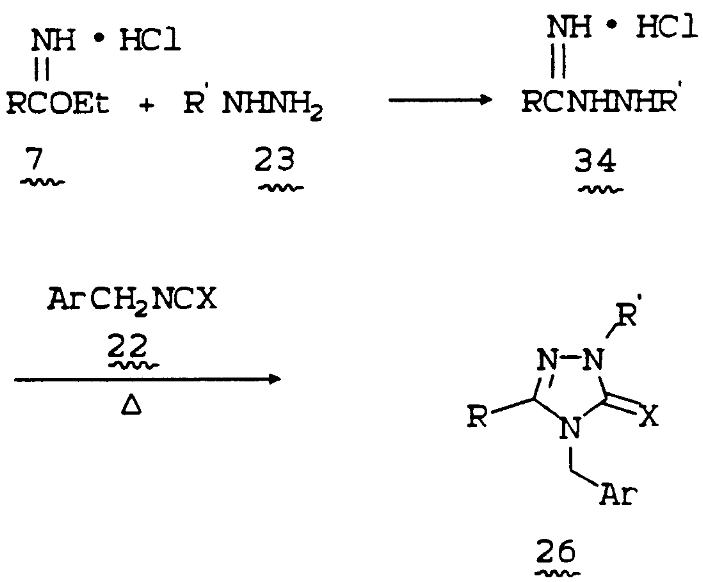


7 23 34 26

In another approach (Reaction Scheme 10), imidate 7 is treated with a substituted hydrazine 23 (especially an aryl or heteroaryl hydrazine) to give the amidrazone 34. Heating 34 with the isocyanate or isothiocyanate 22 gives the triazolinone or triazolinethione 26. Syntheses of this type have been reported by M. Santus, Acta Pol. Pharm., 37, 293 (1980); T. Bany, Rocz. Chem., 42, 247 (1968); and, T. Bany and M. Dobosz, Ann. Univ. Mariae Curie-Sklodowska. Sect.

AA, 26/27, 23 (1971).

## REACTION SCHEME 11

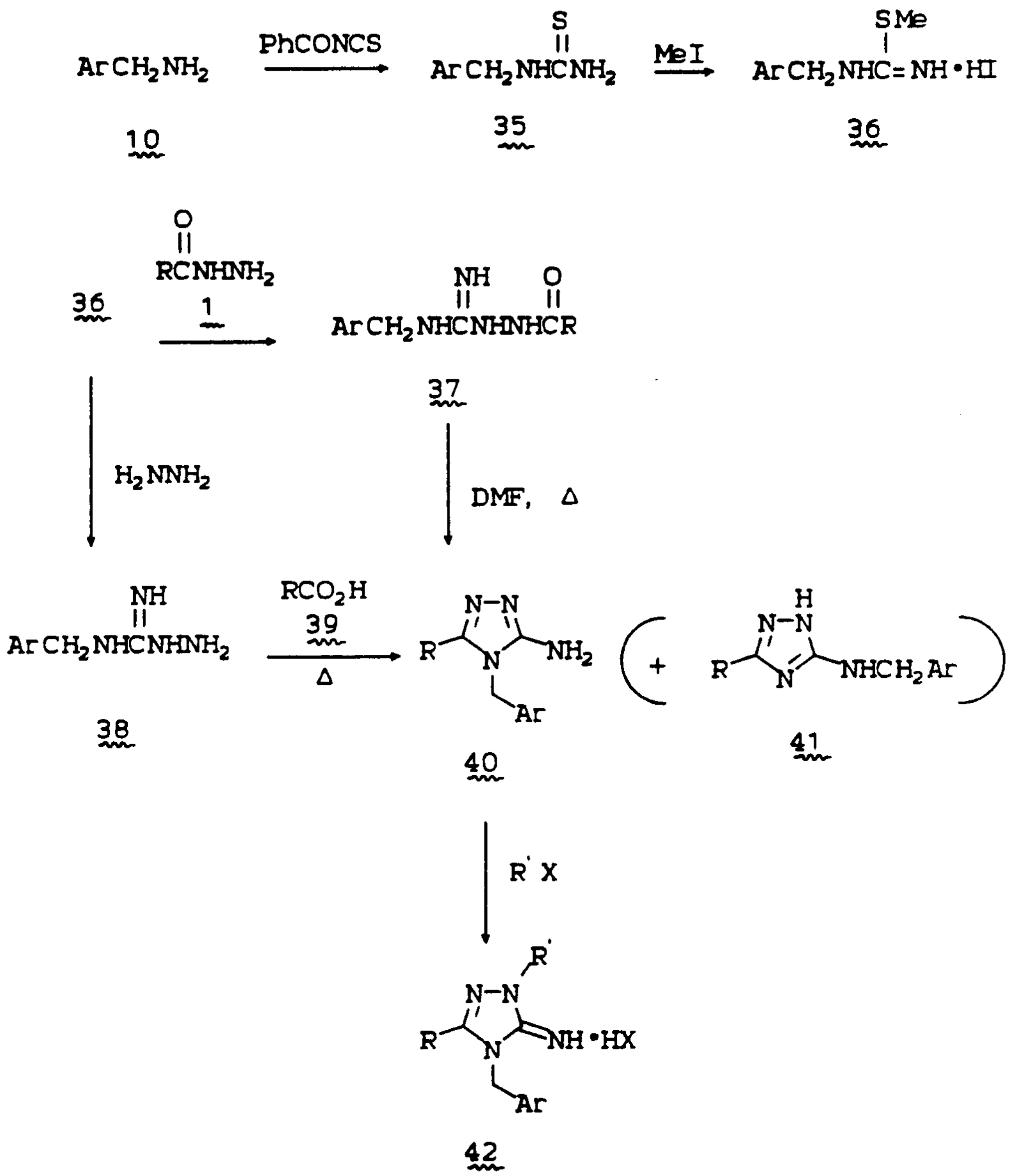


A route to 2,4,5-trisubstituted-2,4-dihydro-3H-1,2,4-triazol-3-imines ("triazolinimines") is outlined in Reaction Scheme 11. Reaction of the (arylmethyl)amine 10 with benzoyl isothiocyanate (or by other means) gives the substituted thiourea 35, which is methylated to prepare the isothiourea derivative 36. Compound 36 can be transformed to the acylaminoguanidine 37 by reacting with the hydrazide 1 or to the aminoguanidine 38 by reacting with hydrazine. Ring closure of 37 by heating in DMF or cyclization of 38 with carboxylic acid 39 at elevated temperature affords the aminotriazole 40, which can be separated from the isomer 41. Such pathways have been described by G.J. Durant, G.M. Smith, R.G.W. Spickett, and S.H.B. Wright, J. Med. Chem., 9, 22 (1966) and E. Akerblom, Acta Chem. Scand., 19, 1135 (1965). Finally, alkylation of 40 with the appropriate R′X (where X is a leaving group such as iodo, bromo, chloro, p-toluenesulfonate, or methanesulfonate) leads to the triazolinimine 42, which can be separated from any other isomers or by-products formed during the reaction. This method has been described by E.B. Akerblom and D.E.S. Campbell, J.

Med. Chem., 16, 312 (1973).

## REACTION SCHEME 12

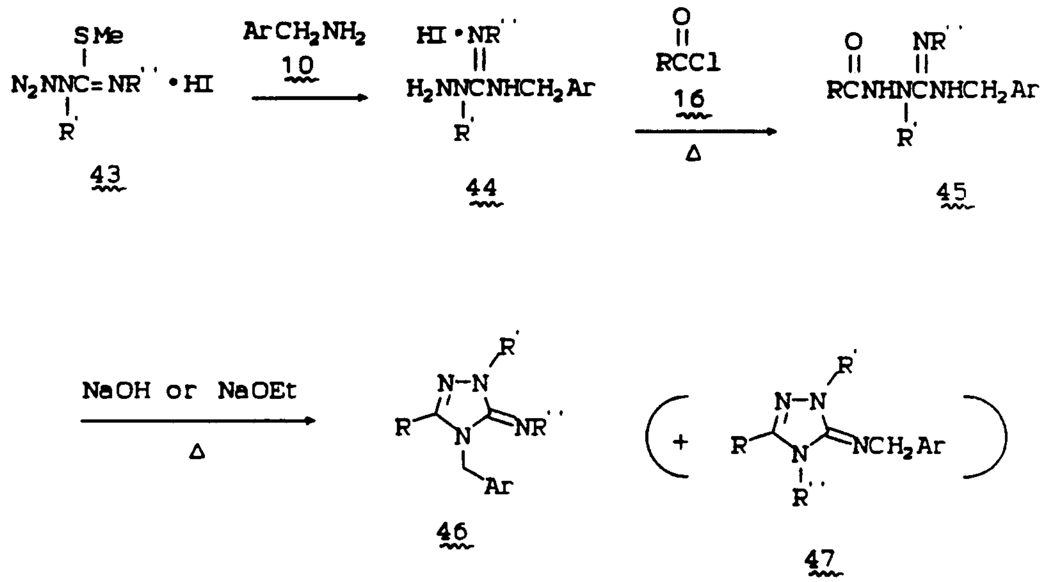


The route shown in Reaction Scheme 12 utilizes chemistry reported by E. Akerblom, Acta Chem. Scand., 19, 1135 (1965). The substituted isothiourea 43 is treated with amine 10 to give the aminoguanidine derivative 44. Acylation of 44 with the acid chloride 16 provides the intermediate 45, which can be cyclized by heating with hydroxide or alkoxide. The desired triazolinimine 46 is separated from the isomeric product 47.

## REACTION SCHEME 13

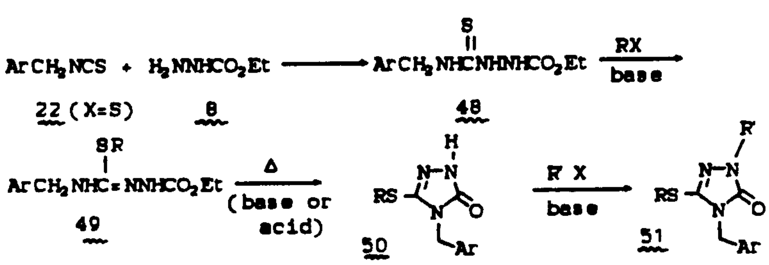


For the synthesis of compounds of formula (I) wherein E = -S-, Reaction Schemes 13 and 14 may be utilized. In Reaction Scheme 13, the isothiocyanate 22 is reacted with ethyl carbazate (8) to give the 1-(carbethoxy)thiosemicarbazide 48. By standard conditions, 48 is S-alkylated to yield 49, which can be cyclized to the triazolinone 50 by heating, optionally in the presence of base or acid [F. Kurzer and D.R. Hanks, Chem. Ind. (London), 1143 (1966)]. Finally, alkylation of the triazolinone as in Reaction Scheme 1 provides the fully substituted product 51.

## REACTION SCHEME 14

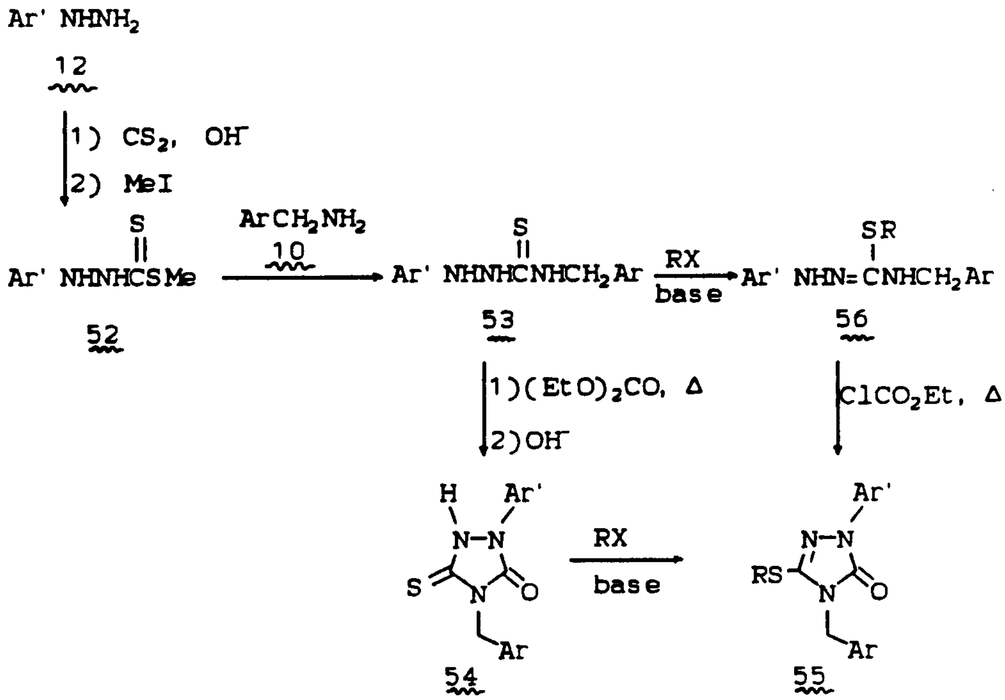


Following the chemistry of K. Sasse [Liebigs Ann. Chem., 735, 158 (1970)](Reaction Scheme 14), an arylhydrazine 12 is treated with carbon disulfide in the presence of base followed by treatment with methyl iodide to give the dithiocarbamoyl derivative 52. Reaction of 52 with the (arylmethyl)amine 10 yields the 1,4-disubstituted thiosemicarbazide 53. Cyclization of 53 to 54 is accomplished in two steps by first heating with diethyl carbonate and then treating with hydroxide to induce ring closure. Further treatment of 54 with an alkyl halide gives the desired S-alkyl triazolinone 55. A modification allowing the synthesis of compounds analogous to 55 in which the "Ar" substituent is replaced by an alkyl (or aralkyl) group has also been described by Sasse (see reference above). In a variation [method of A. Dornow and H. Paucksch, Chem. Ber., 99, 85 (1966)], 53 may be first S-alkylated to give 56, which can be cyclized to 55 upon

heating with ethyl chloroformate.

## REACTION SCHEME 15

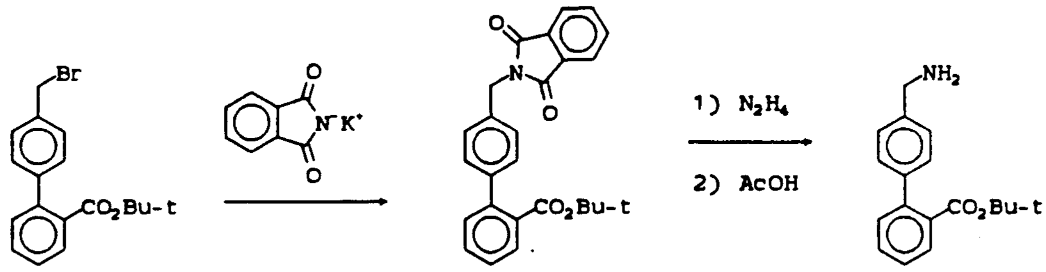


57 58 59

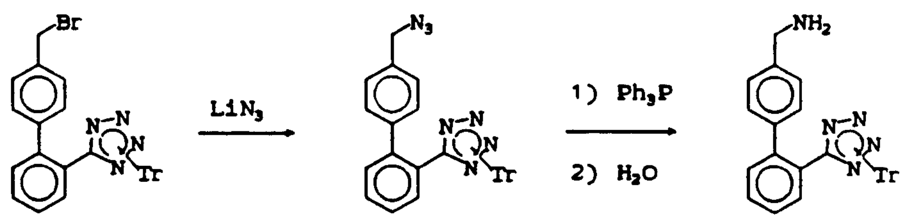


60 61 62

## REACTION SCHEME 15 (CONT'D)

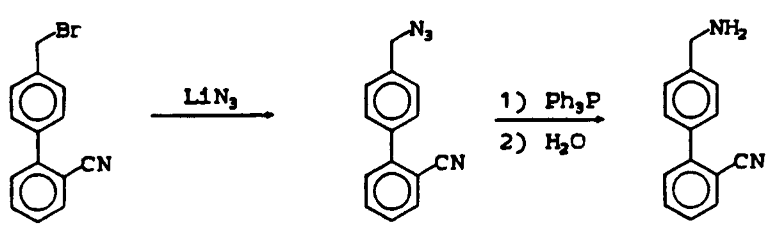


63 64 65

Reaction Scheme 15 shows routes to key intermediates used for incorporation of a (2′-carboxybiphenyl-4-yl)methyl or [2′-(5-tetrazolyl)biphenyl-4-yl]methyl substituent into a 2,4-dihydro-3H-1,2,4-triazol-3-one or -triazole-3-thione at the 4-position. One starting material, 4-bromomethyl-2′-(t-butoxycarbonyl)biphenyl (57), can be prepared as described in European Patent Application 253,310. Treatment of 57 with potassium phthalimide at room temperature in a suitable solvent such as N,N-dimethylformamide gives the phthalimido product 58, which is converted to the amine 59 by a standard hydrazinolysis procedure. Alternatively, using the methods described in European Patent Application 253,310, 57 may be treated with sodium azide in dimethylformamide, and the resulting azide intermediate may be reduced tn the amine 59 by hydrogenation in the presence of palladium catalyst or by other methods known in the literature. After conversion of 57 or 59 to a triazolinone, triazolinethione, or triazolinimine by methods illustrated in the previous schemes, the t-butyl ester is readily deprotected by treatment with trifluoroacetic acid at room temperature.

Transformation of 5-[4′-(bromomethyl)biphenyl-2-yl]-N-trityltetrazole (60) (prepared as in European Patent Application 291,969 to the azido intermediate 61 is accomplished by standard means such as treatment with lithium azide in dimethyl sulfoxide at room temperature. Reduction of 61 to the amine 62 proceeds readily using the conditions of M. Vaultier, N. Knouzi, and R. Carrie [Tetrahedron Lett., 24, 763 (1983)] (triphenylphosphine in tetrahydrofuran followed by water). By use of the methods outlined in previous schemes, 60 or 62 can be converted to a triazolinone, triazolinethione, or triazolinimine. Removal of the trityl protecting group from the tetrazole is achieved by warming in aqueous acetic acid.

Alternatively, 4-bromomethyl-2′-cyanobiphenyl (63) (described in European Patent Application 253,310) can be converted to the azide intermediate 64. Reduction of 64 by the method described above for the synthesis of 62 gives the amine 65. After conversion of 63 or 65 to a triazolinone, triazolinethione, or triazolinimine by methods illustrated in the previous schemes, the cyano substituent may be converted to the desired 5-tetrazolyl group by reaction with trimethyltin azide at elevated temperature in a suitable solvent such as toluene or xylene according to methods described in European Patent Application 291,969. Final destannylation to the free tetrazole may be accomplished by treatment with silica gel.

Although specific examples have been shown for the synthesis of compounds of formula (I) wherein X is a single bond, these methods are readily extended to the preparation of compounds of formula (I) having other X linkages allowed by the specifications. Depending on the nature of X, this linkage may be constructed either before or after assembly of the triazole ring. The construction of heterocyclic side chains analogous to the $N^4$ side chain of compounds of formula (I), in which variations of the X group are exemplified, has been disclosed in European Patent Application 253,310.

## REACTION SCHEME 16

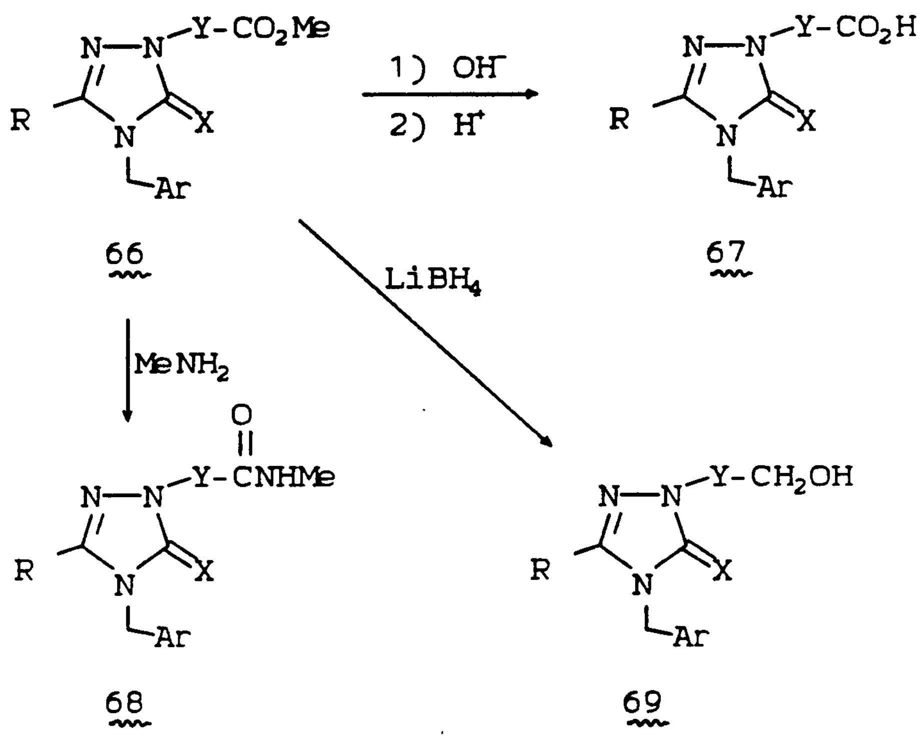


66 67 68 69

wherein:
Y represents an alkyl, aryl, heteroaryl, or aralkyl group bearing the designated substituent (i.e., carbomethoxy, carboxy, etc.)

Further transformations of substituent functional groups can be carried out after assembly of the triazole ring and either before or after full elaboration of the arylmethyl substituent at $N^4$. Typical examples are shown in Reaction Scheme 16. Thus the methyl ester of 66 can be saponified by treatment with aqueous sodium hydroxide (optionally in the presence of a cosolvent such as alcohol, tetrahydrofuran, or dioxane) at room temperature to give, after acidification, the acid 67. The N-methyl amide 68 is readily obtained by reaction of 66 with excess aqueous methylamine at room temperature in the presence of a cosolvent such as methanol. Reduction of the methyl ester 66 to the alcohol 69 can be accomplished by treatment with lithium borohydride in a solvent such as tetrahydrofuran. These examples are in no way exclusive of other functional group transformations which can be accomplished after formation of the triazolinone, triazolinethione, or triazolinimine system, and which will be apparent to anyone skilled in the art.

## REACTION SCHEME 17

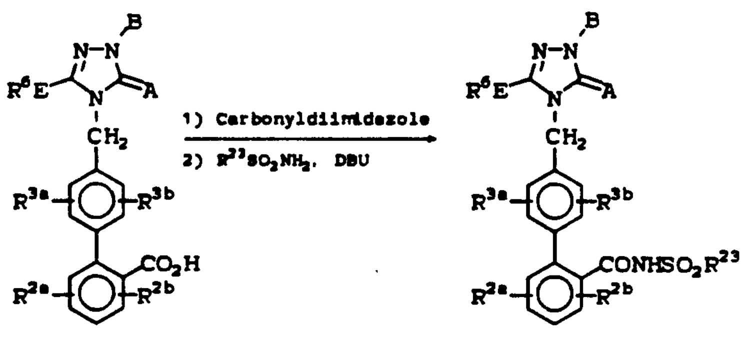


70 71

Alternative Methods:

a) (i) $SOCl_2$, $\Delta$ (ii) $R^{23}SO_2NH^-M^+$ (where M is Na or Li)
b) (i) $(COCl)_2$/DMF,-20°C (ii) $R^{23}SO_2NH^-M^+$
c) (i) N-(N,N-Diphenylcarbamoyl)pyridinium chloride/aq. NaOH (ii) $R^{23}SO_2NH^-M^+$.

Compounds of formula (I) wherein $R^1$ is $-CONHSO_2R^{23}$ (wherein $R^{23}$ is substituted or unsubstituted alkyl, aryl, or heteroaryl) may be prepared from the corresponding carboxylic acid derivatives (70) as outlined in Reaction Scheme 17. The carboxylic acid 70, obtained as described in Reaction Scheme 15 and preceding schemes (followed by deprotection of the t-butyl ester with trifluoroacetic acid), can be converted into the corresponding acid chloride by treatment with thionyl chloride at reflux or, preferably, with oxalyl chloride and a catalytic amount of dimethylformamide at low temperature [A.W. Burgstahler, et al., Synthesis, 767 (1976)]. The acid chloride can then be treated with the alkali metal salt of $R^{23}SO_2NH_2$ to form the desired acylsulfonamide 71. Alternatively, 71 may be prepared from 70 using N,N-diphenylcarbamoyl anhydride intermediates [F.J. Brown, et al., European Patent Application EP 199,543; K.L. Shepard and W. Halczenko, J. Heterocycl. Chem., 16, 321 (1979)]. Preferably, the carboxylic acid 70 is treated with carbonyldiimidazole to give an acyl-imidazole intermediate, which can then be treated with an appropriate aryl- or alkylsulfonamide in the

presence of 1,8-diazabicyclo[5.4.0]undec-7-ene (DBU) to give the desired acylsulfonamide 71.

## SCHEME 18

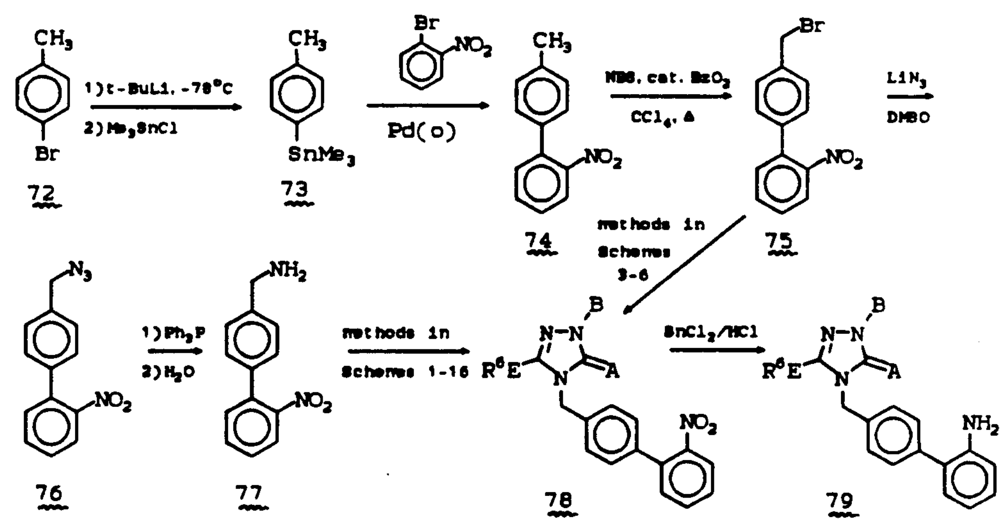


72 73 74 75 76 77 78 79

## REACTION SCHEME 18 (CONT'D)

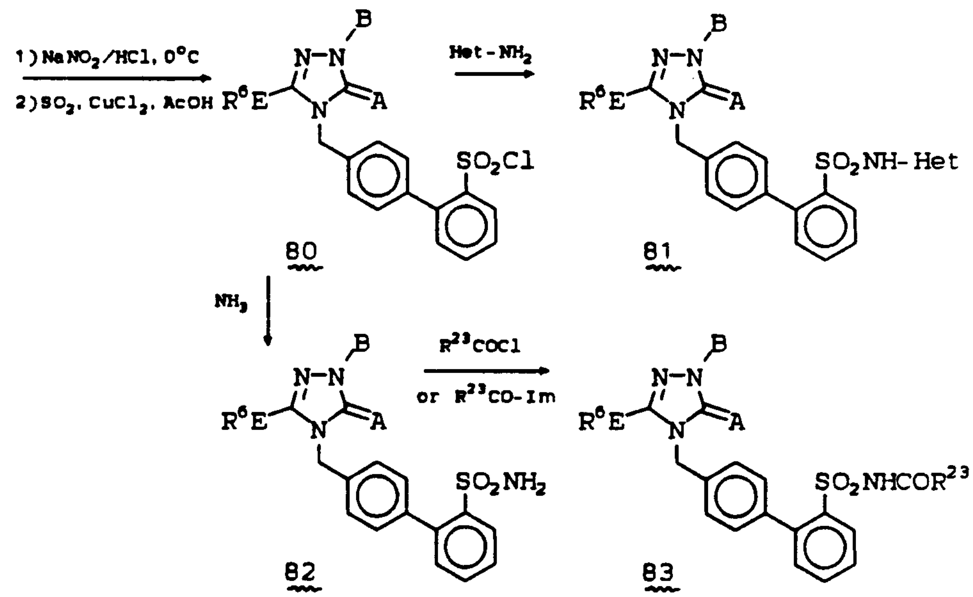


80

81

82

83

where

NBS = N-bromosuccinimide

Bz = benzoyl

Het = heteroaryl

Im = l-imidazolyl.

The preparation of compounds of formula (I) wherein $R^1$ is $-SO_2NH$-heteroaryl or $-SO_2NHCOR^{23}$ is outlined in Reaction Scheme 18. p-Bromotoluene (72) is converted to the trimethylstannane derivative 73 [S.M. Moerlein, J. Organometal. Chem., 319, 29 (1987)], which may be coupled with o-bromonitrobenzene in the presence of $(Ph_3P)_4Pd$ or $(Ph_3P)_2PdCl_2$ catalyst to give the biphenyl derivative 74. Such couplings have been described by J.K. Stille, Pure Appl. Chem., 57, 1771 (1985); T.R. Bailey, Tetrahedron Lett., 27, 4407 (1986); and D.A. Widdowson and Y-Z. Zhang, Tetrahedron, 42, 2111 (1986). Bromination of 74 with N-bromosuccinimide in the presence of catalytic benzoyl peroxide gives 75, which upon treatment with lithium azide in DMSO yields the azido derivative 76. Reduction of 76 to the amine 77 may be accomplished by treatment with triphenylphosphine followed by water. In an alternative route, the bromo group of 75 may be displaced by potassium phthalimide. Hydrazinolysis of the phthalimide derivative yields 77.

By the methods described in the previous schemes, the amine 77 can be converted to a variety of triazolinones, triazolinethiones, and triazolinimines of the general formula 78. Certain triazolinones, especially those in which B is aryl or heteroaryl, may be made directly from 75 by alkylation of a pre-formed triazolinone as in Reaction Schemes 3-6. Reduction of the nitro group of 78, preferably with stannous chloride/hydrochloric acid gives the amino derivative 79.

Diazotization of the amine 79 and reaction of the diazonium salt with sulfur dioxide in the presence of cupric chloride affords the corresponding arylsulfonyl chloride 80 [see H. Meerwein, et al., Chem. Ber., 90, 841 (1957); A.J. Prinsen and H. Cerfontain, Rec. Trav. Chim., 84, 24 (1965); E.E. Gilbert, Synthesis, 3 (1969); and references cited therein]. Treatment of the sulfonyl chloride 80 with an appropriate heteroaryl amine provides the N-heteroaryl sulfonamide 81. Reaction of the sulfonyl chloride with ammonia yields the sulfonamide 82, which is then treated with an appropriate acylating agent (such as an acid chloride, a carbamoyl chloride, or an acyl-imidazole derivative) to give the acylsulfonamide product 83.

## SCHEME 19

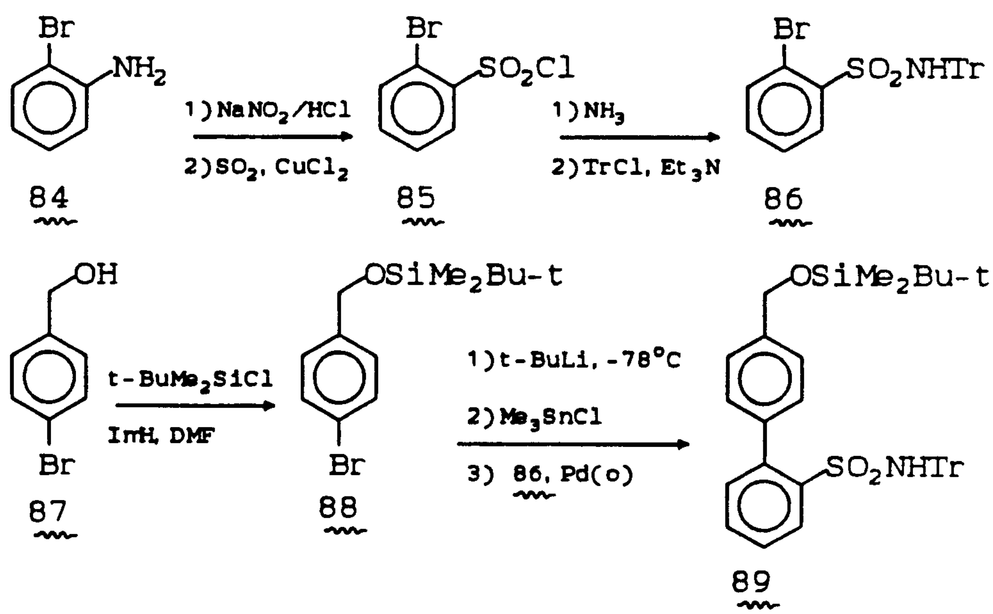


84 85 86 87 88 89

REACTION SCHEME 19 (CONT'D)

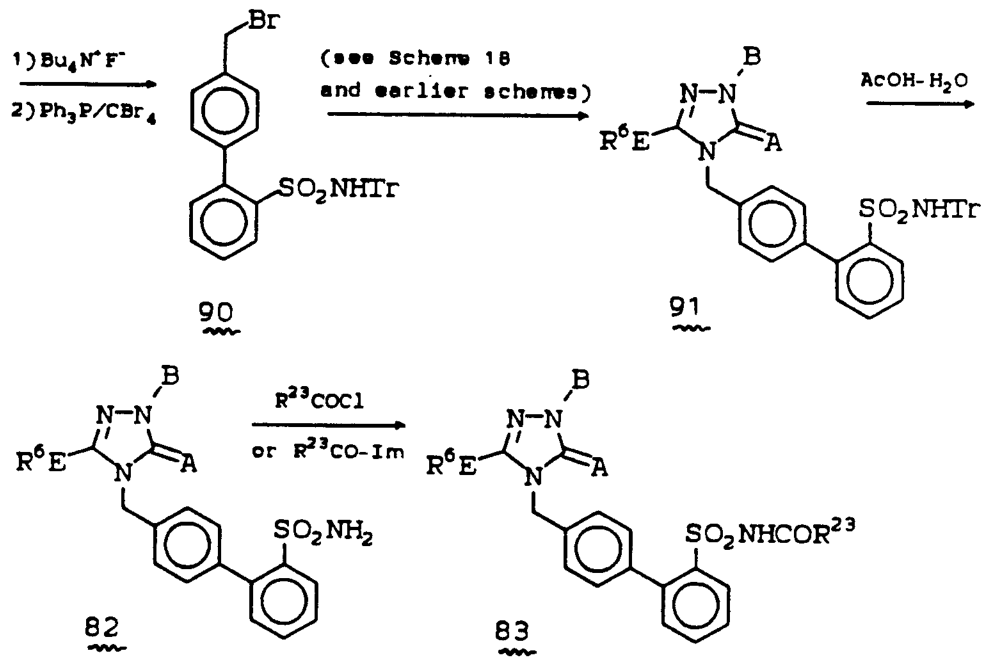


90 91 82 83

where
Tr = trityl (i.e., triphenylmethyl)
Im = l-imidazolyl.

Reaction Scheme 19 shows an alternative sequence leading to 83 in which a protected sulfonamide is present at the time of the biaryl coupling. By the methods described above, o-bromoaniline (84) is converted to the corresponding sulfonyl chloride 85. Treatment of 85 with ammonia and then with trityl chloride in the presence of triethylamine yields the N-trityl sulfonamide 86. p-Bromobenzyl alcohol (87) is t-butyldimethylsilylated, and the resulting 88 is coupled with 86 under the conditions described above to give the biphenyl product 89. The silyl group is removed with tetrabutylammonium fluoride, and treatment of the alcohol with triphenylphosphine/carbon tetrabromide gives the bromo derivative 90. Using the methods of Scheme 18 and earlier schemes, 90 may be transformed into a variety of triazoles of the general formula 91. The trityl protecting group is removed with aqueous acetic acid to give the free sulfonamide 82 which is acylated to yield the target 83 as in Scheme 18.

It will be appreciated by those skilled in the art that the protecting groups used in these syntheses will be chosen to be compatible with subsequent reaction conditions. Ultimately, they will be removed to generate the active compounds of formula (I). For example, $R^1$ as carboxyl is often protected as its t-butyl ester which in the last step is removed by treatment with trifluoroacetic acid. Aqueous acetic acid employed overnight is a preferred method to remove a trityl protecting group to liberate an $R^1$ tetrazole group.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases; e.g., dicyclohexylamine salts, N-methyl-D-glucamine salts, salts with amino acids like arginine, lysine, and the like. Also, salts with organic and inorganic acids may be prepared; e.g., HCl, HBr, $H_2SO_4$, $H_3PO_4$, methanesulfonic, toluensulfonic, maleic, fumaric, camphorsulfonic. The non-toxic, physiologically, acceptable salts are preferred, although other salts are also useful; e.g., in isolating or purifying the product.

The salts can be formed by conventional means such as by reacting the free acid or free base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Angiotensin II (A II) is a powerful arterial vasoconstrictor, and it exerts its action by interacting with specific receptors present on cell membranes. The compounds described in the present invention act as competitive antagonists of A II at the receptors. In order to identify A II antagonists and determine their efficacy in vitro, the following two ligand-receptor binding assays were established.

Receptor binding assay using rabbit aortae membrane preparation:

Three frozen rabbit aortae (obtained from Pel-Freeze Biologicals) were suspended in 5mM Tris-0.25M Sucrose, pH 7.4 buffer (50 ml) homogenized, and then centrifuged. The mixture was filtered through a cheesecloth and the supernatant was centrifuged for 30 minutes at 20,000 rpm at 4°C. The pellet thus obtained was resuspended in 30 ml of 50mM Tris-5 mM $MgCl_2$ buffer containing 0.2% Bovine Serum Albumin and 0.2 mg/ml Bacitracin and the suspension was used for 100 assay tubes. Samples tested for screening were done in duplicate. To the membrane preparation (0.25 ml) there was added $^{125}I$-Sar$^1$ Ile$^8$-angiotensin II [obtained from New England Nuclear] (10μl; 20,000 cpm) with or without the test sample and the mixture was incubated at 37°C for 90 minutes. The mixture was then diluted with ice-cold 50mM Tris-0.9% NaCl, pH 7.4 (4ml) and filtered through a glass fiber filter (GF/B Whatman 2.4" diameter). The filter was soaked in scintillation cocktail (10 ml) and counted for radioactivity using Packard 2660 Tricarb liquid scintillation counter. The inhibitory concentration ($IC_{50}$) of potential A II antagonist which gives 50% displacement of the total specifically bound $^{125}I$-Sar$^1$ Ile$^8$-angiotensin II was presented as a measure of the efficacy of such compounds as A II antagonists.

Receptor assay using Bovine adrenal cortex preparation

Bovine adrenal cortex was selected as the source of A II receptor. Weighed tissue (0.1 g is needed for 100 assay tubes) was suspended in Tris.HCl (50mM), pH 7.7 buffer and homogenized. The homogenate was centrifuged at 20,000 rpm for 15 minutes. Supernatant was discarded and pellets resuspended in buffer [$Na_2HPO_4$ (10mM)-NaCl (120mM)-disodium EDTA (5mM) containing phenylmethane sulfonyl fluoride (PMSF)(0.1mM)]. (For screening of compounds, generally duplicates of tubes are used). To the membrane preparation (0.5 ml) there was added $^3H$-angiotensin II (50mM) (10μl) with or without the test sample and the mixture was incubated at 37°C for 1 hour. The mixture was then diluted with Tris buffer (4ml) and filtered through a glass fiber filter (GF/B Whatman 2.4" diameter). The filter was soaked in scintillation cocktail (10ml) and counted for radioactivity using Packard 2660 Tricarb liquid scintillation counter. The inhibitory concentration ($IC_{50}$) of potential A II antagonist which gives 50% displacement of the total specifically bound $^3H$-angiotensin II was presented as a measure of the efficacy of such compounds as A II antagonists.

The potential antihypertensive effects of the compounds described in the present invention may be evaluated using the methodology described below: Male Charles River Sprague-Dawley rats (300-375 gm) were anesthetized with methohexital (Brevital; 50 mg/kg i.p.) and the trachea was cannulated with PE 205 tubing. A stainless steel pithing rod (1.5 mm thick, 150 mm long) was inserted into the orbit of the right eye and down the spinal column. The rats were immediately placed on a Harvard Rodent Ventilator (rate - 60 strokes per minute, volume - 1.1 cc per 100 grams body weight). The right carotid artery was ligated, both left and right vagal nerves were cut, and the left carotid artery was cannulated with PE 50 tubing for drug administration, and body temperature was maintained at 37°C by a thermostatically controlled heating pad which received input from a rectal temperature probe. Atropine (1 mg/kg iv.) was then administered, and 15 minutes later propranolol (1 mg/kg i.v.). Thirty minutes later angiotensin II or other agonists were administered intravenously at 30-minute intervals and the increase in the diastolic blood pressure was recorded before and after drug or vehicle administration.

Using the methodology described above, representative compounds of the invention were evaluated and all were found to exhibit an activity of at least $IC_{50}<50\mu M$ thereby demonstrating and confirming the utility of the compounds of the invention as effective A II antagonists.

Thus, the compounds of the invention are useful in treating hypertension. They are also of value in the management of acute and chronic congestive heart failure, in the treatment of secondary hyperaldosteronism, primary and secondary pulmonary hyperaldosteronism, primary and secondary pulmonary hypertension, renal failure such as diabetic nephropathy, glomerulonephritis, scleroderma, and the like, renal vascular hypertension, left ventricular dysfunction, diabetic retinopathy, and in the management of vascular disorders such as migraine or Raynaud's disease. The application of the compounds of this invention for these and similar disorders will be apparent to those skilled in the art.

The compounds of this invention are also useful to treat elevated intraocular pressure and can be administered to patients in need of such treatment with typical pharmaceutical formulations such as tablets, capsules, injectables and the like as well as topical ocular formulations in the form of solutions, ointments, inserts, gels, and the like. Pharmaceutical formulations prepared to treat intraocular pressure would typically contain about 0.1% to 15% by weight, preferably 0.5% to 2% by weight, of a compound of this invention.

In the management of hypertension and the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 1 to 1000 mg. per patient per day which can be administered in single or multiple doses. Perferably, the dosage range will be about 2.5 to 250 mg. per patient per day; more preferably about 2.5 to 75 mg. per patient per day.

The compounds of this invention can also be administered in combination with other antihypertensives and/or diuretics and/or angiotensin converting enzyme inhibitors and/or calcium channel blockers. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methyclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, verapamil, and the like, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the angiotensin II antagonists of this invention effective clinically in the 2.5-250 milligrams per day range can be effectively combined at levels at the 0.5-250 milligrams per day range with the following compounds at the indicated per day dose range: hydrochlorothiazide (15-200 mg) chlorothiazide (125-2000 mg), ethacrynic acid (15-200 mg), amiloride (5-20 mg), furosemide (5-80 mg), propranolol (20-480 mg), timolol maleate (5-60 mg.), methyldopa (65-2000 mg), felodipine (5-60 mg), nifedipine (5-60 mg), and nitrendipine (5-60 mg). In addition, triple drug combinations of hydrochlorothiazide (15-200 mg) plus amiloride (5-20 mg) plus angiotensin II antagonist of this invention (3-200 mg) or hydrochlorothiazide (15-200 mg) plus timolol maleate (5-60) plus an angiotensin II antagonist of this invention (0.5-250 mg) or hydrochlorothiazide (15-200 mg) and nifedipine (5-60 mg) plus an angiotensin II antagonist of this invention (0.5-250 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 1 to 100 mg. of compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unitform is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occuring vegetable oil like sesame

oil, coconut oil, peanut oil, cottonseed oil, etc., or a synthetic fatty vehicle like ethyl oleate or the like. Buffers, preservatives, antioxidants and the like can be incorporated as required.

The following examples illustrate the preparation of the compounds of formula (I) and their incorporation into pharmaceutical compositions and as such are not to be considered as limiting the invention set forth in the claims appended hereto.

EXAMPLE 1

5-n-Butyl-4-[(2′-carboxybiphenyl-4-yl)methyl]-2,4-dihydro-3H-1,2,4-triazole-3-thione

Step A: N-[[2′-(t-Butoxycarbonyl)biphenyl-4-yl]methyl]phthalimide

A mixture of 2.99 g (8 mmole, based on 93% purity) of 4-bromomethyl-2′-(t-butoxycarbonyl)biphenyl (EP 253,310), 1.63 g (8.8 mmole) of potassium phthalimide, and 24 ml of dry dimethylformamide (DMF) was stirred at room temperature for 7 hours and then partitioned between 200 ml of ether and 250 ml of $H_2O$. The organic phase was washed with 4x250 ml of $H_2O$, then dried ($MgSO_4$), filtered, and concentrated. The residue was leached twice with hot ether (15-20 ml), which was decanted off after cooling. The remaining solid was collected on a filter, washed with petroleum ether, and dried to yield 2.08g of colorless crystals, mp 108.5-109°, homogeneous by TLC in 4:1 hexane-EtOAc. The residue from evaporation of the mother liquor was triturated with two portions of ether to give a second crop of colorless crystals: 0.58 g, mp 122-123° (preliminary softening). Despite the difference in melting point, the second crop was identical to the first by NMR and TLC. The total yield was thus 2.66 g (82%).

| Analysis ($C_{26}H_{23}NO_4$) | | | |
|---|---|---|---|
| Calcd: | C, 75.53; | H, 5.61; | N, 3.39 |
| Found: | C, 75.25; | H, 5.75; | N, 3.18 |

300 MHz NMR ($CDCl_3$) δ 1.17 (s, 9H), 4.90 (s, 2H), 7.2-7.9 (m, 12 H).

Step B: 4-Aminomethyl-2′-(t-butoxycarbonyl)biphenyl

A mixture of 2.62 g (6.35 mmole) of N-[[2′-(t-butoxycarbonyl)biphenyl-4-yl]methyl]phthalimide, 1.21 ml (1.25 g, 25 mmole) of 100% hydrazine hydrate, and 35 ml of absolute ethanol was stirred at room temperature for 7.5 hours. During this time all of the solid gradually dissolved, followed by precipitation. Glacial acetic acid (3.7 ml) was added, and stirring was continued overnight. The white solid was then removed by filtration, and the filtrate was concentrated at room temperature. The residual oil was taken up in 100 ml of ether and washed with 2x50 ml of saturated aqueous $Na_2CO_3$ solution. Next, the product was extracted by shaking the ethereal solution with 50 ml of 0.5 N HCl. The aqueous layer was separated and basified by addition of excess saturated $Na_2CO_3$. The product, which oiled out, was extracted with 100 ml of ether. The other phase was dried ($Na_2SO_4$), filtered, and concentrated at 30°C to give 1.58 g (88%) of a very pale yellow, viscous oil, homogeneous by TLC in 95:5:0.5 $CH_2Cl_2$-MeOH-concd. $NH_4OH$.

| Analysis ($C_{18}H_{21}NO_2 \cdot 0.25\ H_2O$) | | | |
|---|---|---|---|
| Calcd: | C, 75.10; | H, 7.53; | N, 4.87 |
| Found: | C, 75.14; | H, 7.39; | N, 4.78 |

300 MHz NMR ($CDCl_3$)δ 1.27 (s, 9H), 1.50 (br s, 2H), 3.92 (s, 2H), 7.2-7.8 (m, 8H).

Step C: Methyl N-[[2′-(t-Butoxycarbonyl)biphenyl-4-yl]methyl]dithiocarbamate

A solution of 1.415 g (5 mmole) of 4-aminomethyl-2′-(t-butoxycarbonyl)biphenyl and 751 µl (545 mg, 5.4 mmole) of triethylamine in 5 ml of methanol was stirred under $N_2$ at room temperature as a solution of 342 µl (434 mg, 5.7 mmole) of carbon disulfide in 2 ml of methanol was added dropwise over about 10 minutes. After 2.5 hours the solution was cooled in an ice-methanol bath, and a solution of 311 µl (710 mg, 5 mmole) of methyl iodide in 2 ml of methanol was added dropwise over about 10 minutes. The cooling bath was removed, and the solution was allowed to warm to room

temperature. After 2 hours the solution was concentrated at 25°C. The residue was partitioned between 50 ml of ether plus 10 ml of $CH_2Cl_2$ and 50 ml of 0.2 N HCl. The organic phase was washed with 25 ml of saturated NaCl solution (aqueous), dried over $MgSO_4$, filtered, and concentrated. Crystallization of the residual oil from ether yielded 1.57 g (84%) of nearly colorless crystals, mp 127.5-128.5°C, satisfactory purity by TLC in 4:1 hexane-EtOAc; mass spectrum (FAB) m/e 374 $(M+1)^+$.

| Analysis ($C_{20}H_{23}NO_2S_2$) | | | |
|---|---|---|---|
| Calcd: | C, 64.31; | H, 6.21; | N, 3.75. |
| Found: | C, 64.54; | H, 6.46; | N, 3.82. |

300 MHz NMR ($CDCl_3$)δ 1.28 (s, 9H), 2.66 (s, 3H), 4.97 (d, J= 5Hz, 2H), 7.13 (br, m 1H), 7.2-7.8 (m, 8H).

Step D: 4-[[2′-(t-Butoxycarbonyl)biphenyl-4-yl]methyl]-3-thiosemicarbazide

A mixture of 1.53 g (4.1 mmole) of methyl N-[[2′-(t-butoxycarbonyl)biphenyl-4-yl]methyl]dithiocarbamate, 796 µl (820 mg, 16.4 mmole) of hydrazine hydrate, and 10 ml of absolute ethanol was stirred at reflux under $N_2$. After 2 hours the resulting solution was cooled and concentrated. The residual oil was chromatographed on a column of silica gel (elution with 99:1 and the 98:2 $CH_2Cl_2$) to give (after concentration and vacuum-drying) 1.15 g (79%) of a stiff, white foam, mp > 45°C (gradual); homogeneous by TLC in 19:1 $CH_2Cl_2$-MeOH; mass spectrum (FAB) m/e 358 $(M+1)^+$.

| Analysis ($C_{19}H_{23}N_3O_2S \cdot 0.1\ H_2O$) | | | |
|---|---|---|---|
| Calcd: | C, 63.51; | H, 6.51; | N, 11.70. |
| Found: | C, 63.41; | H, 6.50; | N, 11.54. |

300 MHz NMR ($CDCl_3$)δ 1.28 (s, 9H), 3.76 (br s, 2H), 4.90 (d, J= 5Hz, 2H), 7.2-7.8 (m, 9H).

Step E: 4-[[2′-(t-Butoxycarbonyl)biphenyl-4-yl]-5-n-butyl-2,4-dihydro-3H-1,2,4-triazole-3-thione

A solution of 1.11 g (3.1 mmole) of 4-[[2′-(t-butoxycarbonyl)biphenyl-4-yl)methyl]-3-thiosemicarbazide and 792 µl (745 mg, 4.6 mmole) of trimethyl orthovalerate in 10 ml of 2-methoxyethanol was stirred at reflux under $N_2$ for 15 hours. The cooled solution was concentrated, and the residue was purified by column chromatography on silica gel (gradient elution with 0-1% methanol in $CH_2Cl_2$) to give a gum which could be crystallized by trituration with petroleum ether. The total yield was 828 mg (63%), mp 135-137°C, homogeneous by TLC in 19:1 $CH_2Cl_2$-MeOH; mass spectrum (FAB) m/e 424 $(M+1)^+$.

| Analysis ($C_{24}H_{29}N_3O_2S$) | | | |
|---|---|---|---|
| Calcd: | C, 68.05; | H, 6.90; | N, 9.92. |
| Found: | C, 67.95; | H, 6.65; | N, 9.84. |

300 MHz NMR ($CDCl_3$)δ 0.87 (t, J= 7Hz, 3H), 1.22 (s, 9H), 1.32 (m, 2H), 1.62 (m, 2H), 2.48 (t, J= 7Hz, 2H), 5.27 (s, 2H), 7.2-7.5 (m, 7H), 7.74 (d, J= 8 Hz, 1H)

Step F: 5-n-Butyl-4-[(2′-carboxybiphenyl-4-yl)methyl]-2,4-dihydro-3H-1,2,4-triazole-3-thione

A solution of 51 mg (0.12 mmole) of 4-[[2′-(t-butoxycarbonyl)biphenyl-4-yl]methyl]-5-n-butyl-2,4-dihydro-3H-1,2,4-triazole-3-thione in 0.5 ml of anhydrous trifluoroacetic acid was stirred under $N_2$ at room temperature for 2 hours and then evaporated to dryness under a stream of $N_2$. The residue was dissolved in a small volume of methanol and evaporated onto 1 g of silica gel. This was layered on top of a column of silica gel (43x2.4 cm) packed in $CH_2Cl_2$. Gradient elution with 1-5% methanol in $CH_2Cl_2$ containing 0.1% acetic acid eluted two major products. Concentration of fractions containing the first (higher $R_f$) product gave a residue which solidified upon trituration with ether: 9.5 mg (21%) of white

powder, mp 218-219°C, homogeneous by TLC in 95:5:0.1 $CH_2Cl_2$-MeOH-AcOH; mass spectrum (FAB) m/e 368 $(M+1)^+$.

| Analysis ($C_{20}H_{21}N_3O_2S \cdot 0.5\ H_2O$) | | | |
|---|---|---|---|
| Calcd: | C, 63.80; | H, 5.89; | N, 11.16. |
| Found: | C, 63.93; | H, 5.86; | N, 10.82. |

300 MHz NMR (DMSO-$d_6$)δ 0.79 (t, J= 7.5 Hz, 3H), 1.25 (m, 2H), 1.46 (m, 2H), 2.53 (partially obsured t, J= 8Hz, 2H), 5.29 (s, 2H), 7.25-7.4 (m, 5H), 7.45 (t, J= 8Hz, 1H), 7.57 (t, J= 8Hz, 1H), 7.72 (d, J= 8Hz, 1H), 12.7 (v br s, 1H)

Concentration of fractions containing the second (lower $R_f$) product and work-up as above gave 21.9 mg of a white powder, mp 166.5-168°C dec., identified as 3-n-butyl-5-(t-butylthio)-4-[(2′-carboxybiphenyl-4-yl)methyl]-4H-1,2,4-triazole, a by-product arising from t-butyl migration.

EXAMPLE 2

5-n-Butyl-2,4-dihydro-4-[[2′-(5-tetrazolyl)biphenyl-4-yl]methyl]-3H-1,2,4-triazol-3-one

Step A: Ethyl Valerate Carbethoxyhydrazone

To a solution of 7.0 g (25.3 mmole) of ethyl valerimidate hydrochloride [prepared by method of A.J. Hill and I. Rabinowitz, J. Am. Chem. Soc., 48, 734 (1926)] in 35 ml of dry ethanol stirred under $N_2$ at -78°C was added dropwise a solution of 24 g (23 mmole) of ethyl carbazate in 35 ml of dry ethanol. Precipitation occurred during the addition, which took 20 minutes and was accompanied by a rise in the internal temperature to -50°C. The mixture was allowed to stand at 5°C for 60 hours and then filtered. The filtrate was concentrated, and the residue was flash chromatographed on a silica gel column (elution with 98.5:1.5 $CH_2Cl_2$-MeOH), yielding 3.06 g (61%) of a clear oil, homogeneous by TLC in 97:3 $CH_2Cl_2$-MeOH; mass spectrum (FAB) m/e 217 $(M+1)^+$. NMR suggested a mixture of syn and anti isomers.
200 MHz NMR ($CDCl_3$)δ 0.91 (t, J= 7Hz, 3H), 1.2-1.4 (m, 8 H), 1.4-1.6 (m, 2H), 2.2-2.4 (m, 2H), 3.95-4.3 (m, 4H), 6.91, 8.11 (br s, 1H total).

Step B: 5-[4′-(Azidomethyl)biphenyl-2-yl)-N-trityltetrazole

To a stirred suspension of 11.15 g (20 mmole) of 5-[4′-(bromomethyl)biphenyl-2-yl]-N-trityltetrazole [P.E. Aldrich, M.E. Pierce, and J.J.V. Duncia, European Patent Application 291,969 (1988)] in 55 ml of dry DMSO was added 1.23 g (25 mmole) of freshly pulverized lithium azide, and the mixture was stirred at room temperature under $N_2$. Within a few minutes virtually all of the solid had dissolved, accompanied by a mild exotherm, and this was followed immediately by precipitation of product. After 4 hours the solid was collected on a filter and washed with some methanol, then with a relatively large volume of $H_2O$, and finally again with methanol. The solid was air-dried overnight and then dried further in a vacuum oven at 70°C. (< 1 mm) to give 8.60 g (83%) of white crystals, mp 139-140.5°C. dec., satisfactory purity by TLC (9:1 hexane-ethyl acetate) for use in the next step. From the combined filtrate and washes was recovered a less pure second crop (1.68 g of cream-colored crystals, mp 129-132°C. dec.), which was also usable in the next step. Mass spectrum (FAB) m/e 243 (trityl cation); IR (Nujol) 2100 $cm^{-1}$.
300 MHz NMR ($CDCl_3$)δ 4.22 (s, 2H), 6.90 (d, J= 8Hz, 6H), 7.04, 7.14 (d, J= 8Hz, each 2H), 7.2-7.55 (m, 12H), 7.96 (dd, J= 8, 1Hz, 1H).

Step C: 5-[4′-(Aminomethyl)biphenyl-2-yl)-N-trityltetrazole

A solution of 10.2g (19.6 mmole) of 5-[4′-(azidomethyl)biphenyl-2-yl]-N-trityltetrazole in 39.4 ml of dry tetrahydrofuran (THF) was stirred under $N_2$ at room temperature as 5.16g (19.7 mmole) of triphenylphosphine was added in small portions over a period of about 10 minutes. After 2 hours, by which time gas evolution had ceased, 532 µl (532 mg, 29.6 mmole) of $H_2O$ was added. After an additional 23 hours, the solution was concentrated in vacuo to give a pale golden gum. This material was chromatographed on a column of silica gel (50x8.5 cm) packed in $CH_2Cl_2$. The column was eluted with a gradient of 0-6% methanol in $CH_2Cl_2$. Concentration of the product fractions gave a foam which solidified upon trituration with ether. This material was collected on a filter, washed further with some ether, and dried in vacuo at 50°C to give 5.65 g (58%) of white crystals, mp 134-136°C dec., satisfactory purity by TLC in 95:5:0.5 $CH_2Cl_2$-MeOH-

concd. $NH_4OH$; mass spectrum (FAB) m/e 243 (trityl cation), 494 $(M+1)^+$.

| Analysis ($C_{33}H_{27}N_5 \cdot 0.5\ H_2O$) | | | |
|---|---|---|---|
| Calcd: | C, 78.86; | H, 5.61; | N, 13.94. |
| Found: | C, 78.76; | H, 5.70; | N, 13.89. |

300 MHz NMR ($CDCl_3$)δ 1.44 (br s, 2H, overlapping $H_2O$ peak), 3.75 (s, 2H), 6.88 (d, J= 8Hz, 6H), 7.03, 7.09 (d, J= 8Hz, each 2H), 7.2-7.5 (m, 12 H), 7.95 (dd, J= 8, 1 Hz, 1H).

Step D: 5-n-Butyl-2,4-dihydro-4-[[2′-(N-trityltetrazol-5-yl)biphenyl-4-yl]methyl]-3H-1,2,4-triazol-3-one

A mixture of 1.20g (2.43 mmole) of 5-[4′-(aminomethyl)biphenyl-2-yl]-N-trityltetrazole, 683 mg (3.16 mmole) of ethyl valerimidate carbethoxyhydrazone, and 5 ml of ethanol was stirred under $N_2$ in an oil bath at 80°C. All of the solid dissolved within 15 minutes, and precipitation began after about 2 hours. After 3.5 hours the mixture was cooled and concentrated. The residue was re-concentrated from $CH_2Cl_2$ and then flash chromatographed on a column containing 400 cc of silica gel. Gradient elution with 1-5% methanol in $CH_2Cl_2$ afforded 628 mg (42%) of a white powder, mp 176.5-177.5°C, homogeneous by TLC in 9:1 $CH_2Cl_2$-MeOH; mass spectrum (FAB) m/e 618 $(M+1)^+$.
300 MHz NMR ($CDCl_3$)δ 0.83 (t, J= 7 Hz, 3H), 1.26 (m, 2H), 1.51 (m, 2H), 227 (t, J= 7.5 Hz, 2H), 4.69 (s, 2H), 6.90 (d, J= 7.5 Hz, 6H), 6.99, 7.13 (d, J= 8Hz, each 2H), 7.2-7.5 (m, 12H), 7.91 (dd, J= 8, 1.5Hz, 1H), 9.03 (s, 1H).

Step E: 5-n-Butyl-2,4-dihydro-4-[[2′-(5-tetrazolyl)biphenyl-4-yl]methyl]-3H-1,2,4-triazol-3-one

A mixture of 34 mg (0.055 mmole) of 3-n-butyl-2,4-dihydro-4-[[2′-(N-trityltetrazol-5-yl)biphenyl-4-yl]methyl]-3H-1,2,4-triazol-3-one, 0.9 ml of glacial acetic acid, and 0.4 ml of $H_2O$ was stirred overnight at 55°C. The cooled, filtered solution was concentrated in vacuo, and the residue was re-concentrated from toluene. The resulting residue was triturated with ether, which (after centrifugation) was removed by decantation. This procedure was repeated 4x until TLC indicated no remaining trityl alcohol. The product residue was concentrated from methanol-chloroform, then from $CH_2Cl_2$, and finally from toluene to give 18 mg (86%) of white powder, mp 215-216°C., homogeneous by TLC in 90:10:0.1 $CH_2Cl_2$-MeOH-AcOH; mass spectrum (FAB) m/e 376 $(M+1)^+$.

| Analysis ($C_{20}H_{21}N_7O \cdot 0.4\ H_2O$) | | | |
|---|---|---|---|
| Calcd: | C, 62.78; | H, 5.74; | N, 25.62. |
| Found: | C, 62.96; | H, 5.65; | N, 25.24. |

300 MHz NMR ($CDCl_3$)δ 0.86 (t, J= 7.5 Hz, 3H), 1.33 (m, 2H), 1.59 (m, 2H), 2.44 (t, J= 7.5 Hz, 2H), 4.78 (s, 2H), 7.10 (m, 4H), 7.4-7.6 (m, 3H), 7.88 (d, J= 8Hz, 1H).

EXAMPLE 3

2-Benzyl-5-n-butyl-2,4-dihydro-4-[[2′-(5-tetrazolyl)biphenyl-4-yl]methyl]-3H-1,2,4-triazol-3-one

Step A: 2-Benzyl-5-n-butyl-2,4-dihydro-4-[[2′-(N-trityltetrazol-5-yl)biphenyl-4-yl]methyl]-3H-1,2,4-triazol-3-one

A mixture of 33 mg (0.053 mmole) of 5-n-butyl-2,4-dihydro-4-[[2′-(N-trityltetrazol-5-yl)biphenyl-4-yl]methyl]-3H-1,2,4-triazol-3-one, 8.1 mg (0.169 mmole) of sodium hydride (50% in oil), and 130 µl of dry DMF was stirred under $N_2$ at room temperature for 2 hours. Next, 30 µl (43 mg, 0.252 mmole) of benzyl bromide was added, and stirring at room temperature was continued for an additional 1.5 hours, during which time the mixture decolorized. The mixture was quenched by careful addition of 0.8 ml of $H_2O$ and then shaken with 1.5 ml of ethyl acetate. The aqueous fraction was extracted further with 2x3 ml of ethyl acetate. The combined ethyl acetate fractions were washed with 2x2 ml of $H_2O$ and then with saturated aqueous NaCl. The organic phase was dried over $Na_2SO_4$, filtered, and concentrated. Flash chromatography of the residue on a column of 40 cc of silica gel (elution with 0.6% methanol in $CH_2Cl_2$) gave 32.5 mg

(85%) of a white powder, homogeneous by TLC in 99:1 $CH_2Cl_2$-MeOH, mass spectrum (FAB) m/e 708 $(M+1)^+$.

| Analysis ($C_{46}H_{41}N_7O \cdot 0.2\ CH_2Cl_2$) | | | |
|---|---|---|---|
| Calcd: | C, 76.55; | H, 5.98; | N, 13.53. |
| Found: | C, 76.91; | H, 5.67; | N, 13.07. |

300 MHz NMR ($CDCl_3$)δ 0.80 (t, J= 7Hz, 3H), 1.23 (m, 2H), 1.46 (m, 2H), 2.24 (t, J= 7.5 Hz, 2H), 4.69, 4.97 (s, each 2H), 6.90 (d, J= 7Hz, 6H), 6.98, 7.08 (d, J= 8Hz, each 2H), 7.2-7.9 (m, 17H), 7.91 (dd, J= 8, 1.5 Hz, 1H).

Step B: 2-Benzyl-5-n-butyl-2,4-dihydro-4-[[2′-(5-tetrazolyl)biphenyl-4-yl]methyl]-3H-1,2,4-triazol-3-one

To a solution of 27.5 mg (0.0389 mmole) of 2-benzyl-5-n-butyl-2,4-dihydro-4-[[2′-(N-trityltetrazol-5-yl)biphenyl-4-yl]-3H-1,2,4-triazol-3-one in 0.9 ml of glacial acetic acid was added dropwise 0.4 ml of $H_2O$. The mixture was heated to 55°C for 4 hours and then allowed to stand at room temperature for 3 days. The solvent was removed in vacuo, and the residue was re-concentrated from toluene. Purification by flash chromatography on a column containing 30 cc of silica gel (gradient elution with 5-20% methanol in $CH_2Cl_2$) and subsequent concentration from toluene gave 17 mg (86%) of white, foamy solid, mp>111.5°C (gradual), homogeneous by TLC in 9:1 $CH_2Cl_2$-MeOH and 90:10:0.1 $CH_2Cl_2$-MeOH-AcOH; mass spectrum (FAB) m/e 466 $(M+1)^+$.

| Analysis [$C_{27}H_{27}N_7O \cdot 0.75\ H_2O \cdot 0.3\ C_7H_8$ (toluene)] | | | |
|---|---|---|---|
| Calcd: | C, 68.98; | H, 6.14; | N, 19.35. |
| Found: | C, 69.25; | H, 6.12; | N, 19.15. |

300 MHz NMR ($CDCl_3$)δ 0.86 (t, J= 7.5Hz, 3H), 1.33 (m, 2H), 1.57 (m, 2H), 2.42 (t, J= 8Hz, 2H), 4.79, 4.90 (s, each 2H), 7.16 (apparent s, 4H), 7.2-7.6 (m, 8H), 8.05 (d, J= 8Hz, 1H).

EXAMPLE 4

5-n-Butyl-2,4-dihydro-2-phenyl-4-[[2′-(5-tetrazolyl)biphenyl-4-yl]methyl-3H-1,2,4-triazol-3-one

Step A: Ethyl Valerimidate (Free Base)

A 12.7 g (76.7 mmole) sample of ethyl valerimidate hydrochloride [prepared from valeronitrile, ethanol, and hydrogen chloride gas as described by A.J. Hill and I. Rabinowitz, J. Am. Chem. Soc., 48, 734 (1926)] was dissolved in 33% (w/w) potassium carbonate solution (made by dissolving 15 g of $K_2CO_3$ in 30 ml of $H_2O$) and immediately extracted with ether (3x40 ml). The combined ether layers were dried over $Na_2SO_4$, filtered, and concentrated in vacuo to give 7.09 g (72%) of the product as a clear oil, which was used directly in the next step.
300 MHz NMR ($CDCl_3$)δ 0.88 (t, J= 7Hz, 3H), 1.24 (t, J= 7Hz, 3H), 1.31 (m, 2H), 1.50 (m, 2H), 2.19 (t, J= 7.5Hz, 2H), 4.06 (q, J= 7Hz, 2H), 6.84 (br s, 1H).

Step B: Ethyl N-Carbethoxyvalerimidate

A solution of 6.5 g (50.3 mmole) of ethyl valerimidate (free base) in 90 ml of dry $CH_2Cl_2$ was treated with 7.71 ml (5.60 g, 55.3 mmole) of triethylamine. The resulting solution was stirred under $N_2$ at -10°C in an ice-salt bath as a solution of 4.81 ml (5.46 g, 50.3 mmole) of ethyl chloroformate in 10 ml of $CH_2Cl_2$ was added dropwise over 25 minutes. Upon completion of the addition, the cooling bath was removed, and the mixture was stirred at room temperature for 2 hours. Next, the solvent was removed by evaporation in vacuo. The residue was taken up in hexane and filtered to remove triethylamine hydrochloride. Concentration of the filtrate yielded 7.08 g (70%) of the product as a yellow oil, suitable for use in the next step without further purification. NMR indicated a mixture of syn and anti isomers. TLC (98:2 $CH_2Cl_2$-MeOH) showed a close pair of spots, $R_f$ 0.48, 0.52; mass spectrum (EI) m/e 201 $(M^+)$.
200 MHz NMR ($CDCl_3$)δ 0.86 (distorted t, J= 7.5Hz, 3H), 2.15-2.35 (m, 8H), 2.4-2.65 (m, 2H), 2.19, 2.35 (t, J= 7.5Hz, 2H total), 4.0-4.2 (m, 4H).

Step C: 5-n-Butyl-2,4-dihydro-2-phenyl-3H-1,2,4-triazol-3-one

To a solution of 197 µl (216 mg, 2.0 mmole) of phenylhydrazine in 3 ml of toluene was added 442 mg (2.2 mmole) of ethyl N-carbethoxyvalerimidate, and the mixture was heated at 45-50°C for 1.5 hours. At this time 307 µl (223 mg, 2.2 mmole) of triethylamine was added, and the bath temperature was raised to 95°C. After being maintained at this temperature overnight, the dark red solution was cooled and concentrated in vacuo. Flash chromatography of the residue on silica gel (elution with 0.5% methanol in $CH_2Cl_2$) gave 252 mg (58%) of the product as an off-white solid, mp 107.5-109°C., homogeneous by TLC in 19:1 $CH_2Cl_2$-MeOH; mass spectrum (FAB) m/e 218 $(M+1)^+$.

| Analysis [$C_{12}H_{15}N_3O \cdot 0.1\ H_2O \cdot 0.1\ C_7H_8$ (toluene)] | | | |
|---|---|---|---|
| Calcd: | C, 66.82; | H, 7.06; | N, 18.41. |
| Found: | C, 66.59; | H, 6.89; | N, 18.02. |

200 MHz NMR ($CDCl_3$)δ 0.96 (t, J= 7Hz, 3H), 1.44 (m, 2H), 1.74 (m, 2H), 2.64 (t, J= 7.5Hz, 2H), 7.24 (d, J= 8Hz, 1H), 7.44 (t, J= 8Hz, 2H), 7.95 (d, J= 8Hz, 2H), 11.8 (br s, 1H).

Step D: 5-n-Butyl-2,4-dihydro-2-phenyl-4-[[2′-(N-trityltetrazol-5-yl)biphenyl-4-yl]methyl]-3H-1,2,4-triazol-3-one

A mixture of 100 mg (0.461 mmole) of 5-n-butyl-2,4-dihydro-2-phenyl-3H-1,2,4-triazol-3-one, 18.5 mg (0.461 mmole) of sodium hydride (60% in oil), and 0.5 ml of dry DMF was stirred under $N_2$ at 40-50°C for 20 minutes. After $H_2$ evolution was complete, the mixture was cooled to 35°C., and a solution of 257 mg (0.461 mmole) of 5-[4′-(bromomethyl)biphenyl-2-yl]-N-trityltetrazole in 1.0 ml of DMF was added. The resulting mixture was stirred at 35°C for 2.5 hours and then concentrated in vacuo. The residue was treated with 10 ml of $H_2O$, and the product was extracted by shaking with 3x12 ml of ethyl acetate. The combined organic fractions were washed with $H_2O$ and then with saturated NaCl. The organic phase was dried over $Na_2SO_4$, filtered, and concentrated. Flash chromatography of the residue on silica gel (gradual elution with 20-40% ethyl acetate in hexane) gave 238 mg (74%) of the product as a foam, mp > 69.5°C (gradual), homogeneous by TLC in 99:1 $CH_2Cl_2$-MeOH; mass spectrum (FAB) m/e 694 $(M+1)^+$. Eluted after the product was 22 mg of unreacted 5-n-butyl-2,4-dihydro-2-phenyl-3H-1,2,4-triazol-3-one. Based on recovered starting material, the product yield was 96%.
200 MHz NMR ($CDCl_3$)δ 0.88 (t, J= 7Hz, 3H), 1.33 (m, 2H), 1.61 (m, 2H), 2.40 (t, J= 7.5Hz, 2H), 4.80 (s, 2H), 6.93 (d, J= 7.5Hz, 6H), 7.06, 7.12 (d, J= 8Hz, each 2H), 7.2-7.5 (m, 15H), 7.94 (d, J= 8Hz, 1H), 8.04 (d, J= 8Hz, 2H).

Step E: 5-n-Butyl-2,4-dihydro-2-phenyl-4-[[2′-(5-tetrazolyl)biphenyl-4-yl]methyl-3H-1,2,4-triazol-3-one

A mixture of 70 mg (0.101 mmole) of 5-n-butyl-2,4-dihydro-2-phenyl-4-[[2′-(N-trityltetrazol-5-yl)biphenyl-4-yl]methyl]-3H-1,2,4-triazol-3-one, 1.6 ml of glacial acetic acid, and 1.0 ml of $H_2O$ was stirred at room temperature for 3.5 days. After removal of solvents by evaporation in vacuo, the residue was leached with hexane and a small volume of ether and then flash chromatographed on silica gel (gradient elution with 5-10% methanol in $CH_2Cl_2$) to give 29 mg (61%) of an off-white solid, mp 187-188°C., homogeneous by TLC in 9:1 $CH_2Cl_2$-MeOH; mass spectrum m/e 452 $(M+1)^+$.

| Analysis ($C_{26}H_{25}N_7O \cdot 1.1\ H_2O$) | | | |
|---|---|---|---|
| Calcd: | C, 66.33; | H, 5.82; | N, 20.82. |
| Found: | C, 66.45; | H, 5.43; | N, 20.42. |

300 MHz NMR (DMSO-$d_6$)δ 0.84 (t, J= 7.5Hz, 3H), 1.32 (m, 2H), 1.54 (m, 2H), 2.53 (partially obscured t, J= 7.5Hz, 2H), 4.93 (s, 2H), 7.10, 7.23 (d, J= 8Hz, each 2H), 7.4-7.7 (m, 8H), 7.93 (d, J= 8Hz, 1H).

EXAMPLE 5

5-n-Butyl-2-(2-chlorophenyl)-2,4-dihydro-4-[[2′-(5-tetrazolyl)biphenyl-4-yl]methyl]-3H-1,2,4-triazol-3-one

Step A: 5-n-Butyl-2-(2-chlorophenyl)-2,4-dihydro-3H-1,2,4-triazol-3-one

By the procedure of Example 4, Step C, o-chlorophenylhydrazine (generated from the hydrochloride by partitioning between ether and 1N $Na_2CO_3$) was reacted with N-carbethoxyvalerimidate. After work-up, the residue was purified by flash chromatography on silica gel (gradient elution with 0.6-2% methanol in $CH_2Cl_2$) to give a 51% yield of the product as an off-white solid, mp 103-104°C., homogeneous by TLC in 19:1 $CH_2Cl_2$-MeOH; mass spectrum (FAB) m/e 252 $(M+1)^+$.

| Analysis ($C_{12}H_{14}ClN_3O$) | | | |
|---|---|---|---|
| Calcd: | C, 57.26; | H, 5.61; | N, 16.69. |
| Found: | C, 57.31; | H, 5.69; | N, 16.58. |

200 MHz NMR ($CDCl_3$)δ 0.92 (t, J= 7Hz, 3H), 1.38 (m, 2H), 1.68 (m, 2H), 2.57 (t, J= 7.5Hz, 2H), 7.3-7.55 (m, 4H), 12.04 (br s, 1H).

Step B: 5-n-Butyl-2-(2-chlorophenyl)-2,4-dihydro-4-[[2′-(N-trityltetrazol-5-yl)biphenyl-4-yl]methyl]-3H-1,2,4-triazol-3-one

The alkylation of 5-n-butyl-2-(2-chlorophenyl)-2,4-dihydro-3H-1,2,4-triazol-3-one with 5-[4′-(bromomethyl)biphenyl-2-yl]-N-trityltetrazole was carried out as described in Example 4, Step D, except that a 5% excess of sodium hydride and an 8% excess of the bromo compound were used. After work-up, flash chromatography using a relatively large volume of silica gel (150 cc for 0.5 mmole; gradient elution with 20-40% ethyl acetate in hexane) provided a 69% yield of the product as a glassy solid, mp > 69°C (gradual), homogeneous by TLC in 99:1 $CH_2Cl_2$-MeOH; mass spectrum (FAB) m/e 728 $(M+1)^+$.
300 MHz NMR ($CDCl_3$)δ 0.83 (t, J= 7Hz, 3H), 1.28 (m, 2H), 1.56 (m, 2H), 2.35 (t, J= 7.5Hz, 2H), 4.78 (s, 2H), 6.90 (d, J= 7.5Hz, 6H), 7.06, 7.12 (d, J= 8Hz, each 2H), 7.2-7.55 (m, 16H), 7.93 (d, J= 8Hz)

Step C: 5-n-Butyl-2-(2-chlorophenyl)-2,4-dihydro-4-[[2′-(5-tetrazolyl)biphenyl-4-yl]methyl]-3H-1,2,4-triazol-3-one

Deprotection of 5-n-butyl-2-(2-chlorophenyl)-2,4-dihydro-4-[[2′-(N-trityltetrazol-5-yl)biphenyl-4-yl]methyl]-3H-1,2,4-triazol-3-one in 80% acetic acid (aqueous) was accomplished by the procedure of Example 4, Step E, except that the mixture was kept at 65°C overnight. After concentrating the mixture and azeotroping 3x with toluene, the residue was flash chromatographed on silica gel (40 g for 0.18 mmole; gradient elution with 5-10% methanol in $CH_2Cl_2$). Upon final concentration in vacuo from toluene, a 66% yield of the product was obtained as a glassy solid, mp > 87°C (gradual), homogeneous by TLC in 90:10:0.1 $CH_2Cl_2$-MeOH-AcOH; mass spectrum (FAB) m/e 486 $(M+1)^+$.

| Analysis [$C_{26}H_{24}ClN_7O \cdot 0.75\ H_2O \cdot 0.33\ C_7H_8$ (toluene)] | | | |
|---|---|---|---|
| Calcd: | C, 64.18; | H, 5.35; | N, 18.49. |
| Found: | C, 64.07; | H, 5.18; | N, 18.31. |

300 MHz NMR ($CDCl_3$)δ 0.87 (t. J= 7.5Hz, 3H), 1.37 (m, 2H), 1.63 (m, 2H), 2.49 (t, J= 7.5Hz, 2H), 4.86 (s, 2H), 7.1-7.6 (m, 11H), 7.97 (dd, J= 7.5Hz, 1H)

EXAMPLE 6

5-n-Butyl-2-[2-(carbomethoxy)benzyl]-2,4-dihydro-4-[[2′-(5-tetrazolyl)biphenyl-4-yl]methyl]-3H-1,2,4-triazol-3-one

Step A: 5-n-Butyl-2-[2-(carbomethoxy)benzyl]-2,4-dihydro-4-[[2′-(N-trityltetrazol-5-yl)biphenyl-4-yl]methyl]-3H-1,2,4-triazol-3-one

By the procedure of Example 3, Step A, 5-n-butyl-2,4-dihydro-4-[[2′-(N-trityltetrazol-5-yl)biphenyl-4-yl]methyl]-3H-1,2,4-triazol-3-one was alkylated with methyl 2-(bromomethyl)benzoate [R.M. Scrowston and D.C. Shaw, J. Chem. Soc. Perkin Trans. I, 749 (1976)] to give a 72% yield of the titled compound as a foam, homogeneous by TLC in 98:2 $CH_2Cl_2$-MeOH; mass spectrum (FAB) m/e 766 $(M+1)^+$.

| Analysis ($C_{48}H_{43}N_7O_3 \cdot 0.8\ H_2O$) | | | |
|---|---|---|---|
| Calcd: | C, 73.88; | H, 5.77; | N, 12.56 |
| Found: | C, 73.99; | H, 5.51; | N, 12.38 |

300 MHz NMR ($CDCl_3$) δ 0.82 (t, J= 7.5Hz, 3H), 1.26 (m, 2H), 1.50 (m, 2H), 2.30 (t, J= 7.5Hz, 2H), 3.91 (s, 3H), 4.74 (s, 2H), 5.47 (s, 2H), 6.91 (d, J= 7Hz, 6H), 7.0-7.5 (m, 19H), 7.93, 7.99 (d, J= 8Hz, each 1H).

Step B: 5-n-Butyl-2-[2-(carbomethoxy)benzyl]-2,4-dihydro-4-[[2′-(5-tetrazolyl)biphenyl-4-yl]methyl]-3H-1,2,4-triazol-3-one

Detritylation of 5-n-butyl-2-[2-(carbomethoxy)benzyl]-2,4-dihydro-4-[[2′-(N-trityltetrazol-5-yl)biphenyl-4-yl]methyl]-3H-1,2,4-triazol-3-one was carried out according to the procedure of Example 3, Step B, except that the mixture was stirred initially at 35-40°C and then overnight at room temperature. Purification by flash chromatography on silica gel (gradient elution with 5-10% methanol in $CH_2Cl_2$) afforded a 95% yield of the titled compound as a white powder, mp 79-80°C, homogeneous by TLC in 9:1 $CH_2Cl_2$-MeOH and 90:10:0.1 $CH_2Cl_2$-MeOH-AcOH; mass spectrum (FAB) m/e 524 $(M+1)^+$.

| Analysis ($C_{29}H_{29}N_7O_3 \cdot 1.25\ H_2O$) | | | |
|---|---|---|---|
| Calcd: | C, 63.78; | H, 5.81; | N, 17.95 |
| Found: | C, 64.03; | H, 5.60; | N, 17.66 |

300 MHz NMR ($CDCl_3$) δ 0.86 (t, J= 7.5Hz, 3H), 1.33 (m, 2H), 1.58 (m, 2H), 2.44 (t, J= 7.5Hz, 2H), 3.88 (s, 3H), 4.84 (s, 2H), 5.39 (s, 2H), 7.05 (d, J= 8Hz, 1H), 7.15-7.6 (m, 9H), 7.95, 8.07 (d, J= 8Hz, each 1H).

EXAMPLE 7

5-n-Butyl-2-(2-carboxybenzyl)-2,4-dihydro-4-[[2′-(5-tetrazolyl)biphenyl-4-yl]methyl]-3H-1,2,4-triazol-3-one

To a solution of 20 mg (0.038 mmole) of 5-n-butyl-2-[2-(carbomethoxy)benzyl]-2,4-dihydro-4-[[2′-(5-tetrazolyl)biphenyl-4-yl]methyl]-3H-1,2,4- triazol-3-one in 200 µl of dry THF was added all at once 84 µl (0.084 mmole) of 1N sodium hydroxide in methanol. The mixture was stirred overnight at room temperature and then evaporated to dryness. The residual solid was dissolved in 1.5 ml of methanol and treated with 90 µl of 1N HCl in methanol (final pH~2). The solvent was evaporated, and the residue was leached with $CHCl_3$. After removal of insoluble solid (NaCl) by filtration through glass wool, the filtrate was concentrated in vacuo and then re-concentrated from toluene. The residue was dried overnight in the presence of $P_2O_5$ to give 20 mg (95%) of the titled compound as a stiff, white foam, mp > 106°C (gradual),

homogeneous by TLC in 90:10:0.1 $CH_2Cl_2$-MeOH-AcOH; mass spectrum (FAB) m/e 510 $(M+1)^+$.

| Analysis [$C_{28}H_{27}N_7O_3 \cdot 2H_2O \cdot 0.1C_7H_8$ (toluene)] | | | |
|---|---|---|---|
| Calcd: | C, 62.14; | H, 5.77; | N, 17.67 |
| Found: | C, 62.54; | H, 5.68; | N, 17.42 |

300 MHz NMR ($CDCl_3$) δ 0.84 (t, J= 7.5Hz, 3H), 1.30 (m, 2H), 1.55 (m, 2H), 2.43 (t, J= 7.5Hz, 2H), 4.77 (s, 2H), 5.27 (s, 2H), 7.10 (m, 4H), 7.2-7.6 (m, 6H), 7.92, 7.96 (d, J= 8Hz, each 1H).

EXAMPLE 8

5-n-Butyl-2,4-dihydro-2-[2-(hydroxymethyl)phenyl]-4-[[2′-(5-tetrazolyl)biphenyl-4-yl]methyl]-3H-1,2,4-triazol-3-one and 2-[2-(Acetoxymethyl)phenyl]-5-n-butyl-2,4-dihydro-4-[[2′-(5-tetrazolyl)biphenyl-4-yl]methyl]-3H-1,2,4-triazol-3-one.

Step A: 5-n-Butyl-2-[2-(carbomethoxy)phenyl]-2,4-dihydro-3H-1,2,4-triazol-3-one

By the procedure of Example 4, Step C, o-(carbomethoxy)phenylhydrazine [generated from the hydrochloride which was prepared according to H. Stroh and G. Westphal, Chem. Ber. 96, 184 (1963), by partitioning between ether and 5% aqueous sodium bicarbonate] was reacted with ethyl N-carbethoxyvalerimidate (Example 4, Step B). After work-up, the residue was purified by flash chromatography on silica gel (gradient elution with 0.6-2% methanol in $CH_2Cl_2$) to give a 51% yield of the product as a pale yellow oil, homogeneous by TLC (19:1 $CH_2Cl_2$-MeOH), mass spectrum (FAB) m/e 276 $(M+1)^+$.
200 MHz $^1$HNMR ($CDCl_3$) δ 0.93 (t, J=7.2Hz, 3H), 1.35 (m, 2H), 1.68 (m, 2H) 2.56 (t, J=7.8 Hz, 2H), 3.83 (s, 3H), 7.40 (m, 1H), 7.61 (d, J=3.7Hz, 2H) 7.90 (d, J=7.8Hz, 1H), 12.03 (s, 1H).

Step B: 5-n-Butyl-2-[2-(carbomethoxy)phenyl]-2,4-dihydro-4-[[2′-(N-trityltetrazol-5-yl)biphenyl-4-yl]methyl]-3H-1,2,4-triazol-3-one

The alkylation of 5-n-butyl-2-[2-(carbomethoxy)phenyl]-2,4-dihydro-3H-1,2,4-triazol-3-one with 5-[4′-(bromomethyl)biphenyl-2-yl]-N-trityltetrazole was carried out as described in Example 4, Step D, except that a 20% excess of sodium hydride and a 5% excess of the bromo compound were used, and the anion was generated during 2.5 hours at room temperature. After work-up, flash chromatography on silica gel (eluting with 0.6% methanol in $CH_2Cl_2$) provided a 65% yield of the product as a white foam, homogeneous by TLC in 99:1 $CH_2Cl_2$-MeOH; mass spectrum (FAB) m/e 752 $(M+1)^+$.
300 MHz $^1$HNMR ($CDCl_3$) δ 0.84 (t, J=7.3Hz, 3H), 1.30 (m, 2H), 1.60 (m, 2H), 2.35 (t, J=7.9Hz, 2H), 3.76 (s, 3H), 4.75 (s, 3H), 6.90 (m, 4H), 7.10 (m, 6H), 7.35 (m, 15H), 7.85 (m, 2H).

Step C: 5-n-Butyl-2,4-dihydro-2-[2-(hydroxymethyl)phenyl]-4-[[2′-(N-trityltetrazol-5-yl)biphenyl-4-yl]methyl]-3H-1,2,4-triazol-3-one

To a vigorously stirred solution of 59 mg (0.079 mmole) of 5-N-butyl-2-[2-(carbomethoxy)phenyl]-2,4-dihydro-4-[[(2′-N-trityltetrazol-5-yl)biphenyl-4-yl]methyl]-3H-1,2,4-triazol-3-one in 1.0 mL dry $CH_2Cl_2$ at -25°C was added dropwise 314 µL (0.314 mmole) of 1M diisobutylaluminum hydride in $CH_2Cl_2$. After stirring between -20° and -10°C for 75 minutes, the mixture was cooled to -50°C, treated with 0.75 mL methanol and warmed up to room temperature. Subsequently, the mixture was treated with 1.5mL of 2.5% NaOH, and extracted with 4 mL, $CH_2Cl_2$ 3 times. The combined organic layers were washed with 5 mL $H_2O$ twice followed by 5 mL brine, and dried over anhydrous $Na_2SO_4$. The residue obtained after evaporation of solvents was flash chromatographed over silica gel (eluting with 0.8% methanol in $CH_2Cl_2$) to yield 40 mg (70%) of a white foam, homogeneous by TLC in 98:2 $CH_2Cl_2$-MeOH, mass spectrum (FAB) m/e 724 $(M+1)^+$.
300 MHz $^1$HNMR ($CDCl_3$) δ 0.85 (t, J=7.3Hz, 3H), 1.30 (m, 2H), 1.53 (m, 2H), 2.39 (t, J=8.0Hz, 2H), 4.49 (d, J-6.1Hz, 2H), 4.79 (s, 2H), 6.90 (m, 7H), 7.10 (m, 5H), 7.40 (m, 14H), 7.92 (m, 1H).

Step D: 5-n-Butyl-2.4-dihydro-2-[2-(hydroxymethyl)phenyl]-4-[[(2'-(5-tetrazolyl)biphenyl-4-yl]methyl]-3H-1,2,4-triazol-3-one (A) and 2-[2-(Acetoxymethyl)phenyl]-5-n-butyl-2,4-dihydro-4-[[2'-(5-tetrazoly)biphenyl-4-yl]methyl]-3H-1,2,4-triazol-3-one (B)

A mixture of 35 mg (0.0484 mmole) of 5-n-butyl-2,4-dihydro-2-[2-(hydroxymethyl)phenyl]-4-[[2'-(N-trityltetrazol-5-yl)biphenyl-4-yl]methyl]-3H-1,2,4-triazol-3-one, 0.5mL of glacial acetic acid, and 0.25 mL of $H_2O$ was stirred at 60°C overnight. TLC in 90:10 $CH_2Cl_2$-MeOH showed two products at $R_f$<0.25. After cooling to room temperature, solvents were evaporated and the residue flash chromatographed over silica gel (gradient elution with 4-10% methanol in $CH_2Cl_2$) to give 7 mg of product B (the acetate) as an oil and 12 mg of product A (the hydroxy compound) as a clear oil. Each compound was homogeneous by TLC in 90:10 $CH_2Cl_2$-MeOH, mass spectrum (FAB) for A m/e 482 $(M+1)^+$ and for B m/e 524$(M+1)^+$.

A: 300 MHz $^1$HNMR ($CDCl_3$) δ 0.88 (t, J=7.3Hz, 3H), 1.37 (m, 2H), 1.64 (m, 2H), 2.51 (t, J=7.5Hz, 2H), 4.39 (s, 2H), 4.81 (s, 2H), 7.30 (m, 11H), 7.93 (m, 1H).

B: 300 MHz $^1$HNMR ($CDCl_3$) δ 0.90 (t, J=7.2Hz, 3H), 1.39 (m, 2H), 1.65 (m, 2H), 1.99 (s, 3H), 2.52 (t, J=8.1Hz, 2H), 4.83 (s, 2H), 5.02 (s, 2H), 7.40 (m, 11H), 7.95 (m, 1H)

EXAMPLE 9

5-n-Butyl-2,4-dihydro-2-[4-methylbenzyl]-4-[[2'-(5-tetrazolyl)biphenyl-4-yl]methyl]-3H-1,2,4,-triazol-3-one

Step A: 4-Azidomethyl-2'-cyanobiphenyl

A mixture of 1.97 g (725 mmole) of 4-bromomethyl-2'-cyanobiphenyl (EP 253,310), 445 mg (9.1 mmole) of lithium azide and 5 ml of dry DMSO was stirred at room temperature under nitrogen for one hour and then partitioned between 100 ml of ether and 100 ml of $H_20$. The organic phase was washed with 3 x 100 ml of $H_2O$, then dried ($MgSO_4$), filtered, and concentrated in vacuo to give a residual oil which solidified on standing. This solid was triturated with petroleum ether, collected on a filter, washed with petroleum ether and dried overnight to yield 1.15 g (61%) of the title compound as white crystals, mp 69-70°C; mass spectrum (EI) m/e 234 $(M^+)$. TLC in 4:1 hexane-EtOAc showed only minor impurities and the material was of sufficient purity to use in the next step.
300 MHz NMR ($CDCl_3$) δ 4.41 (s, 2H), 7.4-7.7 (m, 7H), 7.75 (d, J=8 Hz, 1H).

Step B: [(2'-Cyanobiphenyl-4-yl)methyl]amine

A solution of 5.85 g (25 mmole) of 4-azidomethyl-2'-cyanobiphenyl (from Step A) in 50 ml of dry tetrahydrofuran was treated portionwise with 6.55 g (25 mmole) of triphenylphospine over 3-4 minutes. The solution was stirred at ambient temperature under $N_2$, and gas evolution proceeded at a moderate rate. A mild exotherm occurred, and the solution was cooled in a water bath as necessary. After 2 hours, by which time gas evolution had ceased, 675 µl (37.5 mmole) of $H_2O$ was added, and stirring was continued at room temperature under $N_2$. After 22 hours, the solution was concentrated in vacuo, and the residual oil was chromatographed on a column of silica gel (gradient elution with 2-10% methanol in $CH_2Cl_2$). The residue from evaporation of the pooled product fractions was partitioned between ether-$CH_2Cl_2$ and saturated $Na_2CO_3$ (aqueous). The organic phase was dried ($Na_2SO_4$), filtered, and concentrated in vacuo to yield 4.64 g (89%) of air-sensitive, nearly white crystals, mp 54-55°C, homogeneous by TLC in 9:1 $CH_2Cl_2$-MeOH; mass spectrum (FAB) m/e 209$(M+1)^+$.

| Analysis ($C_{14}H_{12}N_2$) | | | |
|---|---|---|---|
| Calcd: | C, 80.74; | H, 5.81; | N, 13.45 |
| Found: | C, 80.53; | H, 5.89; | N, 13.12 |

300 MHz NMR($CDCl_3$) δ 1.50 (br s, 2H), 3.92 (s, 2H), 7.35-7.65 (m, 7H), 7.75 (d, J=8Hz, 1H)

Step C: 5-n-Butyl-4-[(2'-cyanobiphenyl-4-yl)methyl]-2,4-dihydro-3H-1,2,4-triazol-3-one

A mixture of 400 mg (1.923 mmole) of [(2'-cyanobiphenyl-4-yl)methyl]amine, 457 mg (2.12 mmole) of ethyl valerimidate carbethoxyhydrazone (from Example 2, Step A), and 7 mL of ethanol was stirred under $N_2$ in an oil bath at 50°C for 3 hours and then at 80°C for 2 days. The mixture was cooled and concentrated. The residue, re-concentrated from

toluene, was flash chromatographed over silica gel (gradient elution with 1.5-5% methanol in $CH_2Cl_2$) to give 591 mg (93%) of desired product. homogeneous in TLC (90:10 $CH_2Cl_2$-MeOH); mass spectrum (FAB) m/e 333 $(M+1)^+$.
300 MHz $^1$HNMR($CDCl_3$) δ 0.85 (t, J=7.2Hz, 3H), 1.35 (m, 2H), 1.60 (m, 2H), 2.42 (t, J=7.2Hz, 2H), 4.87 (s, 3H), 7.35 (d, J=7Hz, 2H), 7.50 (m, 4H), 7.62 (d, J=8.5Hz, 1H), 7.75 (d, J=8.5Hz, 1H), 9.13 (br s, 1H).

Step D: 5-n-Butyl-4-[(2′-cyanobiphenyl-4-yl)methyl]-2,4-dihydro-2-(4-methylbenzyl)-3H-1,2,4-triazol-3-one

The alkylation of 5-n-butyl-4-[(2′-cyanobiphenyl-4-yl)methyl]-2,4-dihydro-3H-1,2,4-triazol-3-one with 4-methylbenzyl bromide was carried out as described in Example 3, Step A, except that no excess sodium hydride was used. After work-up, flash chromatography of the crude product on silica gel (eluting with 0.6% methanol in $CH_2Cl_2$) provided a 76% yield of the title compound as a clear oil, homogeneous by TLC (98:2 $CH_2Cl_2$-MeOH), mass spectrum (FAB) m/e 437 $(M+1)^+$.
300 MHz $^1$HNMR (CDCl3) δ 0.84 (t, J=7.3Hz, 3H), 1.40 (m, 2H), 1.52 (m, 2H), 2.31 (s, 3H), 2.38 (m, 2H), 4.87 (s, 2H), 4.93 (s, 2H), 7.12 (d, J=7.8Hz, 2H), 7.26 (d, J=8.5Hz, 2H), 7.33 (d, J=8.2Hz, 2H), 7.50 (m, 4H), 7.62 (m, 1H), 7.74 (m, 1H).

Step E: 5-n-Butyl-2,4-dihydro-2-(4-methylbenzyl)-4-[[2′-(5-tetrazolyl)biphenyl-4-yl]methyl]-3H-1,2,4-triazol-3-one

A mixture of 56 mg (0.128 mmole) 5-n-butyl-4-[(2′-cyanobiphenyl-4-yl) methyl]-2,4-dihydro-2-(4-methylbenzyl)-3H-1,2,4-triazol-3-one, 2.0 mL dry toluene, and 134 mg (0.65 mmole) trimethyltin azide was stirred and heated at 60° under $N_2$ for 10 minutes, and then at 110° for 3 days. After cooling to room temperature, toluene was removed under reduced pressure and the residue was taken up in 1.5 mL methanol. The solution was treated with 0.75 g 230-400 mesh silica gel and stirred for 45 min. Upon removal of the methanol, the resulting powder was made into a slurry in $CH_2Cl_2$ and applied on a silica gel column, gradient eluted wih 4-10% methanol in $CH_2Cl_2$ to give 19 mg (13%) of a clear oil, homogeneous on TLC (90:10 $CH_2Cl_2$-MeOH), mass spectrum m/e 480 $(M+1)^+$.
300 MHz $^1$HNMR ($CDCl_3$) δ 0.81 (t, J=7.2Hz, 3H), 1.27 (m, 2H), 1.50 (m, 2H), 2.27 (s, 3H), 2.35 (m, 2H), 4.68 (s, 2H), 4.79 (s, 2H), 7.02 (m, 6H), 7.45 (m, 4H), 7.86 (d, J=7.6Hz, 1H), 8.03 (d, J=7.6Hz, 1H).
Additional 2,5-disubstituted -2,4-dihydro-4-[[2′-(5-tetrazolyl)biphenyl-4-yl]methyl-3H-1,2,4-triazol-3-ones prepared by

the methods described in the foregoing Examples are tabulated below.

## Table I

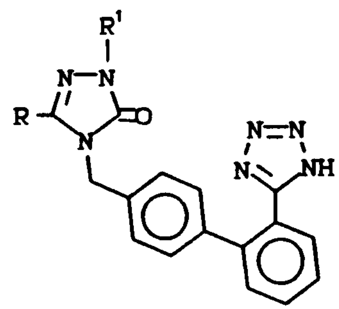

| | R | R' | mp | formula | | Analysis C | Analysis H | Analysis N |
|---|---|---|---|---|---|---|---|---|
| (1) | n-Bu | s-Bu | 79.81°C | $C_{24}H_{29}N_7O \cdot 0.5H_2O \cdot 0.25CHCl_3$ | calcd. | 61.92 | 6.48 | 20.84 |
| | | | | | found: | 61.92 | 6.39 | 20.63 |
| (2) | n-Bu | 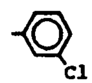 | 89-90°C | $C_{26}H_{24}ClN_7O \cdot 0.1\ CHCl_3$ | calcd. | 62.96 | 4.88 | 19.69 |
| | | | | | found: | 63.12 | 4.61 | 19.50 |
| (3) | n-Bu | 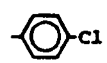 | 94-95°C | $C_{26}H_{24}ClN_7O \cdot 0.15\ CHCl_3$ | calcd. | 62.33 | 4.83 | 19.46 |
| | | | | | found: | 62.33 | 4.85 | 19.36 |

## Table 1 (Cont'd)

| | R | R' | mp | formula | | Analysis C | Analysis H | Analysis N |
|---|---|---|---|---|---|---|---|---|
| (4) | n-Bu | 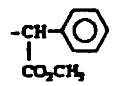 | > 87.5°C (gradual) | $C_{29}H_{29}N_7O_3 \cdot 1\ H_2O \cdot 0.1C_7H_8$ (toluene) | calcd. | 64.76 | 5.82 | 17.80 |
| | | | | | found: | 65.03 | 5.71 | 17.59 |
| (5) | n-Bu | 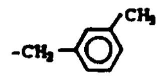 | 72-73.5°C | $C_{28}H_{29}N_2O \cdot 1/6C_7H_8 \cdot 1/5CHCl_3$ (toluene) | calcd. | 67.99 | 5.93 | 18.90 |
| | | | | | found: | 68.03 | 6.07 | 18.59 |
| (6) | n-Bu | 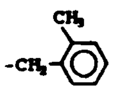 | 192-193.5°C | $C_{28}H_{29}N_7O \cdot 1/5C_7H_8 \cdot 1/8CHCl_3$ (toluene) | calcd. | 68.93 | 6.02 | 19.06 |
| | | | | | found: | 69.13 | 5.60 | 18.84 |
| (7) | n-Bu | 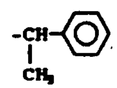 | 162.5-164°C | $C_{28}H_{29}N_7O \cdot 0.1\ CHCl_3$ | calcd. | 68.67 | 5.97 | 19.95 |
| | | | | | found: | 69.04 | 5.74 | 19.65 |
| (8) | n-Bu | 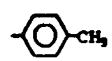 | > 88°C (gradual) | $C_{27}H_{27}N_7O \cdot 0.1\ CHCl_3$ | calcd. | 68.17 | 5.72 | 20.53 |
| | | | | | found: | 68.33 | 5.77 | 20.34 |
| (9) | n-Bu | 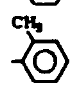 | > 82°C (gradual) | $C_{27}H_{27}N_7O \cdot 1/6\ CHCl_3$ | calcd. | 67.22 | 5.64 | 20.20 |
| | | | | | found: | 67.45 | 5.69 | 20.13 |
| (10) | n-Bu | 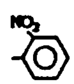 | > 91°C (gradual) | $C_{26}H_{24}N_8O_3 \cdot 1/3\ CHCl_3$ | calcd. | 58.97 | 4.57 | 20.89 |
| | | | | | found: | 59.23 | 4.65 | 20.89 |

## Table 1 (Cont'd)

| | R | R' | mp | Formula | | Analysis C | Analysis H | Analysis N |
|---|---|---|---|---|---|---|---|---|
| (11) | n-Bu | 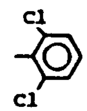 | > 102°C | $C_{26}H_{23}Cl_2N_7O\cdot 1/3H_2O$ | calcd. | 59.32 | 4.53 | 18.63 |
| | | | | | found: | 59.41 | 4.38 | 18.45 |
| (12) | n-Bu | 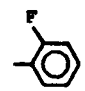 | > 86°C (gradual) | $C_{26}H_{24}FN_7O\cdot 0.4H_2O$ | calcd. | 65.53 | 5.24 | 20.58 |
| | | | | | found: | 65.70 | 5.29 | 20.44 |
| (13) | n-Bu | 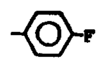 | > 87°C (gradual) | $C_{26}H_{24}FN_7O\cdot 0.4H_2O$ | calcd. | 65.53 | 5.24 | 20.58 |
| | | | | | found: | 65.72 | 5.19 | 20.56 |
| (14) | n-Bu | 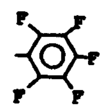 | 89-91°C | $C_{26}H_{20}F_5N_7O$ | calcd. | 57.67 | 3.72 | 18.11 |
| | | | | | found: | 57.39 | 3.83 | 17.81 |
| (15) | n-Bu | 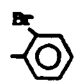 | > 98°C (gradual) | $C_{26}H_{24}BrN_7O\cdot 0.05CH_2Cl_2$ | calcd. | 58.52 | 4.54 | 18.34 |
| | | | | | found: | 58.35 | 4.61 | 18.05 |

## Table 1 (Cont'd)

| R | $R^1$ | mp | Formula | | Analysis C | Analysis H | Analysis N |
|---|---|---|---|---|---|---|---|
| (16) n-Bu | 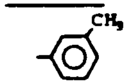 | 152-154.5°C | $C_{27}H_{27}N_7O \cdot 1/4C_7H_8 \cdot 1/2H_2O$ (toluene) | calcd. | 69.40 | 6.08 | 19.70 |
| | | | | found: | 69.54 | 5.97 | 19.62 |
| (17) n-Bu | 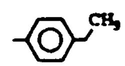 | > 87°C (gradual) | $C_{28}H_{29}N_7O \cdot 0.1H_2O$ | calcd. | 69.86 | 6.11 | 20.36 |
| | | | | found: | 69.70 | 5.94 | 20.13 |
| (18) n-Bu | 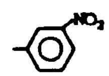 | > 92°C (gradual) | $C_{26}H_{24}N_8O_3 \cdot 0.7H_2O$ | calcd. | 61.34 | 5.03 | 22.01 |
| | | | | found: | 61.65 | 4.92 | 21.81 |
| (19) n-Bu | 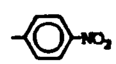 | > 96°C (gradual) | $C_{26}H_{24}N_8O_3 \cdot 1/8CH_2Cl_2$ | calcd. | 61.87 | 4.82 | 22.09 |
| | | | | found: | 61.98 | 4.95 | 21.74 |
| (20) n-Bu | 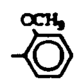 | > 100°C (gradual) | $C_{27}H_{27}N_7O_2 \cdot 1.4H_2O$ | calcd. | 63.99 | 5.92 | 19.35 |
| | | | | found: | 64.21 | 5.95 | 19.14 |

## Table 1 (Cont'd)

| | R | R' | mp | formula | | Analysis C | Analysis H | Analysis N |
|---|---|---|---|---|---|---|---|---|
| (21) | n-Bu | 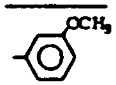 | > 78°C (gradual) | $C_{27}H_{27}N_7O_2 \cdot 0.1C_7H_8 \cdot 0.6H_2O$ (toluene) | calcd. | 66.33 | 5.83 | 19.55 |
| | | | | | found: | 66.59 | 5.95 | 19.18 |
| (22) | n-Bu | 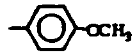 | > 84°C (gradual) | $C_{27}H_{27}N_7O_2 \cdot 0.25CH_2Cl_2$ | calcd. | 65.09 | 5.51 | 19.49 |
| | | | | | found: | 65.36 | 5.25 | 19.09 |
| (23) | n-Bu | 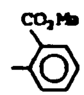 | > 84°C (gradual) | $C_{28}H_{27}N_7O_3 \cdot 0.4CH_2Cl_2$ | calcd. | 62.76 | 5.15 | 18.04 |
| | | | | | found: | 62.97 | 5.33 | 17.86 |
| (24) | n-Bu | 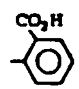 | 117-120°C | $C_{27}H_{25}N_7O_3 \cdot 0.2C_7H_8$ $1.5H_2O$ (toluene) | calcd. | 63.05 | 5.51 | 18.12 |
| | | | | | found: | 63.29 | 5.76 | 18.03 |
| (25) | n-Bu | 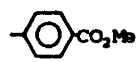 | > 94°C (gradual) | $C_{28}H_{27}N_7O_3 \cdot 1/2H_2O$ | calcd. | 64.85 | 5.44 | 18.90 |
| | | | | | found: | 65.01 | 5.45 | 18.51 |

Table I (Cont'd)

| R | R' | mp | formula | | Analysis C | Analysis H | Analysis N |
|---|---|---|---|---|---|---|---|
| (26) n-Bu | 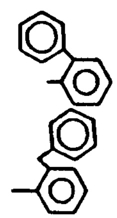 | > 94°C (gradual) | $C_{32}H_{29}N_7O \cdot 0.7CH_2Cl_2$ | calcd. | 66.90 | 5.22 | 16.70 |
| | | | | found: | 67.09 | 5.30 | 16.76 |
| (27) n-Bu | | > 84°C (gradual) | $C_{33}H_{31}N_7O \cdot 4H_2O$ | calcd. | 64.58 | 5.09 | 15.98 |
| | | | | found: | 64.94 | 5.45 | 15.73 |
| (28) n-Bu | 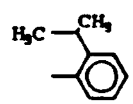 | > 85°C (gradual) | $C_{29}H_{31}N_7O \cdot 0.1C_7H_8 \cdot 0.2CH_2Cl_2$ (toluene) | calcd. | 69.09 | 6.24 | 18.86 |
| | | | | found: | 69.47 | 6.47 | 18.58 |
| (29) n-Bu | 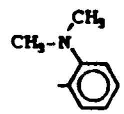 | 179-181°C | $C_{28}H_{30}N_8O \cdot 1/4C_7H_8 \cdot 1/2H_2O$ (toluene) | calcd. | 67.85 | 6.31 | 21.28 |
| | | | | found: | 68.07 | 6.23 | 21.06 |
| (30) n-Bu | 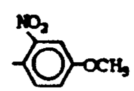 | >95°C (gradual) | $C_{27}H_{26}N_8O_4 \cdot 0.7H_2O \cdot 0.4CH_2Cl_2$ | calcd. | 57.42 | 4.96 | 19.55 |
| | | | | found: | 57.80 | 4.61 | 19.17 |

# Table I (Cont'd)

| | R | R' | mp | formula | | Analysis C | Analysis H | Analysis N |
|---|---|---|---|---|---|---|---|---|
| (31) | n-Pr | 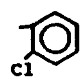 | >94°C (gradual) | $C_{25}H_{22}ClN_7O \cdot 0.8H_2O$ | calc'd:<br>found: | 61.74<br>61.97 | 4.89<br>4.93 | 20.16<br>19.88 |
| (32) | n-Pr | 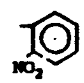 | >92°C (gradual) | $C_{25}H_{22}N_8O_3 \cdot H_2O \cdot 0.2CH_2Cl_2$ | calc'd:<br>found: | 58.49<br>58.55 | 4.75<br>4.53 | 21.65<br>21.44 |
| (33) | n-$C_5H_{11}$ | 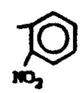 | >76°C (gradual) | $C_{27}H_{26}N_8O_3 \cdot 0.3CH_2Cl_2$ $0.1C_7H_8$ (Toluene) | calc'd:<br>found: | 62.16<br>61.84 | 5.10<br>4.93 | 20.71<br>20.57 |
| (34) | n-Bu | 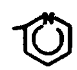 | >117°C (gradual) | $C_{25}H_{24}N_8O \cdot \frac{5}{8}CH_2Cl_2$ | calc'd:<br>found: | 60.87<br>61.20 | 5.03<br>5.17 | 22.16<br>21.81 |

# Table I (Cont'd)

| | R | R' | mp | formula | | Analysis C | Analysis H | Analysis N |
|---|---|---|---|---|---|---|---|---|
| (35) | n-Bu | $-CH_2C_6F_5$ | >79°C (gradual) | $C_{27}H_{22}F_5N_7O \cdot 0.4CH_2Cl_2 \cdot 0.15C_7H_8$ (toluene) | calc'd: | 56.64 | 4.01 | 16.25 |
| | | | | | found: | 56.94 | 3.67 | 16.10 |
| (36) | n-Bu | -CH2 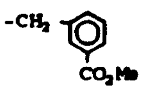 | >74°C (gradual) | $C_{29}H_{29}N_7O_3 \cdot 0.25CHCl_3$ | calc'd: | 63.48 | 5.33 | 17.72 |
| | | | | | found: | 63.54 | 5.42 | 17.46 |
| (37) | n-Bu | -CH2 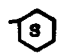 | 180-181°C | $C_{27}H_{33}N_7O \cdot 0.2H_2O$ | calc'd: | 68.24 | 7.08 | 20.63 |
| | | | | | found: | 68.23 | 7.01 | 20.52 |
| (38) | n-Bu | $-CH_3$ | 202-203°C | $C_{21}H_{23}N_7O$ | calc'd: | 64.76 | 5.95 | 25.18 |
| | | | | | found: | 64.57 | 5.97 | 25.16 |
| (39) | n-Bu | $-CH_2CH_3$ | 197-198°C | $C_{22}H_{25}N_7O \cdot 0.2H_2O$ | calc'd: | 64.91 | 6.29 | 24.09 |
| | | | | | found: | 65.03 | 5.94 | 23.92 |
| (40) | n-Bu | $-CH_2CH_2CH_3$ | 155-155.5°C | $C_{23}H_{27}N_7O \cdot 0.2H_2O$ | calc'd: | 65.59 | 6.56 | 23.21 |
| | | | | | found: | 65.53 | 6.51 | 23.00 |

Table I (Cont'd)

| R | R' | mp | formula | | Analysis C | Analysis H | Analysis N |
|---|---|---|---|---|---|---|---|
| (41) n-Bu | $-CH(CH_3)_2$ | 187-188°C | $C_{23}H_{27}N_7O \cdot 0.75H_2O$ | calc'd: | 64.09 | 6.66 | 22.75 |
| | | | | found: | 64.34 | 6.40 | 22.42 |
| (42) n-Bu | $-(CH_2)_3CH_2$ | 175-177°C | $C_{24}H_{29}N_7O \cdot 0.3H_2O \cdot 0.1CH_2Cl_2$ | calc'd: | 64.98 | 6.74 | 22.01 |
| | | | | found: | 65.35 | 6.94 | 21.59 |
| (43) n-Bu | $-CH_2CH(CH_3)_2$ | >80° (gradual) | $C_{24}H_{29}N_7O \cdot 0.6H_2O$ | calc'd: | 65.18 | 6.86 | 22.17 |
| | | | | found: | 65.21 | 6.85 | 21.92 |
| (44) n-Bu | 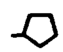 | 226-227°C | $C_{25}H_{29}N_7O \cdot 0.4H_2O$ | calc'd: | 66.61 | 6.66 | 21.75 |
| | | | | found: | 66.76 | 6.63 | 21.50 |
| (45) n-Bu | $-CH_2CO_2Me$ | >100°C (gradual) | $C_{23}H_{25}N_7O_3 \cdot 0.75H_2O \cdot 0.1C_4H_{10}$ | calc'd: | 60.00 | 5.92 | 20.93 |
| | | | | found: | 60.41 | 5.86 | 20.61 |

## Table I (Cont'd)

| | R | $R^1$ | mp | formula | | Analysis C | Analysis H | Analysis N |
|---|---|---|---|---|---|---|---|---|
| (46) | n-Bu | $-CH_2CF_3$ | 66-70°C | $C_{22}H_{22}F_3N_7O \cdot 0.3C_7H_8 \cdot H_2O$ (toluene) | calc'd: | 57.54 | 5.29 | 19.49 |
| | | | | | found: | 57.56 | 5.13 | 19.16 |
| (47) | n-Pentyl | 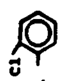 | 87-91°C | $C_{27}H_{28}ClN_7O \cdot 0.4H_2O$ | calc'd: | 63.94 | 5.32 | 19.33 |
| | | | | | found: | 64.09 | 5.27 | 18.93 |
| (48) | n-Bu | 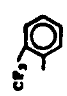 | 88-90°C | $C_{27}H_{24}F_3N_7O \cdot 0.1CH_2Cl_2 \cdot 0.1H_2O$ | calc'd: | 61.44 | 4.64 | 18.51 |
| | | | | | found: | 61.83 | 4.79 | 18.15 |
| (49) | n-Bu | $-(CH_2)_2C_6H_5$ | 71-73°C | $C_{28}H_{29}N_7O \cdot 0.5H_2O$ | calc'd: | 68.83 | 6.19 | 20.07 |
| | | | | | found: | 68.84 | 6.05 | 19.68 |
| (50) | n-Bu | $-CH(CH_3)CO_2Me$ | 69-71°C | $C_{24}H_{27}N_7O_3 \cdot 0.6H_2O$ | calc'd: | 61.03 | 6.02 | 20.76 |
| | | | | | found: | 61.47 | 5.92 | 20.39 |
| (51) | n-Bu | $-CH(CH_3)CO_2H$ | 105-109°C | $C_{23}H_{25}N_7O_3$ | | | | |

The following representative compounds of formula (I) can be prepared using the procedures of Examples 2,3,6,7,8 and 9 and Reaction Schemes 1,2,15 and 16:

(1) 5-n-butyl-2-(α-carboxybenzyl)-2,4-dihydro-4-[[2′-(5-tetrazolyl)biphenyl-4-yl]methyl]-3H-1,2,4-triazol-3-one;
(2) 5-n-butyl-2,4-dihydro-2-[α-(hydroxymethyl)benzyl]-4-[[2′-(5-tetrazolyl)biphenyl-4-yl]methyl]-3H-1,2,4-triazol-3-one;
(3) 2-benzyl-2,4-dihydro-5-n-pentyl-4-[[2′-(5-tetrazolyl)biphenyl-4-yl]methyl]-3H-1,2,4-triazol-3-one;
(4) 5-n-butyl-2-carboxymethyl-2,4-dihydro-4-[[2′-(5-tetrazolyl)biphenyl-4-yl]methyl]-3H-1,2,4-triazol-3-one;
(5) 5-n-butyl-2,4-dihydro-2-(2-hydroxyethyl)4-[[2′-(5-tetrazolyl)biphenyl-4-yl]methyl]-3H-1,2,4-triazol-3-one;
(6) 5-n-butyl-2,4-dihydro-2-(2-pyridylmethyl)-4-[[2′-(5-tetrazolyl)biphenyl-4-yl]methyl]-3H-1,2,4-triazol-3-one;
(7) 5-n-butyl-2-[2-(2-carboxyphenyl)ethyl]-2,4-dihydro-4-[[2′-(5-tetrazolyl)biphenyl-4-yl]methyl]-3H-1,2,4-triazol-3-one;

(8) 5-n-butyl-2,4-dihydro-2-(1H-hexafluoroisopropyl)-4-[[2′-(5-tetrazolyl)biphenyl-4-yl]methyl]-3H-1,2,4-triazol-3-one;
(9) 5-n-butyl-2,4-dihydro-2-(1H,1H-pentafluoropropyl)-4-[[2′-(5-tetrazolyl)biphenyl-4-yl]methyl]-3H-1,2,4-triazol-3-one;
(10) 5-n-butyl-2-(1-carboxy-2-phenylethyl)-2,4-dihydro-4-[[2′-(5-tetrazolyl)biphenyl-4-yl]methyl]-3H-1,2,4-triazol-3-one;
(11) 5-n-butyl-2-(2-carboxy-2-phenylethyl)-2,4-dihydro-4-[[2′-(5-tetrazolyl)biphenyl-4-yl]methyl]-3H-1,2,4-triazol-3-one; and,
(12) 5-n-butyl-2-(cyclopropylmethyl)-2,4-dihydro-4-[[2′-(5-tetrazolyl)biphenyl-4-yl]methyl]-3H-1,2,4-triazol-3-one.

The following representative compounds of formula (I) can be prepared using the procedures of Examples 4,5,7, and 8 and Reaction Schemes 3,15,16:

(1) 5-n-butyl-2-[2-(carbomethoxymethyl)phenyl]-2,4-dihydro-4-[[2′-(5-tetrazolyl)biphenyl-4-yl]methyl]-3H-1,2,4-triazol-3-one;
(2) 5-n-butyl-2-[2-(carboxymethyl)phenyl]-2,4-dihydro-4-[[2′-(5-tetrazolyl)biphenyl-4-yl]methyl]3H-1,2,4-triazol-3-one;
(3) 5-n-butyl-2,4-dihydro-2-[2-(2-hydroxyethyl)phenyl]-4-[[2′-(5-tetrazolyl)biphenyl-4-yl]methyl]-3H-1,2,4-triazol-3-one;
(4) 5-n-butyl-2,4-dihydro-2-[2-(N-methylcarbamoyl)phenyl]-4-[[2′-(5-tetrazolyl)biphenyl-4-yl]methyl]-3H-1,2,4-triazol-3-one;
(5) 5-n-butyl-4-[[2′-carboxybiphenyl-4-yl)methyl]-2-(2-chlorophenyl)-2,4-dihydro-3H-1,2,4-triazol-3-one; and,
(6) 5-n-butyl-2,4-dihydro-2-[2-(2-oxazolin-2-yl)phenyl]-4-[[2′-(5-tetrazolyl)biphenyl-4-yl]methyl]-3H-1,2,4-triazol-3-one.
(7) 5-n-butyl-2-(2-carbomethoxy-6-chlorophenyl)-2,4-dihydro-4-[[2′-(5-tetrazolyl)biphenyl-4-yl]methyl]-3H-1,2,4-triazol-3-one;
(8) 5-n-butyl-2-(2-carboxy-6-chlorophenyl)-2,4-dihydro-4-[[2′-(5-tetrazolyl)biphenyl-4-yl]methyl]-3H-1,2,4-triazol-3-one; and,
(9) 5-n-butyl-2-(2-chloro-3-pyridyl)-2,4-dihydro-4-[[2′-(5-tetrazolyl)biphenyl-4-yl]methyl]-3H-1,2,4-triazol-3-one.

The following representative compounds of formula (I) can be prepared using the procedures of Reaction Schemes 1-19 (optionally including additional methods referred to in the discussion accompanying the Reaction Schemes):

(1) 2-benzyl-5-n-butyl-2,4-dihydro-4-[[2′-(5-tetrazolyl)biphenyl-4-yl]methyl]-3H-1,2,4-triazole-3-thione;
(2) 5-n-butyl-2-(2-chlorophenyl)-2,4-dihydro-4-[[2′-(5-tetrazolyl)biphenyl-4-yl]methyl]-3H-1,2,4-triazole-3-thione;
(3) 2-benzyl-5-n-butyl-2,4-dihydro-4-[[2′-(5-tetrazolyl)biphenyl-4-yl]methyl]-3H-1,2,4-triazol-3-imine;
(4) 5-n-butyl-2,4-dihydro-2-isopropyl-$N^3$-phenyl-4-[[2′-(5-tetrazolyl)biphenyl-4-yl]methyl]-3H-1,2,4-triazol-3-imine;
(5) 5-n-butyl-$N^3$-(2-carboxyphenyl)-2,4-dihydro-2-methyl-4-[[2′-(5-tetrazolyl)biphenyl-4-yl]methyl]-3H-1,2,4-triazol-3-imine;
(6) 2-benzyl-5-n-butyl-4-[4-(2-carboxybenzamido)benzyl]-2,4-dihydro-3H-1,2,4-triazol-3-one;
(7) 5-n-butyl-4-[4-(2-carboxybenzoyl)benzyl]-2-(2-chlorophenyl)-2,4-dihydro-3H-1,2,4-triazol-3-one;
(8) 2-(2-chlorophenyl)-2,4-dihydro-5-(n-propylthio)-4-[[2′-(5-tetrazolyl)biphenyl-4-yl]methyl]-3H-1,2,4-triazol-3-one;
(9) 2-benzyl-2,4-dihydro-5-ethylthio-4-[[2′-(5-tetrazolyl)biphenyl-4-yl]methyl]-3H-1,2,4-triazol-3-one;
(10) 5-n-butyl-2-(2-chlorophenyl)-2,4-dihydro-4-[[2′-[N-(methanesulfonyl)carbamoyl]biphenyl-4-yl]methyl]-3H-1,2,4-triazol-3-one;
(11) 4-[[2′-[N-(benzenesulfonyl)carbamoyl]biphenyl-4-yl]methyl]-5-n-butyl-2,4-dihydro-2-isopropyl-3H-1,2,4-triazol-3-one;
(12) 5-n-butyl-2,4-dihydro-4-[[2′-[N-(dimethylsulfamoyl)carbamoyl]biphenyl-4-yl]methyl]-2-(2-nitrophenyl)-3H-1,2,4-triazol-3-one;
(13) 4-[[2′-(N-acetylsulfamoyl)biphenyl-4-yl]methyl]-5-n-butyl-2-(2-chlorophenyl)-2,4-dihydro-3H-1,2,4-triazol-3-one;
(14) 4-[[2′-(N-benzoylsulfamoyl)biphenyl-4-yl]methyl]-5-n-butyl-2,4-dihydro-2-isopropyl-3H-1,2,4-triazol-3-one;
(15) 5-n-butyl-2,4-dihydro-4-[[2′-[N-(dimethylcarbamoyl)sulfamoyl]biphenyl-4-yl]methyl]-2-(2-nitrophenyl)-3H-1,2,4-triazol-3-one;
(16) 5-n-butyl-2-(2-chlorophenyl)-2,4-dihydro-4-[[2′-[N-(2-pyrimidyl)sulfamoyl]biphenyl-4-yl]methyl]-3H-1,2,4-triazol-3-one

EXAMPLE 10

Typical Pharmaceutical Compositions Containing a Compound of the Invention

A: Dry Filled Capsules Containing 50 mg of Active Ingredient Per Capsule

| Ingredient | Amount per capsule (mg) |
|---|---|
| 5-n-butyl-2-(2-chlorophenyl)-2,4-dihydro-4-[[2′-(5-tetrazolyl)biphenyl-4-yl]methyl]-3H-1,2,4-triazol-3-one | 50 |
| Lactose | 149 |
| Magnesium stearate | 1 |
| Capsule (size No. 1) | 200 |

The 5-n-butyl-2-(2-chlorophenyl)-2,4-dihydro-4-[[2′-(5-tetrazolyl)biphenyl-4-yl]methyl]-3H-1,2,4-triazol-3-one can be reduced to a No. 60 powder and the lactose and magnesium stearate can then be passed through a No. 60 blotting cloth onto the powder. The combined ingredients can then be mixed for about 10 minutes and filled into a No. 1 dry gelatin capsule.

B: Tablet

A typical tablet would contain 5-n-butyl-2-(2-chlorophenyl)-2,4-dihydro-4-[[2′-(5-tetrazolyl)biphenyl-4-yl]methyl]-3H-1,2,4-triazol-3-one (25 mg), pregelatinized starch USP (82 mg), microcrystalline cellulose (82 mg) and magnesium stearate (1 mg).

C: Combination Tablet

A typical combination tablet would contain, for example, a diuretic such as hydrochlorothiazide and consist of 5-n-butyl-2-(2-chlorophenyl)-2,4-dihydro-4-[[2′-(5-tetrazolyl)biphenyl-4-yl]methyl]-3H-1,2,4-triazol-3-one (7.5 mg), hydrochlorothiazide (50 mg) pregelatinized starch USP (82 mg), microcrystalline cellulose (82 mg) and magnesium stearate (1 mg).

D: Suppository

Typical suppository formulations for rectal administration can contain 5-n-butyl-2-(2-chlorophenyl)-2,4-dihydro-4-[[2′-(5-tetrazolyl)biphenyl-4-yl]methyl]-3H-1,2,4-triazol-3-one (1-25 mg), butylated hydroxyanisole (0.08-1.0 mg), disodium calcium edetate (0.25-0.5 mg), and polyethylene glycol (775-1600 mg). Other suppository formulations can be made by substituting, for example, butylated hydroxytoluene (0.04-0.08 mg) for the disodium calcium edetate and a hydrogenated vegetable oil (675-1400 mg) such as Suppocire L, Wecobee FS, Wecobee M, Witepsols, and the like, for the polyethylene glycol. Further, these suppository formulations can also include another active ingredient such as another antihypertensive and/or a diuretic and/or an angiotensin converting enzyme and/or a calcium channel blocker in pharmaceutically effective amounts as described, for example, in C above.

E: Injection

A typical injectible formulation would contain 5-n-butyl-2-(2-chlorophenyl)-2,4-dihydro-4-[[2′-(5-tetrazolyl)biphenyl-4-yl]methyl]-3H-1,2,4-triazol-3-one (5.42 mg), sodium phosphate dibasic anhydrous (11.4 mg) benzyl alcohol (0.01 ml) and water for injection (1.0 ml). Such an injectible formulation can also include a pharmaceutically effective amount of another active ingredient such as another antihypertensive and/or a diuretic and/or an angiotensin converting enzyme inhibitor and/or a calcium channel blocker.

## Claims

1. A compound having the formula (I):

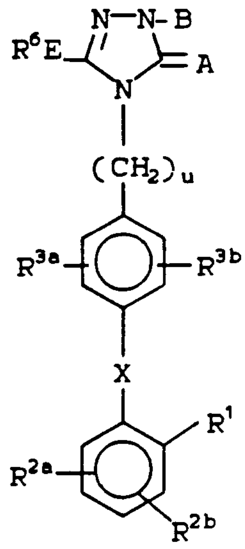

(I)

wherein:

$R^1$ is

(a) $-CO_2R^4$,
(b) $-SO_3R^5$,
(c) $-NHSO_2CF_3$,
(d) $-PO(OR^5)_2$,
(e) $-SO_2$-NH-$R^9$,
(f) -CONHOR$^5$,
(g) $-SO_2$NH-heteroaryl,
(h) $-CH_2SO_2$NH-heteroaryl,
(i) $-SO_2NHCOR^{23}$,
(j) $-CH_2SO_2NHCOR^{23}$,
(k) $-CONHSO_2R^{23}$,
(l) $-CH_2CONHSO_2R^{23}$,
(m) $-NHSO_2NHCOR^{23}$,
(n) $-NHCONHSO_2R^{23}$,
(o)

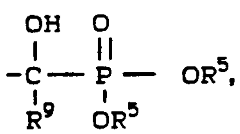

(p)

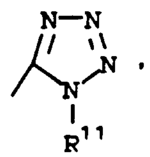

(q)

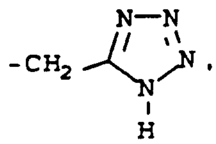

(r)

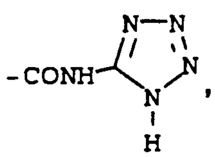

(s) $-CONHNHSO_2CF_3$ ,

(t)

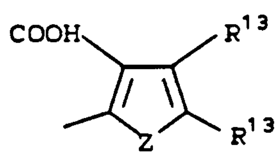

(u)

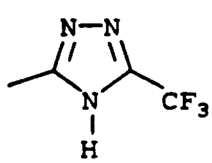

(v)

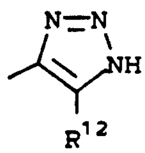

(w)

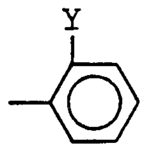

wherein:
heteroaryl is an unsubstituted, monosubstituted or disubstituted 5- or 6-membered aromatic ring which can optionally contain from 1 to 3 heteroatoms selected from the group consisting of O, N and S and wherein the substituents are members selected from the group consisting of -OH, -SH, $C_1$-$C_4$-alkyl, $C_1$-$C_4$-alkoxy, $-CF_3$, halo (Cl, Br, F, I), $-NO_2$, $-CO_2H$, $-CO_2$-$C_1$-$C_4$-alkyl, $-NH_2$, -NH($C_1$-$C_4$-alkyl) or -N($C_1$-$C_4$-alkyl)$_2$ and

Y is

(1) $-CO_2R^4$,
(2) $-SO_3R^5$,
(3) $-NHSO_2CF_3$,
(4) $-PO(OR^5)_2$, or
(5) $-SO_2$-NH-$R^9$;
(6) 1H-tetrazol-5-yl;

$R^{2a}$ and $R^{2b}$ are each independently:

(a) hydrogen,
(b) halogen (Cl, Br, I, F)
(c) $-NO_2$,
(d) $NH_2$,
(e) $C_1$-$C_4$-alkylamino,
(f) $-SO_2NHR^9$,
(g) $CF_3$,
(h) $C_1$-$C_4$-alkyl
(i) $C_1$-$C_4$-alkoxy; or

when $R^{2a}$ and $R^{2b}$ are on adjacent carbons, they can be bonded together to form a phenyl ring;

$R^{3a}$ is

(a) H,
(b) halo (Cl, Br, I, F)
(c) $C_1$-$C_6$-alkyl,
(d) $C_1$-$C_6$-alkoxy,
(e) $C_1$-$C_6$-alkoxy-$C_1$-$C_4$-alkyl;

$R^{3b}$ is

(a) H,
(b) halo (Cl, Br, I, F)
(c) $NO_2$,
(d) $C_1$-$C_6$-alkyl,
(e) $C_1$-$C_5$-alkylcarbonyloxy,
(f) $C_3$-$C_6$-cycloalkyl
(g) $C_1$-$C_6$-alkoxy,
(h) -$NHSO_2R^4$,
(i) hydroxy-$C_1$-$C_4$-alkyl,
(j) aryl-$C_1$-$C_4$-alkyl,
(k) $C_1$-$C_4$-alkylthio,
(l) $C_1$-$C_4$-alkylsulfinyl,
(m) $C_1$-$C_4$-alkylsulfonyl,
(n) $NH_2$,
(o) $C_1$-$C_4$-alkylamino,
(p) di($C_1$-$C_4$-alkyl)amino,
(q) $CF_3$,
(r) -$SO_2$-$NHR^9$,
(s) aryl,
(t) furyl; or

when $R^{3a}$ and $R^{3b}$ are on adjacent carbons, they can be bonded together to form a phenyl ring;
wherein aryl is phenyl or naphthyl optionally substituted with one or two substituents selected from the group consisting of halo (Cl, Br, I, F) $C_1$-$C_4$-alkyl, $C_1$-$C_4$-alkoxy, $NO_2$, $CF_3$, $C_1$-$C_4$-alkylthio, OH or $NH_2$;

$R^4$ is H, straight chain or branched $C_1$-$C_6$ alkyl, benzyl or phenyl;

$R^5$ is H or -$CH(R^4)$-O-CO-$R^4$;

E is a single bond, -$NR^{13}(CH_2)_s$-,-$S(O)_x(CH_2)_s$- where x is 0 to 2 and s is 0 to 5, -CH(OH)-, -$0(CH_2)_s$-, -C0-;

$R^6$ is

(a) aryl as defined above optionally substituted with 1 or 2 substituents selected from the group consisting of halo (Cl, Br, I, F), -O-$C_1$-$C_4$-alkyl, $C_1$-$C_4$-alkyl, -$NO_2$, -$CF_3$, -$SO_2NR^9R^{10}$, -S-$C_1$-$C_4$-alkyl, -OH, -$NH_2$, $C_3$-$C_7$-cycloalkyl, $C_3$-$C_{10}$-alkenyl;
(b) straight chain or branched $C_1$-$C_6$-alkyl, $C_2$-$C_6$-alkenyl or $C_2$-$C_6$-alkynyl each of which can be optionally substituted with one or more substituents selected from the group consisting of aryl as defined above, $C_3$-$C_7$-cycloalkyl, halo (Cl, Br, I, F), -OH, -O-$C_1$-$C_4$-alkyl, -$NH_2$, -NH($C_1$-$C_4$-alkyl), -N($C_1$-$C_4$-alkyl)$_2$, -NH-$SO_2R^4$, -$COOR^4$, -$SO_2NHR^9$, -S-$C_1$-$C_4$-alkyl;
(c) an unsubstituted, monosubstituted or disubstituted aromatic 5- or 6-membered ring which can contain one or two heteroatoms selected from the group consisting of N, O, S, and wherein the substituents are members selected from the group consisting of -OH, -SH, $C_1$-$C_4$-alkyl, $C_1$-$C_4$-alkyloxy, -$CF_3$, halo (Cl, Br, I, F), or $NO_2$;
(d) mono-, di-, tri- or polyfluoro-$C_1$-$C_5$-alkyl;
(e) $C_3$-$C_7$-cycloalkyl optionally substituted with one or more substituents selected from the group consisting of $C_1$-$C_4$-alkyl, O-$C_1$-$C_4$-alkyl, S-$C_1$-$C_4$-alkyl, OH, perfluoro-$C_1$-$C_4$-alkyl, or halo (Cl, Br, F, I); or
(f) $C_3$-$C_7$-cycloalkyl-$C_1$-$C_3$-alkyl wherein the cycloalkyl is substituted as in (e) above;

A is =O, =S or =$NR^{21}$;

B is

(a) H provided A is not $NR^{21}$;
(b) $C_1$-$C_{10}$-alkyl;
(c) substituted $C_1$-$C_{10}$-alkyl in which one or more substituent(s) is selected from

(1) halogen (I, Br, Cl, F),
(2) hydroxy,
(3) $C_1$-$C_{10}$-alkoxy,
(4) $C_1$-$C_5$-alkoxycarbonyl,
(5) $C_1$-$C_4$-alkylcarbonyloxy,
(6) $C_3$-$C_8$-cycloalkyl,
(7) aryl,
(8) substituted aryl in which the substituents are $V_1$, $V_2$, $V_3$, $V_4$ and $V_5$,
(9) $C_1$-$C_{10}$-alkyl-$S(O)_p$ in which p is 0 to 2,
(10) $C_3$-$C_8$-cycloalkyl-$S(O)_p$,
(11) phenyl-$S(O)_p$,
(12) substituted phenyl-$S(O)_p$ in which the substituents are $V_1$-$V_5$,
(13) oxo,
(14) carboxy,
(15) $NR^9R^9$,
(16) $C_1$-$C_5$-alkylaminocarbonyl,
(17) di($C_1$-$C_5$-alkyl)aminocarbonyl,
(18) cyano;

(d) $C_2$-$C_{10}$-alkenyl,
(e) $C_2$-$C_{10}$-alkynyl,
(f) $C_3$-$C_8$-cycloalkyl,
(g) substituted $C_3$-$C_8$-cycloalkyl or substituted $C_3$-$C_8$-cycloalkyl-$C_1$-$C_4$-alkyl having one or more substituents selected from the group:

(1) halo (Cl, Br, F, I),
(2) hydroxy,
(3) $C_1$-$C_6$-alkyl,
(4) $C_1$-$C_6$-alkoxy,
(5) $C_1$-$C_4$-alkylcarbonyloxy,
(6) $C_1$-$C_5$-alkoxycarbonyl,
(7) carboxy,
(8) oxo,
(9) $C_1$-$C_5$-alkylaminocarbonyl,
(10) di($C_1$-$C_5$-alkyl)aminocarbonyl,
(11) $C_1$-$C_4$-alkylcarbonyl;
(12) aryl,
(13) substituted aryl in which the substituents are $V_1$, $V_2$, $V_3$, $V_4$ and $V_5$,

(h) aryl,
(i) substituted aryl in which the substituents are $V_1$, $V_2$, $V_3$, $V_4$ and $V_5$,
(j) aryl-$(CH_2)_r$-$(Q)_c$-$(CH_2)_t$-,
(k) substituted aryl-$(CH_2)_r$-$(Q)_c$-$(CH_2)_t$- in which the aryl group is substituted with $V_1$, $V_2$, $V_3$, $V_4$ and $V_5$,

(l) a heterocyclic ring of 5 to 6 atoms containing one or two heteroatoms selected from:

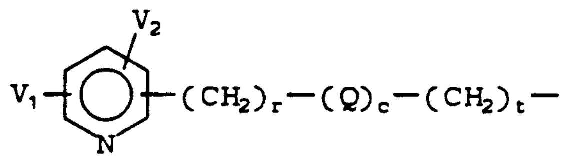

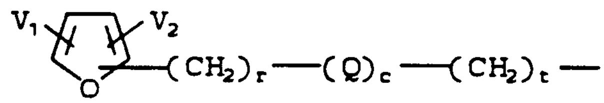

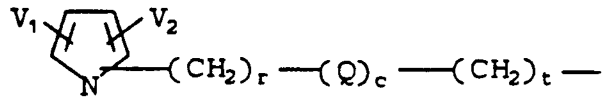

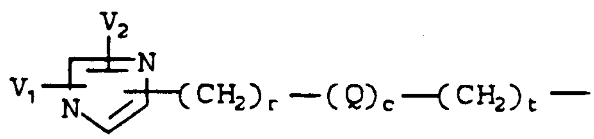

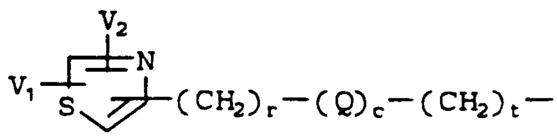
;

| | |
|---|---|
| $R^9$ is | H, $C_1$-$C_5$-alkyl, phenyl or benzyl; |
| $R^{10}$ is | H, $C_1$-$C_4$-alkyl, or |
| $R^9$ and $R^{10}$ | together can be $-(CH_2)_m-$ where m is 3-6; |
| $R^{11}$ is | H, $C_1$-$C_6$-alkyl, $C_2$-$C_4$-alkenyl, $C_1$-$C_4$-alkoxy-$C_1$-$C_4$-alkyl, or $-CH_2-C_6H_4R^{20}$; |
| $R^{12}$ is | -CN, $-NO_2$ or $-CO_2R^4$; |
| $R^{13}$ is | H, $C_1$-$C_4$-acyl, $C_1$-$C_6$-alkyl, allyl, $C_3$-$C_6$-cycloalkyl, phenyl or benzyl; |
| $R^{14}$ is | H, $C_1$-$C_8$-alkyl, $C_1$-$C_8$-perfluoroalkyl, $C_3$-$C_6$-cycloalkyl, phenyl or benzyl; |
| $R^{15}$ is | H, $C_1$-$C_6$-alkyl, hydroxy; |
| $R^{16}$ is | H, $C_1$-$C_6$-alkyl, $C_3$-$C_6$-cycloalkyl, phenyl or benzyl; |

| | |
|---|---|
| $R^{17}$ is | $-NR^9R^{10}$, $-OR^{10}$, $-NHCONH_2$, $-NHCSNH_2$, $-NHSO_2CF_3$, |

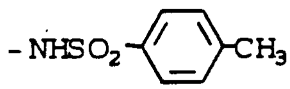 or 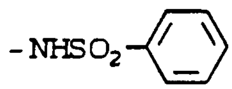 ;

| | |
|---|---|
| $R^{18}$ and $R^{19}$ | are independently $C_1$-$C_4$-alkyl or taken together are $-(CH_2)_q$-where q is 2 or 3; |
| $R^{20}$ is | H, $-NO_2$, $-NH_2$, -OH or $-OCH_3$; |

$R^{21}$ is

(a) H,
(b) aryl as defined above optionally substituted with 1 or 2 substituents selected from the group consisting of halo (Cl, Br, I, F) -0-$C_1$-$C_4$-alkyl, $C_1$-$C_4$-alkyl, $-NO_2$, $-CF_3$, -$SO_2NR^9R^{10}$, -S-$C_1$-$C_4$-alkyl, -OH, $-NH_2$, $-COOR^4$, $C_3$-$C_7$-cycloalkyl, $C_3$-$C_{10}$-alkenyl;
(c) straight chain or branched $C_1$-$C_6$-alkyl, $C_2$-$C_6$-alkenyl or $C_2$-$C_6$-alkynyl each of which can be optionally substituted with one or more substituents selected from the group consisting of aryl as defined above, $C_3$-$C_7$-cycloalkyl, halo (Cl, Br, I, F), -OH, -O-$C_1$-$C_4$-alkyl, $-NH_2$, -NH($C_1$-$C_4$-alkyl), -N($C_1$-$C_4$-alkyl)$_2$, -NH-$SO_2R^4$, $-COOR^4$, -$SO_2NHR^9$, -S-$C_1$-$C_4$-alkyl;
(d) an unsubstituted, monosubstituted or disubstituted aromatic 5 or 6 membered ring which can contain one or two heteroatoms selected from the group consisting of N, O, S, and wherein the substituents are members selected from the group consisting of -OH, -SH, $C_1$-$C_4$-alkyl, $C_1$-$C_4$-alkyloxy $-CF_3$, $-COOR^4$, halo (Cl, Br, I, F), or $NO_2$; or
(e) $C_3$-$C_7$-cycloalkyl optionally substituted with one or more substituents selected from the group consisting of $C_1$-$C_4$-alkyl, -O-$C_1$-$C_4$-alkyl, -S-$C_1$-$C_4$-alkyl, -OH, -$COOR^4$, perfluoro-$C_1$-$C_4$-alkyl or halo (Cl, Br, F, I);

$R^{23}$ is

(a) aryl as defined above,
(b) heteroaryl as defined above,
(c) $C_3$-$C_7$-cycloalkyl,
(d) $C_1$-$C_4$-alkyl optionally substituted with a substituent selected from the group consisting of aryl as defined above, heteroaryl as defined above, -OH, -SH, $C_1$-$C_4$-alkyl, -O($C_1$-$C_4$-alkyl), S($C_1$-$C_4$-alkyl), $-CF_3$, halo (Cl, Br, F, I), $-NO_2$, $-CO_2H$, $-CO_2$-$C_1$-$C_4$-alkyl, $-NH_2$, -NH($C_1$-$C_4$-alkyl), -N($C_1$-$C_4$-alkyl)$_2$, $-N(CH_2CH_2)_2L$ where L is a single bond, $CH_2$, O, $S(O)_p$, or $NR^9$, $-PO_3H$, -PO(OH)(O-$C_1$-$C_4$-alkyl);

X is

(a) a single bond,
(b) -CO-,
(c) -O-,
(d) -S-,
(e)

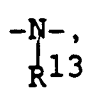

(f)

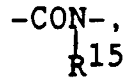

(g)

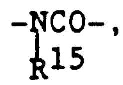

(h) $-OCH_2-$,
(i) $-CH_2O-$
(j) $-SCH_2-$,
(k) $-CH_2S-$,
(l) $-NHC(R^9)(R^{10})-$,
(m) $-NR^9SO_2-$,
(n) $-SO_2NR^9-$,
(o) $-C(R^9)(R^{10})NH-$,
(p) $-CH=CH-$,
(q) $-CF=CF-$,
(r) $-CH=CF-$,
(s) $-CF=CH-$,
(t) $-CH_2CH_2-$,
(u) $-CF_2CF_2-$,
(v)

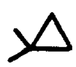 or 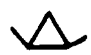

(w)

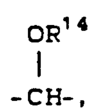

(x)

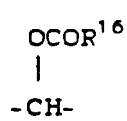

(y)

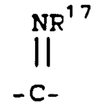

,

or

(z)

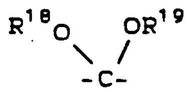

;

Q is -C(O)-, -S-, -O- or $-NR^4$;

c is 0 or 1;

r and t are 0 to 2;

$V_1$, $V_2$, $V_3$, $V_4$ and $V_5$ are each independently selected from:

(a) H,
(b) $C_1$-$C_5$-alkoxy,
(c) $C_1$-$C_5$-alkyl,
(d) hydroxy,
(e) $C_1$-$C_5$-alkyl-$S(O)_p$,
(f) -CN,
(g) $-NO_2$,
(h) $-NR^9R^{10}$;
(i) $C_1$-$C_5$-alkyl-$CONR^9R^{10}$,
(j) $-CONR^9R^{10}$,
(k) $-CO_2R^9$,
(l) $C_1$-$C_5$-alkyl-carbonyl,
(m) $CF_3$,
(n) halogen (I, Br, Cl, F),
(o) hydroxy-$C_1$-$C_4$-alkyl-,
(p) carboxy-$C_1$-$C_4$-alkyl-,
(q) -1H-tetrazol-5-yl,
(r) $-NH-SO_2CF_3$,
(s) aryl,
(t) $C_1$-$C_5$-alkyl-$CO_2R^9$,
(u) aryloxy,
(v) aryl-$C_1$-$C_3$-alkoxy,
(w) aryl-$C_1$-$C_3$-alkyl,
(x) carboxyphenyl,
(y) heteroaryl,
(z) 2-oxazolin-2-yl optionally bearing one or more $C_1$-$C_4$-alkyl substituents,
(aa) $-(CH_2)_tOCOR^9$,
(bb) $-(CH_2)_tOCONR^9R^{10}$,
(cc) $-(CH_2)_tNR^4COR^9$,
(dd) $-(CH_2)_tNR^4CO_2R^9$,
(ee) $-(CH_2)_tNR^4CONR^9R^{10}$,
(ff) $-(CH_2)_tNR^4CON(CH_2CH_2)_2G$,
(gg) $-(CH_2)_tOCON(CH_2CH_2)_2G$,

(hh) $-N(CH_2CH_2)_2G$,
(ii) $-C_1$-$C_5$-alkyl-$CON(CH_2CH_2)_2G$,
(jj) $-CON(CH_2CH_2)G$,

wherein G is O, $S(O)_p$ or $NR^9$,

u is 1 or 2;

Z is O, $NR^{13}$ or S; and,

the pharmaceutically acceptable salts thereof.

**2.** The compound of Claim 1 wherein:

$R^1$ is

(a) -COOH,
(b)

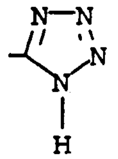

(c) $-NH-SO_2CF_3$;
(d) $-CONH-SO_2R^{23}$,
(e) $-SO_2NH-COR^{23}$,
(f) $-SO_2NH$-heteroaryl;

$R^{2a}$ is H;

$R^{2b}$ is H, F, Cl, $CF_3$ or $C_1$-$C_4$-alkyl;

$R^{3a}$ is H;

$R^{3b}$ is H, F, Cl, $CF_3$, $C_1$-$C_4$-alkyl, $C_5$-$C_6$-cycloalkyl, $-COOCH_3$, $-COOC_2H_5$, $-SO_2-CH_3$, $NH_2$, $-N(C_1$-$C_4$-alkyl$)_2$ or $-NH-SO_2CH_3$;

E is a single bond, -O- or -S-;

$R^6$ is

(a) $C_1$-$C_6$-alkyl optionally substituted with a substituent selected from the group consisting of Cl, $CF_3$, -OH, $-O-CH_3$, $-OC_2H_5$, $-S-CH_3$, $-S-C_2H_5$, phenyl, or cyclopropyl;
(b) $C_2$-$C_6$-alkenyl or $C_2$-$C_6$-alkynyl;
(c) aryl as defined above optionally substituted with a substituent selected from the group consisting of halo (Cl, F, Br, I), $-CF_3$, $-NO_2$, -OH, $-NH_2$, $-S-CH_3$, $-S-C_2H_5$, $-SO_2NH_2$ $-O-CH_3$;
(d) a heteroaryl which is a member selected from the group consisting of 2-pyridyl, 4-pyridyl, 2-pyrimidyl, 4-pyrimidyl, imidazoyl, thiazolyl, thienyl, or furyl; or,
(e) perfluoro-$C_1$-$C_4$-alkyl which is a member selected from the group consisting of $CF_3$-, $CF_3CF_2$-, $CF_3CF_2CF_2$-, or $CF_3CF_2CF_2CF_2$-;
(f) $C_3$-$C_7$-cycloalkyl optionally substituted with a substituent selected from the group consisting of methyl, ethyl, $CF_3$ or $CF_3CF_2$;

A is =0, =S or $=NR^{21}$;

B is

(a) H provided A is not $NR^{21}$,
(b) $C_1$-$C_{10}$-alkyl,
(c) substituted $C_1$-$C_{10}$-alkyl in which one or two substituents are selected from:

(1) hydroxy,
(2) $C_1$-$C_5$-alkoxy,
(3) $C_1$-$C_5$-alkoxycarbonyl,
(4) $C_1$-$C_4$-alkylcarbonyloxy,
(5) $C_3$-$C_8$-cycloalkyl,
(6) phenyl,
(7) substituted phenyl in which the substituents are $V_1$, $V_2$, $V_3$, $V_4$ and $V_5$,
(8) $C_1$-$C_5$-alkyl-$S(O)_p$
(9) phenyl-$S(O)_p$
(10) substituted phenyl-$S(O)_p$ in which the substituent is V,
(11) oxo,
(12) carboxy,
(13) $C_1$-$C_5$-alkylaminocarbonyl;

(d) mono-, di-, tri-, or polyfluoro-$C_1$-$C_{10}$-alkyl,
(e) $C_2$-$C_{10}$-alkenyl,
(f) $C_2$-$C_{10}$-alkynyl,
(g) $C_3$-$C_8$-cycloalkyl,
(h) substituted $C_3$-$C_8$-cycloalkyl or substituted $C_3$-$C_8$-cycloalkyl-$C_1$-$C_4$-alkyl in which one or more substituent(s) is selected from:

(1) hydroxy,
(2) $C_1$-$C_5$-alkoxy,
(3) $C_1$-$C_5$-alkoxycarbonyl,
(4) $C_1$-$C_5$-alkylcarbonyloxy,
(5) $C_1$-$C_6$-alkyl,
(6) phenyl,
(7) substituted phenyl in which the substituents are $V_1$, $V_2$, $V_3$, $V_4$, and $V_5$,
(8) oxo,
(9) carboxy,
(10) $C_1$-$C_5$-alkylaminocarbonyl;

(i) aryl,
(j) substituted aryl in which the substituents are $V_1$, $V_2$, $V_3$, $V_4$ and $V_5$,
(k) aryl-$(CH_2)_r$-$(Q)_c$-$(CH_2)_t$-,
(l) substituted aryl-$(CH_2)_r$-$(Q)_c$-$(CH_2)_t$-,

(m) a heterocyclic ring of 5 to 6 atoms containing one or two heteroatoms selected from:

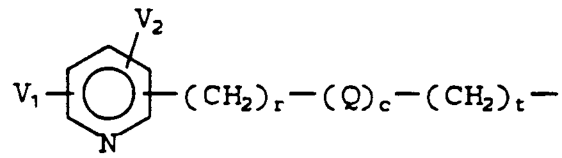

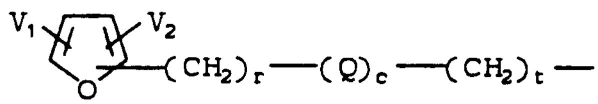

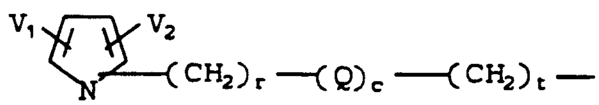

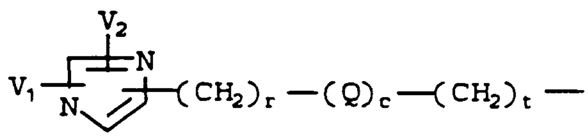

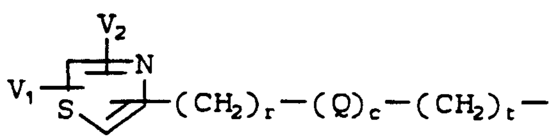
;

$V_1$, $V_2$, $V_3$, $V_4$ and $V_5$ are independently selected from:

(a) hydrogen,
(b) $C_1$-$C_5$-alkoxy,
(c) $C_1$-$C_5$-alkyl,
(d) hydroxy,
(e) $NR^9R^{10}$,
(f) $CO_2R^9$,
(g) trifluoromethyl;
(h) halogen;
(i) hydroxy-$C_1$-$C_4$-alkyl;
(j) -1H-tetrazol-5-yl,
(k) $-NH-SO_2CF_3$;
(l) CN;
(m) $NO_2$;
(n) $C_1$-$C_5$-alkyl-$CO_2R^9$,
(o) aryl,
(p) aryl-$C_1$-$C_3$-alkyl,
(q) heteroaryl,
(r) $C_1$-$C_5$-alkyl-$CONR^9R^{10}$,
(s) $-CONR^9R^{10}$,
(t) 2-oxazolin-2-yl optionally bearing one or more $C_1$-$C_4$-alkyl substituents,
(u) $C_1$-$C_5$-alkyl-$S(O)_p$,
(v) $(CH_2)_tOCOR^9$,
(w) $(CH_2)_tNR^4COR^9$;

u is 1;

X is:

(a) a single bond;
(b) -C(O)-;
(c) $-NR^{15}C(O)-$.

**3.** The compound of Claim 2 wherein:

E is a single bond or -S-;

$R^{2a}$, $R^{2b}$, $R^{3a}$ and $R^{3b}$ are each H;

$R^6$ is $C_1$-$C_6$ alkyl.

**4.** The compound of Claim 3 wherein:

A is =0, =S or $=NR^{21}$;
B is

(a) H provided A is not $NR^{21}$,
(b) $C_1$-$C_{10}$-alkyl,
(c) $C_3$-$C_8$-cycloalkyl,
(d) $C_3$-$C_8$-cycloalkyl-$C_1$-$C_4$-alkyl,
(e) substituted $C_1$-$C_{10}$-alkyl, $C_3$-$C_8$-cycloalkyl, or $C_3$-$C_8$-cycloalkyl-$C_1$-$C_4$-alkyl each of which can have one or two substituents selected from the group:

(1) hydroxy,
(2) $C_1$-$C_5$-alkoxy,
(3) $C_1$-$C_5$-alkoxycarbonyl,
(4) phenyl,
(5) carboxy,
(6) $C_1$-$C_5$-alkylaminocarbonyl,
(7) oxo;

(f) mono-, di-, tri-, or polyfluoro-$C_1$-$C_{10}$-alkyl,
(g) phenyl,
(h) phenyl substituted with $V_1$, $V_2$, $V_3$, $V_4$ and $V_5$,
(i) phenyl-$(CH_2)_r$-$(Q)_c$-$(CH_2)_t$-,
(j) phenyl-$(CH_2)_r$-$(Q)_c$-$(CH_2)_t$- in which the phenyl is substituted with $V_1$, $V_2$, $V_3$, $V_4$ and $V_5$,
(k) a heterocyclic moiety selected from:

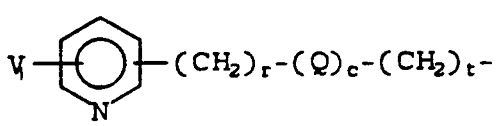 or

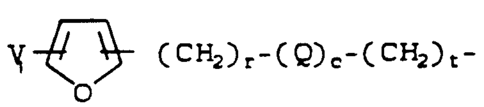;

$V_1$, $V_2$, $V_3$, $V_4$ and $V_5$ are selected from:

(a) hydrogen,
(b) $C_1$-$C_5$-alkyl,
(c) $C_1$-$C_5$-alkoxy,

(d) $CO_2R^9$,
(e) halogen,
(f) hydroxy-$C_1$-$C_4$-alkyl-,
(g) $C_1$-$C_5$-alkyl-$CO_2R^9$,
(h) $C_1$-$C_5$-alkyl-$CONR^9R^{10}$,
(i) $CONR^9R^{10}$,
(j) CN,
(k) $NO_2$,
(l) $CF_3$;
(m) aryl,
(n) heteroaryl,
(o) 2-oxazolin-2-yl optionally bearing one or more $C_1$-$C_4$-alkyl substituents;
(p) $C_1$-$C_5$-alkyl-$S(O)_p$,
(q) $(CH_2)_tOCOR^9$,
(r) $(CH_2)_tNR^4COR^9$,
(s) hydroxy,
(t) $NR^9R^{10}$;

| | |
|---|---|
| X is | $-NR^{15}C(O)-$ or a carbon-carbon single bond. |

**5.** The compound of Claim 4 wherein:

| | |
|---|---|
| $R^1$ is | 5-tetrazolyl or carboxy; |
| $R^{2a}$, $R^{2b}$, $R^{3a}$, and $R^{3b}$ | are each H; |
| $R^6$ is | $C_1$-$C_6$-alkyl; |
| A is | =O or =S; |
| B is | H; substituted or unsubstituted $C_1$-$C_{10}$-alkyl, $C_3$-$C_8$-cycloalkyl or $C_3$-$C_8$-cycloalkyl-$C_1$-$C_4$-alkyl; substituted or unsubstituted aryl, heteroaryl, or aralkyl; |
| E and X | are each single bonds; and, |
| u is | 1. |

**6.** The compound of Claim 4 wherein:

| | |
|---|---|
| $R^1$ is | 5-tetrazolyl or carboxy; |
| $R^{2a}$, $R^{2b}$, $R^{3a}$, and $R^{3b}$ | are each H; |
| $R^6$ is | $C_1$-$C_6$-alkyl; |
| A is | O; |
| B is | H, substituted or unsubstituted $C_1$-$C_{10}$-alkyl, substituted or unsubstituted aralkyl or heteroarylalkyl; |
| E and X | are each single bonds; and, |
| u is | 1. |

**7.** The compound of Claim 4 wherein:

| | |
|---|---|
| $R^1$ is | 5-tetrazolyl or carboxy; |
| $R^{2a}$, $R^{2b}$, $R^{3a}$, and $R^{3b}$ | are each H; |
| $R^6$ is | $C_1$-$C_6$-alkyl; |
| A is | O; |
| B is | substituted or unsubstituted aryl or heteroaryl; |
| E and X | are each single bonds; and, |
| u is | 1. |

**8.** The compound of Claim 4 wherein:

| | |
|---|---|
| $R^1$ is | 5-tetrazolyl, carboxy, $-CONHSO_2R^{23}$, $-SO_2NHCOR^{23}$ or $-SO_2NH$-heteroaryl; |
| $R^{2a}$, $R^{2b}$, $R^{3a}$, and $R^{3b}$ | are each H; |
| $R^6$ is | $C_1$-$C_6$-alkyl; |
| A is | O, S or $NR^{21}$; |
| B is | $C_1$-$C_{10}$-alkyl, substituted or unsubstituted aryl or aralkyl; |

$R^{21}$ is H, substituted or unsubstituted aryl;

$R^{23}$ is

(a) substituted or unsubstituted aryl,
(b) substituted or unsubstituted alkyl;

E is a single bond or S,

X is a single bond, -NHCO- or -CO-; and,

u is 1.

**9.** A pharmaceutical composition useful in the treatment of hypertension which comprises a pharmaceutically acceptable carrier and a pharmaceutically effective amount of a compound of Claim 1.

**10.** The composition of Claim 9 which includes an antihypertensive or a diuretic or an angiotensin converting enzyme inhibitor or a calcium channel blocker which are members selected from the group consisting of: amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methylclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, verapamil, as well as admixtures and combinations thereof.

**11.** An ophthalmological formulation for the treatment of ocular hypertension comprising an ophthalmologically acceptable carrier and an effective ocular antihypertensive amount of a compound of Claim 1.

**Patentansprüche**

**1.** Eine Verbindung mit der Formel (I):

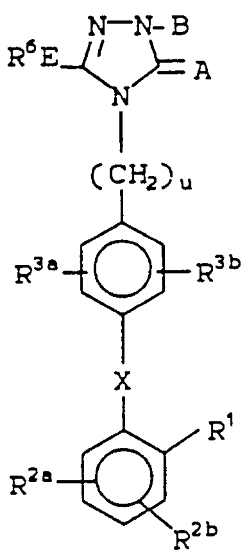

(I)

worin

$R^1$
(a) $-CO_2R^4$,
(b) $-SO_3R^5$,
(c) $-NHSO_2CF_3$,
(d) $-PO(OR^5)_2$,
(e) $-SO_2-NH-R^9$,
(f) $-CONHOR^5$,

(g) $-SO_2NH$-heteroaryl,
(h) $-CH_2SO_2NH$-heteroaryl,
(i) $-SO_2NHCOR^{23}$,
(j) $-CH_2SO_2NHCOR^{23}$,
(k) $-CONHSO_2R^{23}$,
(l) $-CH_2CONHSO_2R^{23}$,
(m) $-NHSO_2NHCOR^{23}$,
(n) $-NHCONHSO_2R^{23}$,
(o)

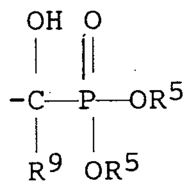

(p)

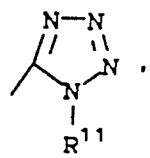

(q)

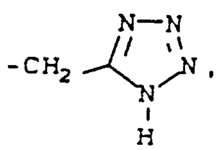

(r)

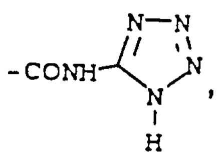

(s) $-CONHNHSO_2CF_3$ ,

(t)

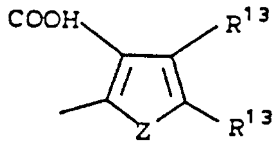

(u)

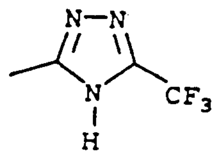

(v)

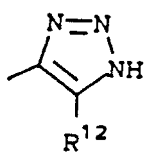 ;

(w)

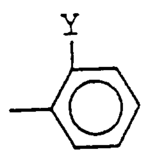

worin Heteroaryl ein unsubstituierter, monosubstituierter oder disubstituierter 5- oder 6-gliedriger aromatischer Ring ist, der gegebenenfalls 1 bis 3 Heteroatome, ausgewählt aus der Gruppe, die aus O, N und S besteht, enthalten kann, und worin die Substituenten Glieder sind, die aus der Gruppe ausgewählt sind, die aus -OH, -SH, $C_1$-$C_4$-Alkyl, $C_1$-$C_4$-Alkoxy, $-CF_3$, Halogen (Cl, Br, F, I), $-NO_2$, $-CO_2H$, $-CO_2$-$C_1$-$C_4$-Alkyl, $-NH_2$, -NH($C_1$-$C_4$-Alkyl) oder -N($C_1$-$C_4$-Alkyl)$_2$ besteht, und

-Y bedeutet

(1) $-CO_2R^4$,
(2) $-SO_3R^5$,
(3) $-NHSO_2CF_3$,
(4) $-PO(OR^5)_2$, oder
(5) $-SO_2$-NH-$R^9$;
(6) 1H-Tetrazol-5-yl;

$R^{2a}$ und $R^{2b}$ bedeuten beide unabhängig voneinander

(a) Wasserstoff
(b) Halogen (Cl, Br, F, I),
(c) $-NO_2$,
(d) $NH_2$,
(e) $C_1$-$C_4$-Alkylamino,
(f) $-SO_2NHR^9$,
(g) $CF_3$,
(h) $C_1$-$C_4$-Alkyl,
(i) $C_1$-$C_4$-Alkoxy; oder,

wenn $R^{2a}$ und $R^{2b}$ sich an benachbarten Kohlenstoffatomen befinden, können sie miteinander unter Bildung eines Phenylrings verbunden sein;

$R^{3a}$ bedeutet

(a) H,
(b) Halogen (Cl, Br, I, F),
(c) $C_1$-$C_6$-Alkyl,
(d) $C_1$-$C_6$-Alkoxy,
(e) $C_1$-$C_6$-Alkoxy-$C_1$-$C_4$-Alkyl,

$R^{3b}$ bedeutet

(a) H,
(b) Halogen (Cl, Br, I, F),
(c) $NO_2$,
(d) $C_1$-$C_6$-Alkyl,
(e) $C_1$-$C_5$-Alkylcarbonyloxy,
(f) $C_3$-$C_6$-Cycloalkyl,
(g) $C_1$-$C_6$-Alkoxy,
(h) $-NHSO_2R^4$,
(i) Hydroxy-$C_1$-$C_4$-alkyl,
(j) Aryl-$C_1$-$C_4$-alkyl,
(k) $C_1$-$C_4$-Alkylthio,
(l) $C_1$-$C_4$-Alkylsulfinyl,
(m) $C_1$-$C_4$-Alkylsulfonyl,
(n) $NH_2$,
(o) $C_1$-$C_4$-Alkylamino,
(p) Di-($C_1$-$C_4$-alkyl)amino,
(q) $CF_3$,
(r) $-SO_2$-$NHR^9$,
(s) Aryl,
(t) Furyl; oder

wenn $R^{3a}$ und $R^{3b}$ sich an benachbarten Kohlenstoffatomen befinden, können sie miteinander unter Bildung eines Phenylrings verbunden sein;
worin Aryl Phenyl oder Naphthyl bedeutet, die gegebenenfalls mit einem oder zwei Substituenten substituiert sind, die aus der Gruppe ausgewählt sind, die aus Halogen (Cl, Br, I, F), $C_1$-$C_4$-Alkyl, $C_1$-$C_4$-Alkoxy, $NO_2$, $CF_3$, $C_1$-$C_4$-Alkylthio, OH oder $NH_2$ besteht,

$R^4$ bedeutet H, geradkettiges oder verzweigtes $C_1$-$C_6$-Alkyl, Benzyl oder Phenyl;
$R^5$ bedeutet H oder $-CH(R^4)$-O-CO-$R^4$;
E bedeutet eine Einfachbindung, $-NR^{13}(CH_2)_s-$, $-S(O)_x(CH_2)_s-$, worin x 0 bis 2 und s 0 bis 5 ist, -CH(OH)-, $-O(CH_2)_s-$, -CO-;
$R^6$ bedeutet

(a) Aryl, das wie oben definiert und gegebenenfalls durch 1 oder 2 Substituenten substituiert ist, die aus der Gruppe ausgewählt sind, welche aus Halogen (Cl, Br, I,

F), $-O-C_1-C_4$-Alkyl, $C_1-C_4$-Alkyl, $NO_2$, $-CF_3$, $-SO_2NR^9R^{10}$, $-S-C_1-C_4$-Alkyl, -OH, $-NH_2$, $C_3-C_7$-Cycloalkyl, $C_3-C_{10}$-Alkenyl besteht;

(b) geradkettiges oder verzweigtes $C_1-C_6$-Alkyl, $C_2-C_6$-Alkenyl oder $C_2-C_6$-Alkinyl, von denen jedes gegebenenfalls mit einem oder mehreren Substituenten substituiert sein kann, welche aus der Gruppe ausgewählt sind, die aus Aryl, wie oben definiert, $C_3-C_7$-Cycloalkyl, Halogen (Cl, Br, I, F), -OH, $-O-C_1-C_4$-Alkyl, $-NH_2$, $-NH(C_1-C_4$-Alkyl), $-N(C_1-C_4\text{-Alkyl})_2$, $-NH-SO_2R^4$, $-COOR^4$, $-SO_2NHR^9$, $-S-C_1-C_4$-Alkyl besteht;

(c) einen unsubstituierten, monosubstituierten oder disubstituierten 5- oder 6-gliedrigen aromatischen Ring , der 1 oder 2 Heteroatome, ausgewählt aus der Gruppe, die aus N, O, S besteht, enthalten kann, und worin die Substituenten Glieder sind, die aus der Gruppe ausgewählt sind, die aus -OH, -SH, $C_1-C_4$-Alkyl, $C_1-C_4$-Alkyloxy, $-CF_3$, Halogen (Cl, Br, F, I) oder $-NO_2$ besteht;

(d) Mono-, Di-, Tri- oder Polyfluor-$C_1-C_5$-alkyl;

(e) $C_3-C_7$-Cycloalkyl, gegebenenfalls durch einen oder mehrere Substituenten substituiert, die aus der Gruppe ausgewählt sind, welche aus $C_1-C_4$-Alkyl, $O-C_1-C_4$-Alkyl, $S-C_1-C_4$-Alkyl, OH, Perfluor-$C_1-C_4$-alkyl oder Halogen (Cl, Br, F, I) besteht; oder

(f) $C_3-C_7$-Cycloalkyl-$C_1-C_3$-alkyl, worin das Cycloalkyl so wie oben unter (e) substituiert ist;

A bedeutet =O, =S oder $=NR^{21}$;

B bedeutet

(a) H, unter der Bedingung, daß A nicht $NR^{21}$ ist;

(b) $C_1-C_{10}$-Alkyl;

(c) substituiertes $C_1-C_{10}$-Alkyl, in welchem ein oder mehrere Substituenten ausgewählt sind aus

(1) Halogen (I, Br, Cl, F)
(2) Hydroxy,
(3) $C_1-C_{10}$-Alkoxy,
(4) $C_1-C_5$-Alkoxycarbonyl,
(5) $C_1-C_4$-Alkylcarbonyloxy,
(6) $C_3-C_8$-Cycloalkyl,
(7) Aryl,
(8) substituiertes Aryl, worin die Substituenten $V_1$, $V_2$, $V_3$, $V_4$ und $V_5$ sind,
(9) $C_1-C_{10}$-Alkyl-$S(O)_p$, worin p 0 bis 2 ist,
(10) $C_3-C_8$-Cycloalkyl-$S(O)_p$,
(11) Phenyl-$S(O)_p$,
(12) substituiertes Phenyl-$S(O)_p$, worin die Substituenten $V_1$-$V_5$ sind,
(13) Oxo,
(14) Carboxy,
(15) $NR^9R^9$,
(16) $C_1-C_5$-Alkylaminocarbonyl,
(17) Di-($C_1-C_5$-alkyl)-aminocarbonyl,
(18) Cyan;

(d) $C_2-C_{10}$-Alkenyl,

(e) $C_2-C_{10}$-Alkinyl,

(f) $C_3-C_8$-Cycloalkyl,

(g) substituiertes $C_3-C_8$-Cycloalkyl oder substituiertes $C_3-C_8$-Cycloalkyl-$C_1-C_4$-alkyl mit einem oder mehreren Substituenten ausgewählt aus der Gruppe:

(1) Halogen (Cl, Br, F, I),
(2) Hydroxy,
(3) $C_1-C_6$-Alkyl,
(4) $C_1-C_6$-Alkoxy,
(5) $C_1-C_4$-Alkylcarbonyloxy,
(6) $C_1-C_5$-Alkoxycarbonyl,
(7) Carboxy,

(8) Oxo,
(9) $C_1$-$C_5$-Alkylaminocarbonyl,
(10) Di-($C_1$-$C_5$-alkyl)aminocarbonyl,
(11) $C_1$-$C_4$-Alkylcarbonyl,
(12) Aryl,
(13) substituiertes Aryl, worin die Substituenten $V_1$, $V_2$, $V_3$, $V_4$ und $V_5$ sind,

(h) Aryl,
(i) substituiertes Aryl, worin die Substituenten $V_1$, $V_2$, $V_3$, $V_4$ und $V_5$ sind,
(j) Aryl-$(CH_2)_r$-$(Q)_c$-$(CH_2)_t$-,
(k) substituiertes Aryl-$(CH_2)_r$-$(Q)_c$-$(CH_2)_t$-, worin die Arylgruppe mit $V_1$, $V_2$, $V_3$, $V_4$ und $V_5$ substituiert ist,
(l) einen heterocyclischen Ring mit 5 bis 6 Atomen, der ein oder zwei Heteroatome enthält, ausgewählt aus:

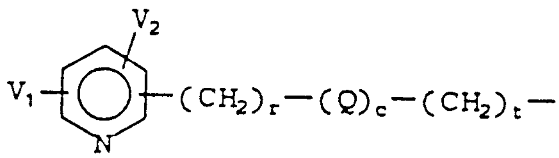

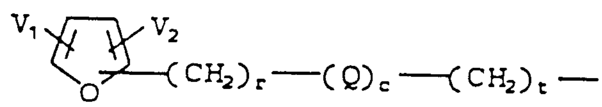

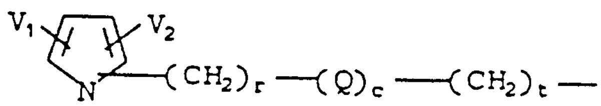

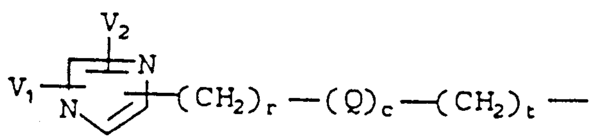

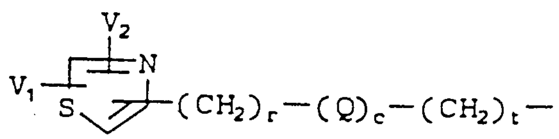
;

R9 bedeutet H, $C_1$-$C_5$-Alkyl, Phenyl oder Benzyl,
R10 bedeutet H, $C_1$-$C_4$-Alkyl, oder
R9 und R10 können zusammen $-(CH_2)_m-$, worin m 3-6 ist, bedeuten;
R11 bedeutet H, $C_1$-$C_6$-Alkyl, $C_2$-$C_4$-Alkenyl, $C_1$-$C_4$-Alkoxy-$C_1$-$C_4$-alkyl oder $-CH_2-C_6H_4R^{20}$;
R12 bedeutet -CN, $-NO_2$ oder $-CO_2R^4$;
R13 bedeutet H, $C_1$-$C_4$-Acyl, $C_1$-$C_6$-Alkyl, Allyl, $C_3$-$C_6$-Cycloalkyl, Phenyl oder Benzyl:
R14 bedeutet H, $C_1$-$C_8$-Alkyl, $C_1$-$C_8$-Perfluoralkyl, $C_3$-$C_6$-Cycloalkyl, Phenyl oder Benzyl;
R15 bedeutet H, $C_1$-$C_6$-Alkyl, Hydroxy;
R16 bedeutet H, $C_1$-$C_6$-Alkyl, $C_3$-$C_6$-Cycloalkyl, Phenyl oder Benzyl;

$R^{17}$ bedeutet $-NR^9R^{10}$, $-OR^{10}$, $-NHCONH_2$, $-NHCSNH_2$, $-NHSO_2CF_3$,

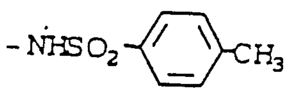 oder 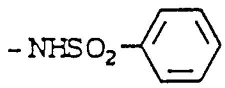 ;

$R^{18}$ und $R^{19}$ bedeuten unabhängig voneinander $C_1$-$C_4$-Alkyl oder zusammen $-(CH_2)_q-$, worin q 2 oder 3 ist,

$R^{20}$ bedeutet H, $-NO_2$, $-NH_2$, -OH oder $-OCH_3$;

$R^{21}$ bedeutet

(a) H,
(b) Aryl, das wie oben definiert und gegebenenfalls durch 1 oder 2 Substituenten substituiert ist, die aus der Gruppe ausgewählt sind, welche aus Halogen (Cl, Br, I, F), $-O-C_1$-$C_4$-Alkyl, $C_1$-$C_4$-Alkyl, $-NO_2$, $-CF_3$, $-SO_2NR^9R^{10}$, $-S-C_1$-$C_4$-Alkyl, -OH, -$NH_2$, $-COOR^4$, $C_3$-$C_7$-Cycloalkyl, $C_3$-$C_{10}$-Alkenyl besteht;
(c) geradkettiges oder verzweigtes $C_1$-$C_6$-Alkyl, $C_2$-$C_6$-Alkenyl oder $C_2$-$C_6$-Alkinyl, von denen jedes gegebenenfalls mit einem oder mehreren Substituenten substituiert sein kann, welche aus der Gruppe ausgewählt sind, die aus Aryl, wie oben definiert, $C_3$-$C_7$-Cycloalkyl, Halogen (Cl, Br, I, F), -OH, $-O-C_1$-$C_4$-Alkyl, $-NH_2$, $-NH(C_1$-$C_4$-Alkyl), $-N(C_1$-$C_4$-Alkyl$)_2$, $-NH-SO_2R^4$, $-COOR^4$, $-SO_2NHR^9$, $-S-C_1$-$C_4$-Alkyl besteht;
(d) einen unsubstituierten, monosubstituierten oder disubstituierten 5- oder 6-gliedrigen aromatischen Ring, der 1 oder 2 Heteroatome, ausgewählt aus der Gruppe, die aus N, O, S besteht, enthalten kann, und worin die Substituenten Glieder sind, die aus der Gruppe ausgewählt sind, die aus -OH, -SH, $C_1$-$C_4$-Alkyl, $C_1$-$C_4$-Alkyloxy, $-CF_3$, $-COOR^4$, Halogen (Cl, Br, F, I) oder $-NO_2$ besteht; oder
(e) $C_3$-$C_7$-Cycloalkyl, gegebenenfalls mit einem oder mehreren Substituenten substituiert, welche aus der Gruppe ausgewählt sind, die aus $C_1$-$C_4$-Alkyl, $-O-C_1$-$C_4$-Alkyl, $-S-C_1$-$C_4$-Alkyl, OH, $-COOR^4$, Perfluor-$C_1$-$C_4$-alkyl oder Halogen (Cl, Br, F, I) besteht;

$R^{23}$ bedeutet

(a) Aryl, wie oben definiert,
(b) Heteroaryl, wie oben definiert,
(c) $C_3$-$C_7$-Cycloalkyl,
(d) $C_1$-$C_4$-Alkyl, gegebenenfalls mit einem Substituenten substituiert, der aus der Gruppe ausgewählt ist, die aus Aryl, wie oben definiert, Heteroaryl, wie oben definiert, -OH, -SH, $C_1$-$C_4$-Alkyl, $-O(C_1$-$C_4$-Alkyl), $S(C_1$-$C_4$-Alkyl), $-CF_3$, Halogen (Cl, Br, F, I), $-NO_2$, $-CO_2H$, $-CO_2-C_1$-$C_4$-alkyl, $-NH_2$, $-NH(C_1$-$C_4$-Alkyl), $-N(C_1$-$C_4$-Alkyl$)_2$, -$N(CH_2CH_2)_2L$, wobei L eine Einfachbindung, $CH_2$, O, $S(O)_p$ darstellt, oder $NR^9$, -$PO_3H$, $-PO(OH)(O-C_1$-$C_4$-Alkyl) besteht;

X bedeutet

(a) eine Einfachbindung,
(b) -CO-,
(c) -O-,
(d) -S-,

(e)

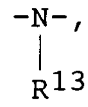

(f)

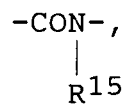

(g)

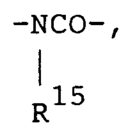

(h) $-OCH_2-$,
(i) $-CH_2O-$,
(j) $-SCH_2-$,
(k) $-CH_2S-$,
(l) $-NHC(R^9)(R^{10})-$,
(m) $-NR^9SO_2-$,
(n) $-SO_2NR^9$,
(o) $-C(R^9)(R^{10})NH-$,
(p) -CH=CH-,
(q) -CF=CF-,
(r) -CH=CF-,
(s) -CF=CH-,
(t) $-CH_2CH_2-$,
(u) $-CF_2CF_2-$,
(v)

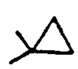 oder 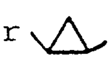

(w)

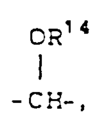

(x)

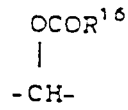

(y)

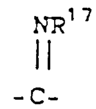 ,

oder

(z)

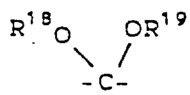 ;

| | |
|---|---|
| Q | bedeutet -C(O)-, -S-, -O- oder $-NR^4$; |
| c | ist 0 oder 1, |
| r und t | sind 0 bis 2; |
| $V_1$, $V_2$, $V_3$, $V_4$ und $V_5$ | sind jedes unabhängig voneinander ausgewählt aus: |

(a) H,
(b) $C_1$-$C_5$-Alkoxy,
(c) $C_1$-$C_5$-Alkyl,
(d) Hydroxy,
(e) $C_1$-$C_5$-Alkyl-$S(O)_p$,
(f) -CN,
(g) $-NO_2$,
(h) $-NR^9R^{10}$,
(i) $C_1$-$C_5$-Alkyl-$CONR^9R^{10}$,
(j) $-CONR^9R^{10}$,
(k) $-CO_2R^9$,
(l) $C_1$-$C_5$-Alkyl-carbonyl,
(m) $CF_3$,
(n) Halogen (I, Br, Cl, F),
(o) Hydroxy-$C_1$-$C_4$-alkyl-,
(p) Carboxy-$C_1$-$C_4$-alkyl-,
(q) -1H-Tetrazol-5-yl,
(r) $-NH-SO_2CF_3$,
(s) Aryl,
(t) $C_1$-$C_5$-Alkyl-$CO_2R^9$,
(u) Aryloxy,
(v) Aryl-$C_1$-$C_3$-alkoxy,
(w) Aryl-$C_1$-$C_3$-alkyl,
(x) Carboxyphenyl,
(y) Heteroaryl,
(z) 2-Oxazolin-2-yl, gegebenenfalls einen oder mehrere $C_1$-$C_4$-Alkylsubstituenten enthaltend,
(aa) $-(CH_2)_tOCOR^9$,

(bb) $-(CH_2)_tOCONR^9R^{10}$,
(cc) $-(CH_2)_tNR^4COR^9$,
(dd) $-(CH_2)_tNR^4CO_2R^9$,
(ee) $-(CH_2)_tNR^4CONR^9R^{10}$,
(ff) $-(CH_2)_tNR^4CON(CH_2CH_2)_2G$,
(gg) $-(CH_2)_tOCON(CH_2CH_2)_2G$,
(hh) $-N(CH_2CH_2)_2G$,
(ii) $-C_1$-$C_5$-Alkyl-$CON(CH_2CH_2)_2G$,
(jj) $-CON(CH_2CH_2)_2G$,

worin G O, $S(O)_p$ oder $NR^9$ bedeutet,

| | |
|---|---|
| u | ist 1 oder 2; |
| Z | ist O, $NR^{13}$ oder S; und |

die pharmazeutisch annehmbaren Salze hiervon.

**2.** Die Verbindung des Anspruchs 1, worin

$R^1$

(a) -COOH,
(b)

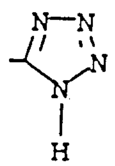
,

(c) $-NH-SO_2CF_3$,
(d) $-CONH-SO_2R^{23}$,
(e) $-SO_2NH-COR^{23}$,
(f) $-SO_2NH$-heteroaryl bedeutet;

| | |
|---|---|
| $R^{2a}$ | bedeutet H; |
| $R^{2b}$ | bedeutet H, F, Cl, $CF_3$ oder $C_1$-$C_4$-Alkyl; |
| $R^{3a}$ | bedeutet H; |
| $R^{3b}$ | bedeutet H, F, Cl, $CF_3$, $C_1$-$C_4$-Alkyl, $C_5$-$C_6$-Cycloalkyl, $-COOCH_3$, $-COOC_2H_5$, $-SO_2$-$CH_3$, $NH_2$, $-N(C_1$-$C_4$-Alkyl$)_2$ oder $-NH-SO_2CH_3$; |
| E | bedeutet eine Einfachbindung, -O- oder -S-; |
| $R^6$ | bedeutet |

(a) $C_1$-$C_6$-Alkyl, gegebenenfalls durch einen Substituenten substituiert, der aus der Gruppe ausgewählt ist, welche aus Cl, $CF_3$, -OH, $-O-CH_3$, $-OC_2H_5$, $-S-CH_3$, $-S-C_2H_5$, Phenyl oder Cyclopropyl besteht;
(b) $C_2$-$C_6$-Alkenyl oder $C_2$-$C_6$-Alkinyl;
(c) Aryl, das wie oben definiert und gegebenenfalls durch einen Substituenten substituiert ist, der aus der Gruppe ausgewählt sind, welche aus Halogen (Cl, F, Br, I), -$CF_3$, $-NO_2$, -OH, $-NH_2$, $-S-CH_3$, $-S-C_2H_5$, $-SO_2NH_2$, $-O-CH_3$ besteht;
(d) ein Heteroaryl, das ein Glied ausgewählt aus der Gruppe ist, welche aus 2-Pyridyl, 4-Pyridyl, 2-Pyrimidyl, 4-Pyrimidyl, Imidazolyl, Thiazolyl, Thienyl oder Furyl besteht; oder
(e) Perfluor-$C_1$-$C_4$-alkyl, das ein Glied ausgewählt aus der Gruppe ist, welche aus $CF_3$-, $CF_3CF_2$-, $CF_3CF_2CF_2$- oder $CF_3CF_2CF_2CF_2$- besteht;
(f) $C_3$-$C_7$-Cycloalkyl, gegebenenfalls durch einen Substituenten substituiert, der aus der Gruppe ausgewählt ist, welche aus Methyl, Ethyl, $CF_3$ oder $CF_3CF_2$ besteht;

A bedeutet =O, =S oder $=NR^{21}$;

B bedeutet

(a) H, unter der Bedingung, daß A nicht $NR^{21}$ ist;
(b) $C_1$-$C_{10}$-Alkyl;
(c) substituiertes $C_1$-$C_{10}$-Alkyl, in welchem ein oder zwei Substituenten ausgewählt sind aus

(1) Hydroxy,
(2) $C_1$-$C_5$-Alkoxy,
(3) $C_1$-$C_5$-Alkoxycarbonyl,
(4) $C_1$-$C_4$-Alkylcarbonyloxy,
(5) $C_3$-$C_8$-Cycloalkyl,
(6) Phenyl,
(7) substituiertes Phenyl, worin die Substituenten $V_1$, $V_2$, $V_3$, $V_4$ und $V_5$ sind,
(8) $C_1$-$C_5$-Alkyl-$S(O)_p$,
(9) Phenyl-$S(O)_p$,
(10) substituiertes Phenyl-$S(O)_p$, worin der Substituent V ist,
(11) Oxo,
(12) Carboxy,
(13) $C_1$-$C_5$-Alkylaminocarbonyl;

(d) Mono-, Di-, Tri- oder Polyfluor-$C_1$-$C_{10}$-alkyl,
(e) $C_2$-$C_{10}$-Alkenyl,
(f) $C_2$-$C_{10}$-Alkinyl
(g) $C_3$-$C_8$-Cycloalkyl,
(h) substituiertes $C_3$-$C_8$-Cycloalkyl oder substituiertes $C_3$-$C_8$-Cycloalkyl-$C_1$-$C_4$-alkyl, in welchen ein oder mehrere Substituenten ausgewählt sind aus:

(1) Hydroxy,
(2) $C_1$-$C_5$-Alkoxy,
(3) $C_1$-$C_5$-Alkoxycarbonyl,
(4) $C_1$-$C_5$-Alkylcarbonyloxy,
(5) $C_1$-$C_6$-Alkyl,
(6) Phenyl,
(7) substituiertes Phenyl, worin die Substituenten $V_1$, $V_2$, $V_3$, $V_4$ und $V_5$ sind,
(8) Oxo,
(9) Carboxy,
(10) $C_1$-$C_5$-Alkylaminocarbonyl,

(i) Aryl,
(j) substituiertes Aryl worin die Substituenten $V_1$, $V_2$, $V_3$, $V_4$ und $V_5$ sind,
(k) Aryl-$(CH_2)_r$-$(Q)_c$-$(CH_2)_t$-,
(l) substituiertes Aryl-$(CH_2)_r$-$(Q)_c$-$(CH_2)_t$-,

(m) ein heterocyclischer Ring mit 5 bis 6 Atomen, der ein oder zwei Heteroatome enthält, ausgewählt aus:

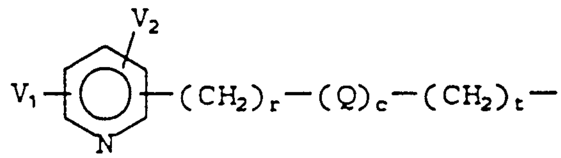

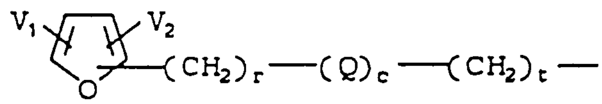

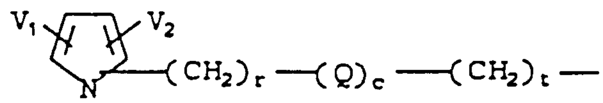

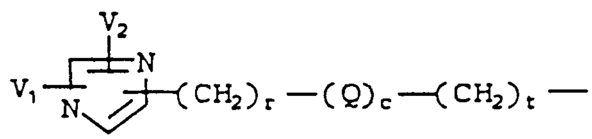

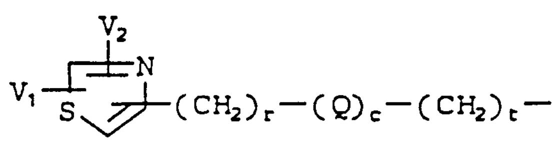
;

$V_1$, $V_2$, $V_3$, $V_4$ und $V_5$ sind unabhängig voneinander ausgewählt aus:

(a) Wasserstoff,
(b) $C_1$-$C_5$-Alkoxy,
(c) $C_1$-$C_5$-Alkyl,
(d) Hydroxy,
(e) $-NR^9R^{10}$,
(f) $-CO_2R^9$,
(g) Trifluormethyl,
(h) Halogen,
(i) Hydroxy-$C_1$-$C_4$-alkyl-,
(j) -1H-Tetrazol-5-yl,
(k) $-NH-SO_2CF_3$,
(l) CN,
(m) $NO_2$,
(n) $C_1$-$C_5$-Alkyl-$CO_2R^9$,
(o) Aryl,
(p) Aryl-$C_1$-$C_3$-alkyl,
(q) Heteroaryl,
(r) $C_1$-$C_5$-Alkyl-$CONR^9R^{10}$,
(s) $-CONR^9R^{10}$,
(t) 2-Oxazolin-2-yl, gegebenenfalls einen oder mehrere $C_1$-$C_4$-Alkylsubstituenten enthaltend,
(u) $C_1$-$C_5$-Alkyl-$S(O)_p$,
(v) $-(CH_2)_tOCOR^9$,
(w ) $-(CH_2)_tNR^4COR^9$;

u ist 1;

X bedeutet

(a) eine Einfachbindung,
(b) -C(O)-;
(c) $-NR^{15}C(O)-$.

**3.** Die Verbindung des Anspruchs 2, worin:

E eine Einfachbindung oder -S-;
$R^{2a}$, $R^{2b}$, $R^{3a}$ und $R^{3bH}$ jedes Wasserstoff;
$R^6$ $C_1$-$C_6$-Alkyl bedeuten.

**4.** Die Verbindung des Anspruchs 3, worin:

A bedeutet =O, =S oder $=NR^{21}$;

B bedeutet

(a) H, unter der Bedingung, daß A nicht $NR^{21}$ ist,
(b) $C_1$-$C_{10}$-Alkyl,
(c) $C_3$-$C_8$-Cycloalkyl,
(d) $C_3$-$C_8$-Cycloalkyl-$C_1$-$C_4$-alkyl,
(e) substituiertes $C_1$-$C_{10}$-Alkyl, $C_3$-$C_8$-Cycloalkyl, oder $C_3$-$C_8$-Cycloalkyl-$C_1$-$C_4$-alkyl, von denen jedes einen oder zwei Substituenten enthalten kann, ausgewählt aus der Gruppe:

(1) Hydroxy,
(2) $C_1$-$C_5$-Alkoxy,
(3) $C_1$-$C_5$-Alkoxycarbonyl,
(4) Phenyl,
(5) Carboxy
(6) $C_1$-$C_5$-Alkylaminocarbonyl,
(7) Oxo;

(f) Mono-, Di-, Tri- oder Polyfluor-$C_1$-$C_{10}$-alkyl,
(g) Phenyl,
(h) Phenyl substituiert mit $V_1$, $V_2$, $V_3$, $V_4$ und $V_5$,
(i) Phenyl-$(CH_2)_r$-$(Q)_c$-$(CH_2)_t$-,
(j) Phenyl-$(CH_2)_r$-$(Q)_c$-$(CH_2)_t$-, worin Phenyl mit $V_1$, $V_2$, $V_3$, $V_4$ und $V_5$ substituiert ist,
(k) eine heterocyclische Gruppierung ausgewählt aus:

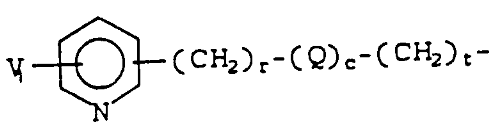

oder

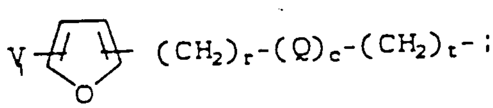

$V_1$, $V_2$, $V_3$, $V_4$ und $V_5$ sind ausgewählt aus:

(a) Wasserstoff,
(b) $C_1$-$C_5$-Alkyl,

(c) $C_1$-$C_5$-Alkoxy,
(d) $CO_2R^9$,
(e) Halogen,
(f) Hydroxy-$C_1$-$C_4$-alkyl-,
(g) $C_1$-$C_5$-Alkyl-$CO_2R^9$,
(h) $C_1$-$C_5$-Alkyl-$CONR^9R^{10}$,
(i) $CONR^9R^{10}$,
(j) CN,
(k) $NO_2$,
(l) $CF_3$,
(m) Aryl,
(n) Heteroaryl,
(o) 2-Oxazolin-2-yl, gegebenenfalls einen oder mehrere $C_1$-$C_4$-Alkylsubstituenten enthaltend,
(p) $C_1$-$C_5$-Alkyl-$S(O)_p$,
(q) $(CH_2)_tOCOR^9$,
(r) $(CH_2)_tNR^4COR^9$,
(s) Hydroxy,
(t) $NR^9R^{10}$;

X bedeutet $-NR^{15}C(O)-$ oder eine Kohlenstoff-Kohlenstoff-Einfachbindung.

**5.** Die Verbindung des Anspruchs 4, worin:

| | |
|---|---|
| $R^1$ | 5-Tetrazolyl oder Carboxy; |
| $R^{2a}$, $R^{2b}$, $R^{3a}$ und $R^{3b}$ | jedes Wasserstoff; |
| $R^6$ | $C_1$-$C_6$-Alkyl; |
| A | =O oder =S; |
| B | H, substituiertes oder unsubstituiertes $C_1$-$C_{10}$-Alkyl, $C_3$-$C_8$-Cycloalkyl oder $C_3$-$C_8$-Cycloalkyl-$C_1$-$C_4$-alkyl, substituiertes oder unsubstituiertes Aryl, Heteroaryl oder Aralkyl bedeuten; |
| E und X | sind jedes Einfachbindungen; und |
| u | ist 1. |

**6.** Die Verbindung des Anspruchs 4, worin:

| | |
|---|---|
| $R^1$ | 5-Tetrazolyl oder Carboxy; |
| $R^{2a}$, $R^{2b}$, $R^{3a}$ und $R^{3b}$ | jedes Wasserstoff; |
| $R^6$ | $C_1$-$C_6$-Alkyl; |
| A | O; |
| B | H, substituiertes oder unsubstituiertes $C_1$-$C_{10}$-Alkyl, substituiertes oder unsubstituiertes Aralkyl oder Heteroarylalkyl bedeuten; |
| E und X | sind jedes Einfachbindungen; und |
| u | ist 1. |

**7.** Die Verbindung des Anspruchs 4, worin:

| | |
|---|---|
| $R^1$ | 5-Tetrazolyl oder Carboxy; |
| $R^{2a}$, $R^{2b}$, $R^{3a}$ und $R^{3b}$ | jedes Wasserstoff; |
| $R^6$ | $C_1$-$C_6$-Alkyl; |
| A | O; |
| B | substituiertes oder unsubstituiertes Aryl oder Heteroaryl bedeuten; |
| E und X | sind jedes Einfachbindungen; und |
| u | ist 1. |

**8.** Die Verbindung des Anspruchs 4, worin:

| | |
|---|---|
| $R^1$ | 5-Tetrazolyl, Carboxy, $-CONHSO_2R^{23}$, $-SO_2NHCOR^{23}$ oder $-SO_2NH$-heteroaryl; |
| $R^{2a}$, $R^{2b}$, $R^{3a}$ und $R^{3bH}$ | jedes Wasserstoff; |

| | |
|---|---|
| $R^6$ | $C_1$-$C_6$-Alkyl; |
| A | O, S oder $NR^{21}$; |
| B | $C_1$-$C_{10}$-Alkyl, substituiertes oder unsubstituiertes Aryl oder Aralkyl; |
| $R^{21}$ | H, substituiertes oder unsubstituiertes Aryl; |
| $R^{23}$ | |

(a) substituiertes oder unsubstituiertes Aryl,
(b) substituiertes oder unsubstituiertes Alkyl

| | |
|---|---|
| E | eine Einfachbindung oder S; |
| X | eine Einfachbindung, -NHCO- oder -CO- bedeuten; und |
| u | 1 ist. |

9. Eine pharmazeutische Zusammensetzung, welche bei der Behandlung von Hypertonie verwendbar ist und welche einen pharmazeutisch annehmbaren Träger und eine pharmazeutisch wirksame Menge einer Verbindung des Anspruchs 1 umfaßt.

10. Die Zusammensetzung des Anspruchs 9, welche ein Antihypertonikum oder ein Diuretikum oder einen Angiotensin-Conversionsenzymhemmer oder einen Calciumkanalblocker enthält, die aus der Gruppe ausgewählte Glieder sind, welche aus Amilorid, Atenolol, Bendroflumethiazid, Chlorothalidon, Chlorothiazid, Clonidin, Cryptenaminacetaten und Cryptenamintannaten, Deserpidin, Diazoxid, Guanethidinsulfat, Hydralazinhydrochlorid, Hydrochlorothiazid, Metolazon, Metoprololtartrat, Methylclothiazid, Methyldopa, Methyldopathydrochlorid, Minoxidil, Pargylinhydrochlorid, Polythiazid, Prazosin, Propanolol, Rauwolfia serpentina, Rescinnamin, Reserpin, Nitroprussidnatrium, Spironolacton, Timololmaleat, Trichlormethiazid, Trimethophancamsylat, Benzthiazid, Chinethazon, Ticrynafan, Triamteren, Acetazolamid, Aminophyllin, Cyclothiazid, Ethacrinsäure, Furosemid, Merethoxyllinprocain, Natriumethacrinat, Captopril, Delaprilhydrochlorid, Enalapril, Enalaprilat, Fosinoprilnatrium, Lisinopril, Pentopril, Chinaprilhydrochlorid, Ramapril, Teprotid, Zofenoprilcalcium, Diflusinal, Diltiazem, Felodipin, Nicardipin, Nifedipin, Niludipin, Nimodipin, Nisoldipin, Nitrendipin, Verapamil besteht, ebenso aus Mischungen und Kombinationen hiervon.

11. Eine ophthalmologische Formulierung zur Behandlung der Augenhypertonie, die einen ophthalmologisch annehmbaren Träger und eine gegen Augenhypertonie wirksame Menge einer Verbindung des Anspruchs 1 enthält.

**Revendications**

1. Composé de formule (I) :

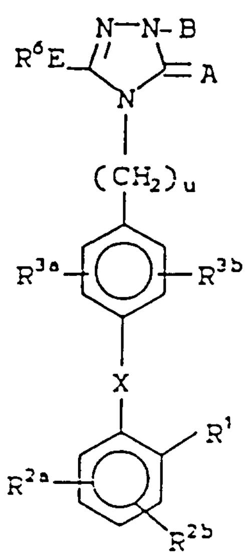

(I)

dans laquelle :
$R^1$ représente

(a) $-CO_2R^4$,
(b) $-SO_3R^5$,
(c) $-NHSO_2CF_3$
(d) $-PO(OR^5)_2$,
(e) $-SO_2-NH-R^9$,
(f) $-CONHOR^5$,
(g) $-SO_2NH$-hétéroaryle,
(h) $-CH_2SO_2NH$-hétéroaryle,
(i) $-SO_2NHCOR^{23}$,
(j) $-CH_2SO_2NHCOR^{23}$,
(k) $-CONHSO_2R^{23}$,
(l) $-CH_2CONHSO_2R^{23}$,
(m) $-NHSO_2NHCOR^{23}$,
(n) $-NHCONHSO_2R^{23}$,
(o)

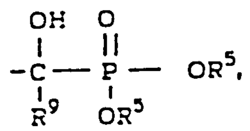

(p)

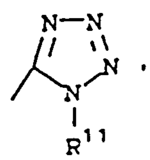

(q)

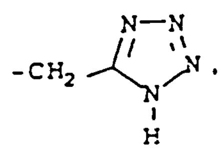

(r)

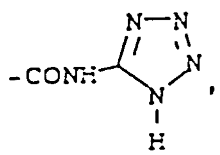

(s) $-CONHNHSO_2CF_3$ ,

(t)

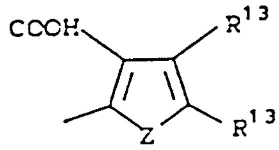

(u)

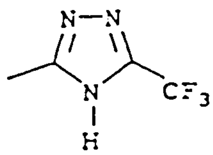

(v)

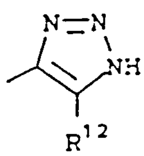 ;

(w)

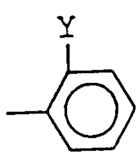 ;

où :

hétéroaryle est un noyau aromatique à 5 ou 6 chaînons non-substitué, monosubstitué ou disubstitué qui peut facultativement contenir de 1 à 3 hétéroatomes choisis dans le groupe constitué par O, N et S et où les substituants sont des membres choisis dans le groupe constitué par -OH, -SH, alkyle en $C_{1-4}$, alcoxy en $C_{1-4}$, - $CF_3$, halo (Cl, Br, F, I), -$NO_2$, -$CO_2H$, -$CO_2$-alkyle en $C_{1-4}$, -$NH_2$, -NH(alkyle en $C_{1-4}$) ou -N(alkyle en $C_{1-4}$)$_2$ et

Y représente

(1) -$CO_2R^4$,
(2) -$SO_3R^5$,
(3) -$NHSO_2CF_3$,
(4) -$PO(OR^5)_2$, ou
(5) -$SO_2$-NH-$R^9$ ;
(6) 1H-tétrazol-5-yle ;

$R^{2a}$ et $R^{2b}$ représentent indépendamment :

(a) un hydrogène,
(b) un halogène (Cl, Br, I, F)

(c) -$NO_2$,
(d) $NH_2$,
(e) alkylamino en $C_{1-4}$,
(f) -$SO_2NHR^9$,
(g) $CF_3$,
(h) alkyle en $C_{1-4}$,
(i) alcoxy en $C_{1-4}$ ; ou

lorsque $R^{2a}$ et $R^{2b}$ sont des atomes de carbone adjacents, ils peuvent être liés ensemble pour former un noyau phényle ;
$R^{3a}$ représente

(a) H,
(b) halo (Cl, Br, I, F)
(c) alkyle en $C_{1-6}$,
(d) alcoxy en $C_{1-6}$,
(e) alcoxy en $C_{1-6}$-alkyle en $C_{1-4}$ ;

$R^{3b}$ représente

(a) H,
(b) halo (Cl, Br, I, F)
(c) $NO_2$,
(d) alkyle en $C_{1-6}$,
(e) alkyle en $C_{1-5}$-carbonyloxy,
(f) cycloalkyle en $C_{3-6}$,
(g) alcoxy en $C_{1-6}$,
(h) -$NHSO_2R^4$,
(i) hydroxy-alkyle en $C_{1-4}$,
(j) aryl-alkyle en $C_{1-4}$,
(k) alkylthio en $C_{1-4}$,
(l) alkyle en $C_{1-4}$-sulfinyle,
(m) alkyle en $C_{1-4}$-sulfonyle,
(n) $NH_2$,
(o) alkylamino en $C_{1-4}$,
(p) di(alkyle en $C_{1-4}$)amino,
(q) $CF_3$,
(r) -$SO_2$-$NHR^9$,
(s) aryle,
(t) furyle ; ou

lorsque $R^{3a}$ et $R^{3b}$ sont sur des atomes de carbone adjacents, ils peuvent être liés ensemble pour former un noyau phényle ;
où aryle est un phényle ou un naphtyle facultativement substitué par un ou deux substituants choisis dans le groupe constitué par halo (Cl, Br, I, F), alkyle en $C_{1-4}$, alcoxy en $C_{1-4}$, $NO_2$, $CF_3$, alkylthio en $C_{1-4}$, OH, ou $NH_2$ ;
$R^4$ représente H, un alkyle en $C_{1-6}$ à chaîne droite ou ramifiée, un benzyle ou un phényle ;
$R^5$ représente H ou -CH($R^4$)-O-CO-$R^4$ ;
E est une liaison simple, -$NR^{13}(CH_2)_s$-, -S-O)$_x$($CH_2$)$_s$- où x vaut de 0 à 2 et s vaut de 0 à 5, -CH(OH)-, -O($CH_2$)$_s$-, -CO- ;
$R^6$ représente

(a) un aryle tel que défini ci-dessus, facultativement substitué par un ou deux substituants choisis dans le groupe constitué par halo (Cl, Br, I, F), -O-alkyle en $C_{1-4}$, alkyle en $C_{1-4}$, -$NO_2$, -$CF_3$, -$SO_2NR^9R^{10}$, -S-alkyle en $C_{1-4}$, -OH, -$NH_2$, cycloakyle en $C_{3-7}$, alcényle en $C_{3-10}$ ;
(b) alkyle en $C_{1-6}$, alcényle en $C_{2-6}$ ou alcynyle en $C_{2-6}$ à chaîne droite ou ramifiée, chacun pouvant être facultativement substitué par un ou plusieurs substituants choisis dans le groupe constitué par aryle tel que défini ci-dessus, cycloalkyle en $C_{3-7}$, halo (Cl, Br, I, F), -OH, -O-alkyle en $C_{1-4}$, -$NH_2$, -NH(alkyle en $C_{1-4}$), -N(alkyle en $C_{1-4}$)$_2$, -NH-$SO_2R^4$, -$COOR^4$, -$SO_2NHR^9$, -S-alkyle en $C_{1-4}$ ;

(c) un noyau aromatique à 5 ou 6 chaînons non-substitué, monosubstitué ou disubstitué qui peut contenir un ou deux hétéroatomes choisis dans le groupe constitué par N, O, S, et où les substituants sont des membres choisis dans le groupe constitué par -OH, -SH, alkyle en $C_{1-4}$, alkyloxy en $C_{1-4}$, $-CF_3$, halo (Cl, Br, I, F), ou $NO_2$ ;

(d) un mono-, di-, tri- ou polyfluoroalkyle en $C_{1-5}$ ;

(e) un cycloalkyle en $C_{3-7}$ facultativement substitué par un ou plusieurs substituants choisis dans le groupe constitué par alkyle en $C_{1-4}$, O-alkyle en $C_{1-4}$, S-alkyle en $C_{1-4}$, OH, perfluoroalkyle en $C_{1-4}$, ou halo (Cl, Br, F, I) ;

(f) cycloalkyle en $C_{3-7}$-alkyle en $C_{1-3}$ où le cycloalkyle est substitué comme dans (e) ci-dessus ;

A représente =O, =S ou $=NR^{21}$ ;
B représente

(a) H à condition que A ne soit pas $NR^{21}$ ;
(b) un alkyle en $C_{1-10}$ ;
(c) un alkyle en $C_{1-10}$ substitué dans lequel un ou plusieurs substituants sont choisis parmi

(1) halogène (I, Br, Cl, F)
(2) hydroxy,
(3) alcoxy en $C_{1-10}$,
(4) alcoxy en $C_{1-5}$-carbonyle,
(5) alkyle en $C_{1-4}$-carbonyloxy,
(6) cycloalkyle en $C_{3-8}$,
(7) aryle,
(8) aryle substitué dans lequel les substituants sont $V_1$, $V_2$, $V_3$, $V_4$ et $V_5$,
(9) alkyle en $C_{1-10}$-$S(O)_p$ où p vaut de 0 à 2,
(10) cycloalkyle en $C_{3-8}$-$S(O)_p$,
(11) phényl-$S(O)_p$,
(12) phényl-$S(O)_p$ dans lequel les substituants dans $V_1$ - $V_5$,
(13) oxo,
(14) carboxy,
(15) $NR^9R^9$,
(16) alkyle en $C_{1-5}$-aminocarbonyle,
(17) di(alkyle en $C_{1-5}$)aminocarbonyle,
(18) cyano,

(d) alcényle en $C_{2-10}$,
(e) alcynyle en $C_{2-10}$,
(f) cycloalkyle en $C_{3-8}$,
(g) cycloalkyle en $C_{3-8}$ substitué ou cycloalkyle en $C_{3-8}$ substitué-alkyle en $C_{1-4}$ ayant un ou plusieurs substituants choisis dans le groupe :

(1) halo (Cl, Br, F, I),
(2) hydroxy,
(3) alkyle en $C_{1-6}$,
(4) alcoxy en $C_{1-6}$,
(5) alkyle en $C_{1-4}$-carbonyloxy,
(6) alcoxy en $C_{1-5}$-carbonyle,
(7) carboxy,
(8) oxo,
(9) alkyle en $C_{1-5}$-aminocarbonyle,
(10) di(alkyle en $C_{1-5}$)aminocarbonyle,
(11) alkyle en $C_{1-4}$-carbonyle,
(12) aryle,
(13) aryle substitué dans lequel les substituants sont $V_1$, $V_2$, $V_3$, $V_4$, et $V_5$ ;

(h) aryle,
(i) aryle substitué dans lequel les substituants sont $V_1$, $V_2$, $V_3$, $V_4$ et $V_5$,
(j) aryl-$(CH_2)_r$-$(Q)_c$-$(CH_2)_t$-
(k) aryl-$(CH_2)_r$-$(Q)_c$-$(CH_2)_t$- dans lequel le groupe aryle est substitué par $V_1$, $V_2$, $V_3$, $V_4$ et $V_5$,

(l) un noyau hétérocyclique à 5 à 6 atomes contenant 1 ou 2 hétéroatomes choisis parmi :

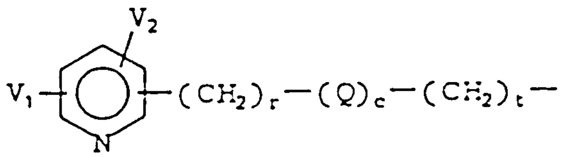

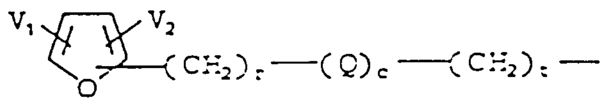

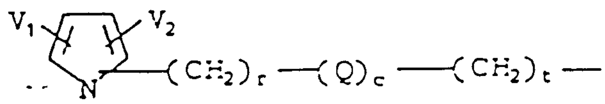

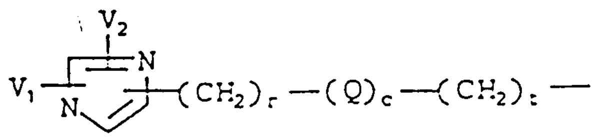

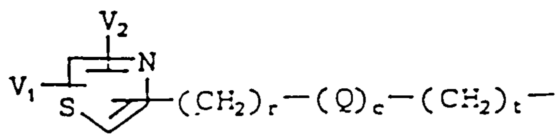 ;

$R^9$ représente H, alkyle en $C_{1-5}$, phényle ou benzyle ;
$R^{10}$ représente H, alkyle en $C_{1-4}$, ou
$R^9$ et $R^{10}$ peuvent représenter ensemble $-(CH_2)_m-$ où m vaut de 3 à 6 ;
$R^{11}$ représente H, alkyle en $C_{1-6}$, alcényle en $C_{2-4}$, alcoxy en $C_{1-4}$-alkyle en $C_{1-4}$, ou $-CH_2-C_6H_4R^{20}$ ;
$R^{12}$ représente -CN, $-NO_2$, ou $-CO_2R^4$ ;
$R^{13}$ représente H, acyle en $C_{1-4}$, alkyle en $C_{1-6}$, allyle, cycloalkyle en $C_{3-6}$, phényle ou benzyle ;
$R^{14}$ représente H, alkyle en $C_{1-8}$, perfluoroalkyle en $C_{1-8}$, cycloalkyle en $C_{3-6}$, phényle ou benzyle ;
$R^{15}$ représente H, alkyle en $C_{1-6}$, hydroxy ;
$R^{16}$ représente H, alkyle en $C_{1-6}$, cycloalkyle en $C_{3-6}$, phényle ou benzyle ;
$R^{17}$ représente $-NR^9R^{10}$, $-OR^{10}$, $-NHCONH_2$, $-NHCSNH_2$, $-NHSO_2CF_3$,

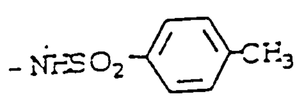 OU 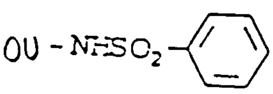 ;

$R^{18}$ et $R^{19}$ représentent indépendamment un alkyle en $C_{1-4}$ ou pris ensemble $-(CH_2)_q-$ où q vaut 2 ou 3 ;
$R^{20}$ représente H, $-NO_2$, $-NH_2$, -OH ou $-OCH_3$ ;
$R^{21}$ représente

(a) H,
(b) un aryle tel que défini ci-dessus facultativement substitué par un ou deux substituants choisis dans le groupe constitué par halo (Cl, Br, I, F), -O-alkyle en $C_{1-4}$, alkyle en $C_{1-4}$, $-NO_2$, $-CF_3$, $-SO_2NR^9R^{10}$, -S-alkyle en $C_{1-4}$, -OH, $-NH_2$, $-COOR^4$, cycloalkyle en $C_{3-7}$, alcényle en $C_{3-10}$,
(c) un alkyle en $C_{1-6}$, alcényle en $C_{2-6}$ ou alcynyle en $C_{2-6}$ à chaîne droite ou ramifiée, chacun pouvant être facultativement substitué par un ou plusieurs substituants choisis dans le groupe constitué par aryle tel que

défini ci-dessus, cycloalkyle en $C_{3-7}$, halo (Cl, Br, I, F), -OH, -O-alkyle en $C_{1-4}$, $-NH_2$, -NH(alkyle en $C_{1-4}$), -N(alkyle en $C_{1-4})_2$, $-NH-SO_2R^4$, $-COOR^4$, $-SO_2NHR^9$, -S-alkyle en $C_{1-4}$ ;

(d) un noyau aromatique à 5 ou 6 chaînons non-substitué, monosubstitué ou disubstitué qui peut contenir un ou deux hétéroatomes choisis dans le groupe constitué par N, O, S et où les substituants sont des membres choisis dans le groupe constitué par -OH, -SH, alkyle en $C_{1-4}$, alkyloxy en $C_{1-4}$, $-CF_3$, $-COOR^4$, halo (Cl, Br, I, F), ou $NO_2$ ; ou

(e) un cycloalkyle en $C_{3-7}$ facultativement substitué par un ou plusieurs substituants choisis dans le groupe constitué par alkyle en $C_{1-4}$, -O-alkyle en $C_{1-4}$, -S-alkyle en $C_{1-4}$, -OH, $-COOR^4$, perfluoroalkyle en $C_{1-4}$, ou halo (Cl, Br, F, I) ;

$R^{23}$ représente

(a) un aryle tel que défini ci-dessus,
(b) un hétéroaryle tel que défini ci-dessus,
(c) un cycloalkyle en $C_{3-7}$,
(d) un alkyle en $C_{1-4}$ facultativement substitué par un substituant choisi dans le groupe constitué par un aryle tel que défini ci-dessus, un hétéroaryle tel que défini ci-dessus, -OH, -SH, un alkyle en $C_{1-4}$, -O(alkyle en $C_{1-4}$), S(alkyle en $C_{1-4}$), $-CF_3$, halo (Cl, Br, F, I), $-NO_2$, $-CO_2H$, $-CO_2$-alkyle en $C_{1-4}$, $-NH_2$, -NH(alkyle en $C_{1-4}$), -N(alkyle en $C_{1-4})_2$, $-N(CH_2CH_2)_2L$ où L est une liaison simple, $CH_2$, O, $S(O)_p$, ou $NR^9$, $-PO_3H$, -PO(OH) (O-alkyle en $C_{1-4}$) ;

X représente

(a) une liaison simple,
(b) -CO-,
(c) -O-,
(d) -S-,
(e)

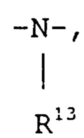

(f)

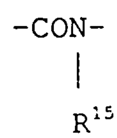

(g)

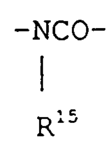

(h) $-OCH_2-$,
(i) $-CH_2O-$
(j) $-SCH_2-$,
(k) $-CH_2S-$,
(l) $-NHC(R^9)(R^{10})-$,

(m) $-NR^9SO_2-$,
(n) $-SO_2NR^9$,
(o) $-C(R^9)(R^{10})NH-$,
(p) -CH=CH-,
(q) -CF=CF-,
(r) -CH=CF-,
(s) -CF=CH-,
(t) $-CH_2CH_2-$,
(u) $-CF_2CF_2-$,
(v)

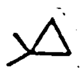 ou 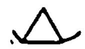

(w)

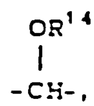

(x)

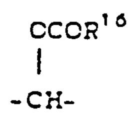

(y)

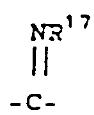 ,

(z)

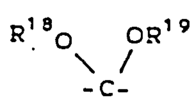 ;

ou

Q représente -C(O)-, -S-, -O- ou $-NR^4$ ;
c vaut 0 ou 1 ;
r et t valent de 0 à 2 ;
$V_1$, $V_2$, $V_3$, $V_4$ et $V_5$ sont chacun choisis indépendamment parmi :

(a) H,
(b) alcoxy en $C_{1-5}$,

(c) alkyle en $C_{1-5}$,
(d) hydroxy,
(e) alkyle en $C_{1-5}$-$S(O)_p$,
(f) -CN,
(g) -$NO_2$,
(h) -$NR^9R^{10}$,
(i) alkyle en $C_{1-5}$-$CONR^9R^{10}$,
(j) -$CONR^9R^{10}$,
(k) -$CO_2R^9$,
(l) alkyle en $C_{1-5}$-carbonyle,
(m) $CF_3$,
(n) halogène (I, Br, Cl, F),
(o) hydroxyalkyle en $C_{1-4}$,
(p) carboxyalkyle en $C_{1-4}$,
(q) -1H-tétrazol-5-yle,
(r) -NH-$SO_2CF_3$,
(s) aryle
(t) alkyle en $C_{1-5}$-$CO_2R^9$,
(u) aryloxy,
(v) aryl-alcoxy en $C_{1-3}$,
(w) aryl-alkyle en $C_{1-3}$,
(x) carboxyphényle,
(y) hétéroaryle,
(z) 2-oxazolin-2-yle portant facultativement un ou plusieurs substituants alkyle en $C_{1-4}$,
(aa) -$(CH_2)_tOCOR^9$,
(bb) -$(CH_2)_tOCONR^9R^{10}$,
(cc) -$(CH_2)_tNR^4COR^9$,
(dd) -$(CH_2)_tNR^4CO_2R^9$,
(ee) -$(CH_2)_tNR^4CONR^9R^{10}$,
(ff) -$(CH_2)_tNR^4CON(CH_2CH_2)_2G$,
(gg) -$(CH_2)_tOCON(CH_2CH_2)_2G$,
(hh) -$N(CH_2CH_2)_2G$,
(ii) -$C_1$-$C_5$-alkyl-$CON(CH_2CH_2)_2G$,
(jj) -$CON(CH_2CH_2)G$,

où G représente O, $S(O)_p$ ou $NR^9$ et
u vaut 1 ou 2 ;
z représente O, $NR^{13}$, ou S ; et
leurs sels pharmaceutiquement acceptables.

**2.** Composé de la revendication 1 dans lequel :
$R^1$ représente

(a) -COOH,
(b)

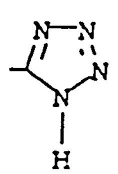 ,

(c) -NH-$SO_2CF_3$;
(d) -CONH-$SO_2R^{23}$,
(e) -$SO_2$NH-$COR^{23}$,
(f) -$SO_2$NH-heteroaryl;
(f) -$SO_2$NH-hétéroaryle ;

$R^{2a}$ représente H,
$R^{2b}$ représente H, F, Cl, $CF_3$, ou alkyle en $C_{1-4}$,
$R^{3a}$ représente H,
$R^{3b}$ représente H, F, Cl, $CF_3$, alkyle en $C_{1-4}$, cycloalkyle en $C_{5-6}$, $-COOCH_3$, $-COOC_2H_5$, $-SO_2-CH_3$, $NH_2$, -N(alkyle en $C_{1-4})_2$ ou $-NH-SO_2CH_3$ ;
E est une liaison simple, -O-, ou -S- ;
$R^6$ représente

(a) un alkyle en $C_{1-6}$ facultativement substitué par un substituant choisi dans le groupe constitué par Cl, $CF_3$, -OH, $-O-CH_3$, $-OC_2H_5$, $-S-CH_3$, $-S-C_2H_5$, phényle, ou cyclopropyle ;
(b) un alcényle en $C_{2-6}$ ou un alcynyle en $C_{2-6}$ ;
(c) un aryle tel que défini ci-dessus facultativement substitué par un substituant choisi dans le groupe constitué par halo (Cl, F, Br, I), $-CF_3$, $-NO_2$, -OH, $-NH_2$, $-S-CH_3$, $-S-C_2H_5$, $-SO_2NH_2$, $-O-CH_3$ ;
(d) un hétéroaryle qui est un membre choisi dans le groupe constitué par 2-pyridyle, 4-pyridyle, 2-pyrimidyle, 4-pyrimidyle, imidazoyle, thiazolyle, thiényle, ou furyle ; ou
(e) un perfluoroalkyle en $C_{1-4}$ qui est un membre choisi dans le groupe constitué par $CF_3$-, $CF_3CF_2$-, $CF_3CF_2CF_2$-, ou $CF_3CF_2CF_2CF_2$- ;
(f) un cycloalkyle en $C_{3-7}$ facultativement substitué par un substituant choisi dans le groupe constitué par méthyle, éthyle, $CF_3$, ou $CF_3CF_2$ ;

A représente =O, =S ou $=NR^{21}$ ;
B représente

(a) H à condition que A ne représente pas $NR^{21}$,
(b) un alkyle en $C_{1-10}$,
(c) un alkyle en $C_{1-10}$ substitué dans lequel un ou deux substituants sont choisis parmi :

(1) hydroxy,
(2) alcoxy en $C_{1-5}$,
(3) alcoxy en $C_{1-5}$-carbonyle,
(4) alkyle en $C_{1-4}$-carbonyloxy,
(5) cycloalkyle en $C_{3-8}$,
(6) phényle,
(7) phényle substitué dans lequel les substituants sont $V_1$, $V_2$, $V_3$, $V_4$ et $V_5$,
(8) alkyle en $C_{1-5}$-$S(O)_p$,
(9) phényl-$S(O)_p$,
(10) phényl-$S(O)_p$ substitué dans lequel le substituant est V,
(11) oxo,
(12) carboxy,
(13) alkyle en $C_{1-5}$-aminocarbonyle ;

(d) mono-, di-, tri-, ou polyfluoroalkyle en $C_{1-10}$,
(e) alcényle en $C_{2-10}$,
(f) alcynyle en $C_{2-10}$,
(g) cycloalkyle en $C_{3-8}$,
(h) cycloalkyle en $C_{3-8}$ substitué ou un cycloalkyle en $C_{3-8}$ substitué-alkyle en $C_{1-4}$, dans lequel un ou plusieurs substituants sont choisis parmi :

(1) hydroxy,
(2) alcoxy en $C_{1-5}$,
(3) alcoxy en $C_{1-5}$-carbonyle,
(4) alkyle en $C_{1-4}$-carbonyloxy,
(5) alkyle en $C_{1-6}$,
(6) phényle,
(7) phényle substitué dans lequel les substituants sont $V_1$, $V_2$, $V_3$, $V_4$ et $V_5$,
(8) oxo,
(9) carboxy,
(10) alkyle en $C_{1-5}$-aminocarbonyle ;

(i) aryle,
(j) aryle substitué dans lequel les substituants sont $V_1$, $V_2$, $V_3$, $V_4$ et $V_5$,
(k) aryl-$(CH_2)_r$-$(Q)_c$-$(CH_2)_t$-,
(l) aryl-$(CH_2)_r$-$(Q)_c$-$(CH_2)_t$- substitué,
(m) un noyau hétérocyclique à 5 à 6 chaînons contenant un ou deux hétéroatomes choisis parmi :

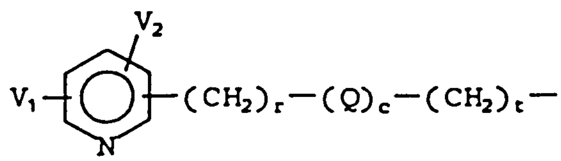

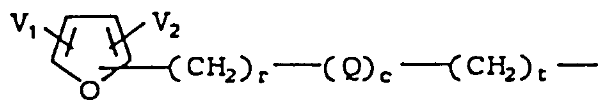

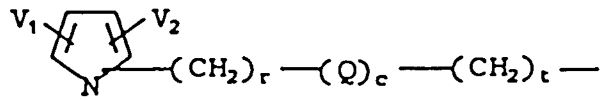

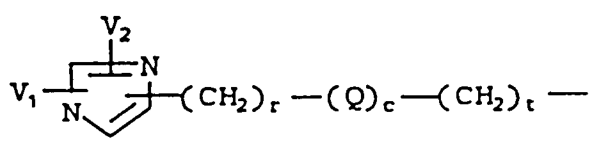

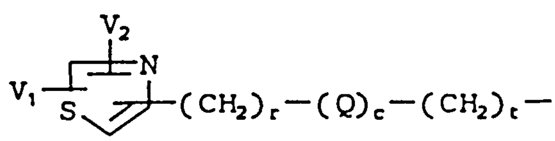

$V_1$, $V_2$, $V_3$, $V_4$ et $V_5$ sont indépendamment choisis parmi :

(a) hydrogène,
(b) alcoxy en $C_{1-5}$,
(c) alkyle en $C_{1-5}$,
(d) hydroxy,
(e) $NR^9R^{10}$,
(f) $CO_2R^9$,
(g) trifluorométhyle,
(h) halogène,
(i) hydroxyalkyle en $C_{1-4}$,
(j) -1H-tétrazol-5-yle,
(k) -NH-$SO_2CF_3$,
(l) CN,
(m) $NO_2$,
(n) alkyle en $C_{1-5}$-$CO_2R^9$,
(o) aryle,
(p) aryl-alkyle en $C_{1-3}$,
(q) hétéroaryle,
(r) alkyle en $C_{1-5}$-$CONR^9R^{10}$,
(s) -$CONR^9R^{10}$,
(t) 2-oxazolin-2-yle portant facultativement un ou plusieurs substituants alkyle en $C_{1-4}$,
(u) alkyle en $C_{1-5}$-$S(O)_p$,
(v) $(CH_2)_tOCOR^9$,

(w) $(CH_2)_tNR^4COR^9$ ;

u vaut 1 ;
X représente

(a) une liaison simple ;
(b) -C(O)- ;
(c) $-NR^{15}C(O)-$.

**3.** Composé de la revendication 2 dans lequel :
E est une liaison simple ou -S- ;
$R^{2a}$, $R^{2b}$, $R^{3a}$ et $R^{3b}$ représentent chacun H ;
$R^6$ est un alkyle en $C_{1-6}$.

**4.** Composé de la revendication 3, dans lequel :
A représente =O, =S, ou $=NR^{21}$ ;
B représente

(a) H à condition que A ne représente pas $NR^{21}$,
(b) alkyle en $C_{1-10}$,
(c) cycloalkyle en $C_{3-8}$,
(d) cycloalkyle en $C_{3-8}$-alkyle en $C_{1-4}$,
(e) alkyle en $C_{1-10}$ ,
cycloalkyle en $C_{3-8}$, ou cycloalkyle en $C_{3-8}$-alkyle en $C_{1-4}$ substitués pouvant chacun avoir un ou deux substituants choisis dans le groupe :

(1) hydroxy,
(2) alcoxy en $C_{1-5}$,
(3) alcoxy en $C_{1-5}$-carbonyle,
(4) phényle,
(5) carboxy,
(6) alkyle en $C_{1-5}$-aminocarbonyle,
(7) oxo,

(f) mono-, di-, tri-, ou polyfluoroalkyle en $C_{1-10}$,
(g) phényle,
(h) phényle substitué par $V_1$, $V_2$, $V_3$, $V_4$ et $V_5$,
(i) phényl-$(CH_2)_r$-$(Q)_c$-$(CH_2)_t$-,
(j) phényl-$(CH_2)_r$-$(Q)_c$-$(CH_2)_t$- dans lequel le phényle est substitué par $V_1$, $V_2$, $V_3$, $V_4$ et $V_5$,
(k) une partie hétérocyclique choisie parmi :

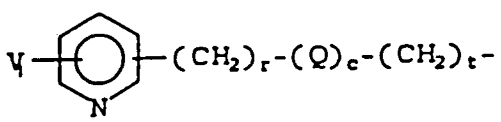 ou

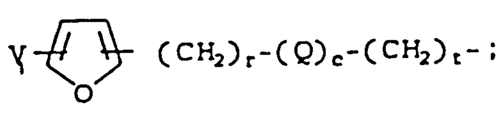

$V_1$, $V_2$, $V_3$, $V_4$ et $V_5$ sont choisis parmi :

(a) hydrogène,
(b) alkyle en $C_{1-5}$,
(c) alcoxy en $C_{1-5}$,
(d) $CO_2R^9$,

(e) halogène,
(f) hydroxyalkyle en $C_{1-4}$,
(g) alkyle en $C_{1-5}$-$CO_2R^9$,
(h) alkyle en $C_{1-5}$-$CONR^9R^{10}$,
(i) $CONR^9R^{10}$,
(j) CN,
(k) $NO_2$,
(l) $CF_3$,
(m) aryle,
(n) hétéroaryle,
(o) 2-oxazolin-2-yle portant facultativement un ou plusieurs substituants alkyle en $C_{1-4}$,
(p) alkyle en $C_{1-5}$-$S(O)_p$,
(q) $(CH_2)_tOCOR^9$,
(r) $(CH_2)_tNR^4COR^9$,
(s) hydroxy,
(t) $NR^9R^{10}$ ;

X représente -$NR^{15}C(O)$- ou une liaison simple carbone - carbone.

**5.** Composé de la revendication 4, dans lequel :
$R^1$ est un 5-tétrazolyle ou un carboxy ;
$R^{2a}$, $R^{2b}$, $R^{3a}$, et $R^{3b}$ représentent chacun H ;
$R^6$ est un alkyle en $C_{1-6}$ ;
A représente =O ou =S ;
B représente H ; un alkyle en $C_{1-10}$, un cycloalkyle en $C_{3-8}$ ou cycloalkyle en $C_{3-8}$-alkyle en $C_{1-4}$ substitué ou non-substitué ; un aryle, hétéroaryle, ou aralkyle substitué ou non-substitué ;
E et X représentent chacun des liaisons simples ; et
u vaut 1.

**6.** Composé de la revendication 4, dans lequel :
$R^1$ représente un 5-tétrazolyle ou un carboxy ;
$R^{2a}$, $R^{2b}$, $R^{3a}$ et $R^{3b}$ représentent chacun H ;
$R^6$ est un alkyle en $C_{1-6}$ ;
A vaut 0
B représente H, un alkyle en $C_{1-10}$ substitué ou non-substitué, un aralkyle ou un hétéroalkyle substitué ou non-substitué ;
E et X représentent chacun des liaisons simples ; et
u vaut 1.

**7.** Composé de la revendication 4, dans lequel :
$R^1$ représente un 5-tétrazolyle ou un carboxy ;
$R^{2a}$, $R^{2b}$, $R^{3a}$, et $R^{3b}$ représentent chacun H ;
$R^6$ est un alkyle en $C_{1-6}$ ;
A vaut 0 ;
B est un aryle ou un hétéroaryle substitué ou non-substitué ;
E et X représentent chacun des liaisons simples ; et
u vaut 1.

**8.** Composé de la revendication 4, dans lequel :
$R^1$ est un 5-tétrazolyle, carboxy, -$CONHSO_2R^{23}$, -$SO_2NHCOR^{23}$, ou -$SO_2NH$-hétéroaryle ;
$R^{2a}$, $R^{2b}$, $R^{3a}$, et $R^{3b}$ représentent chacun H ;
$R^6$ est un alkyle en $C_{1-6}$ ;
A représente O, S ou $NR^{21}$,
B est un alkyle en $C_{1-10}$, un aryle ou aralkyle substitué ou non-substitué ;
$R^{21}$ représente H, un aryle substitué ou non-substitué ;
$R^{23}$ est

(a) un aryle substitué ou non-substitué,
(b) un alkyle substitué ou non-substitué,

E est une liaison simple ou S,
X est une liaison simple, -NHCO- ou -CO- ; et
u vaut 1.

**9.** Composition pharmaceutique utile dans le traitement de l'hypertension, qui comprend un support pharmaceutiquement acceptable et une quantité pharmaceutiquement efficace d'un composé de la revendication 1.

**10.** Composition de la revendication 9, qui inclut un antihypertensif ou un diurétique, ou un inhibiteur de l'enzyme transformant l'angiotensine ou un agent bloquant le canal du calcium qui sont des membres choisis dans le groupe constitué par les composés suivants : l'amiloride, l'aténolol, le bendrofluméthiazide, la chlorothalidone, le chlorothiazide, la clonidine, les acétates de crypténamine et les tannates de crypténamine, la déserpidine, le diazoxyde, le sulfate de guanéthidène, le chlorhydrate d'hydralazine, l'hydrochlorothiazide, la métolazone, le tartrate de métoprolol, le méthylclothiazide, le méthyldopa, le chlorhydrate de méthyldopa, le minoxidil, le chlorhydrate de paregyline, le polythiazide, la prazosine, le propranolol, rauwolfia serpentina, la rescinnamine, la réserpine, le nitroprussiate de sodium, la spironolactone, le maléate de timolol, le trichlorométhiazide, le camsylate de trimétophan, le benzthiazide, la quinéthazone, le ticrynafan, le triamtérène, l'acétazolamide, l'aminophylline, le cyclothiazide, l'acide éthacrynique, le furosémide, la méréthoxylline procaïne, l'éthacrylate de sodium, le captopril, le chlorhydrate de délapril, l'énalapril, l'analaprilate, le fosinopril sodique, le lisinopril, le pentopril, le chlorhydrate de quinapril, le ramapril, le teprotide, le zofénopril calcique, le diflunisal, le diltiazem, la félodipine, la nicardipine, la nifédipine, la niludipine, la nimodipine, la nisoldipine, la nitrendipine, le vérapamil, et leurs mélanges et combinaisons.

**11.** Formulation ophtalmologique pour le traitement de l'hypertension oculaire comprenant un support ophtalmologiquement acceptable et une quantité antihypertensive oculaire efficace d'un composé de la revendication 1.